(19) Europäisches Patentamt
European Patent Office
Office européen des brevets

(11) EP 4 372 085 A1

(12) EUROPEAN PATENT APPLICATION
published in accordance with Art. 153(4) EPC

(43) Date of publication:
**22.05.2024 Bulletin 2024/21**

(21) Application number: **22841197.1**

(22) Date of filing: **30.06.2022**

(51) International Patent Classification (IPC):
***C12N 15/113*** (2010.01) ***A61K 47/54*** (2017.01)
***A61P 3/06*** (2006.01) ***A61K 48/00*** (2006.01)
***A61K 31/713*** (2006.01)

(52) Cooperative Patent Classification (CPC):
**A61K 31/713; A61K 47/54; A61K 48/00; A61P 3/06; C12N 15/113**

(86) International application number:
**PCT/CN2022/103049**

(87) International publication number:
**WO 2023/284559 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2021 CN 202110807464**

(71) Applicant: **Suzhou Ribo Life Science Co., Ltd.**
**Kunshan, Jiangsu 215300 (CN)**

(72) Inventors:
- **LIANG, Zicai**
**Kunshan, Jiangsu 215300 (CN)**
- **ZHANG, Hongyan**
**Kunshan, Jiangsu 215300 (CN)**
- **GAO, Shan**
**Kunshan, Jiangsu 215300 (CN)**

(74) Representative: **SSM Sandmair Patentanwälte Rechtsanwalt Partnerschaft mbB**
**Joseph-Wild-Straße 20**
**81829 München (DE)**

Remarks:
The complete document including Reference Table(s) and the Sequence Listing(s) can be downloaded from the EPO website

# (54) NUCLEIC ACID, COMPOSITION AND CONJUGATE CONTAINING SAME, AND PREPARATION METHOD AND USE

(57) An siRNA capable of inhibiting expression of apolipoprotein C3(APOC3) gene, comprising a sense strand and an antisense strand; the sense strand and the antisense strand respectively comprise a nucleotide sequence I and a nucleotide sequence II consisting of 19 modified or unmodified nucleotides, the nucleotide sequence I and the nucleotide sequence II are at least partially reversely complementary to form a double-stranded region, and the nucleotide sequence II is at least partially reversely complementary to a nucleotide sequence in an mRNA expressed by APOC3 gene; in a direction from 5' end to 3' end, at least one of the 3rd to 6th nucleotides of the nucleotide sequence II is a stabilizing modified nucleotide. The siRNA and a pharmaceutical composition and an siRNA conjugate containing same can effectively treat and/or prevent diseases or disorders related to expression of APOC3 gene, and have a significantly reduced off-target effect.

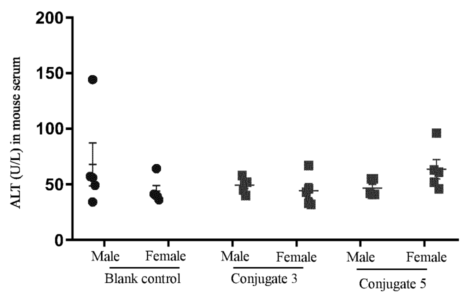


FIG. 1A

EP 4 372 085 A1

## Description

### TECHNICAL FIELD

The present disclosure relates to a nucleic acid capable of inhibiting the expression of an apolipoprotein C3 (APOC3) gene with reduced off-target effects as well as a composition and a conjugate comprising the nucleic acid. The present disclosure also relates to a preparation method for and use of the nucleic acid, composition and conjugate.

### BACKGROUND

Dyslipidemia, also known as hyperlipidemia, is a systemic disease in which fat metabolism or operation is abnormal, causing plasma lipids to be higher than normal, and it is seriously threatening the health of patients around the world. The apolipoprotein C3 (APOC3) plays an important role in lipid metabolism. The expression of APOC3 in the blood circulation of people carrying the APOC3 mutation gene decreases by 46%, and the level of triglyceride in plasma decreases by 39% compared with ordinary people. Therefore, using small interfering RNA (siRNA) to silence gene expression at the gene level and block APOC3 is undoubtedly an ideal means for treating dyslipidemia related to APOC3. In recent years, considerable progress has been made in the preparation of an siRNA for inhibiting APOC3 gene expression into drugs.

In the pharmaceutical research of the siRNA, off-target effect is one of the important side effects related to toxicity. At present, many siRNAs that have shown excellent pharmaceutical activity in preclinical pharmaceutical studies are difficult to use in actual drug development due to the toxicity caused by their off-target effects. However, the ideal siRNA expected to be used to prepare drugs that are actually applied to patients should undoubtedly have low toxicity, including low toxicity caused by off-target effects. Therefore, how to obtain siRNAs with low off-target effects still needs to be further explored in this field.

### SUMMARY

In order to develop an siRNA capable of inhibiting the APOC3 gene with significantly reduced off-target effects, the inventors unexpectedly found that siRNAs with stabilizing modified nucleotides at specific positions in a sequence exhibited significantly lower off-target effects than siRNAs without stabilizing modified nucleotides at corresponding positions. Furthermore, certain siRNAs with stabilizing modified nucleotides at specific positions in the sequence show significantly lower off-target effects than the siRNAs without stabilizing modified nucleotides, and also show not significantly reduced or comparable APOC3 gene inhibition activity than the siRNAs without stabilizing modified nucleotides. Therefore, the inventors made the following inventions.

In one aspect, the present disclosure provides an siRNA, the siRNA comprising an antisense strand and a sense strand, wherein the sense strand comprises a nucleotide sequence I, and the antisense strand comprises a nucleotide sequence II; both the nucleotide sequence I and the nucleotide sequence II consist of 19 nucleotides, and each of the nucleotides in the nucleotide sequence I and the nucleotide sequence II is a modified or unmodified nucleotide; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence with 19 nucleotides in length in an mRNA expressed by an APOC3 gene; in the direction from the 5' end to the 3' end, at least one of the 3rd-6th nucleotides in the nucleotide sequence II is a stabilizing modified nucleotide; the stabilizing modified nucleotide refers to a nucleotide whose ribose 2' hydroxyl is substituted with a stabilizing modification group; compared with an siRNA whose nucleotides at the corresponding positions are unmodified nucleotides, the thermostability of the siRNA containing the stabilizing modified nucleotide is increased, and the steric hindrance of the stabilizing modification group is greater than that of 2'-O-methyl.

In another aspect, the present disclosure also provides a pharmaceutical composition, comprising the siRNA provided by the present disclosure and a pharmaceutically acceptable carrier.

In yet another aspect, the present disclosure also provides an siRNA conjugate, comprising the siRNA provided by the present disclosure and a conjugated group conjugated to the siRNA, wherein the conjugated group comprises a linker and a pharmaceutically acceptable targeting group, and the siRNA, the linker and the targeting group are sequentially linked covalently or non-covalently; each of the targeting groups is selected from ligands capable of binding to a cell surface receptor.

In yet another aspect, the present disclosure also provides use of the siRNA, the pharmaceutical composition and the siRNA conjugate of the present disclosure in the preparation of a medicament for treating and/or preventing diseases or symptoms associated with the level of an mRNA expressed by an APOC3 gene.

In yet another aspect, the present disclosure also provides a method for treating and/or preventing diseases

or symptoms associated with the level of the mRNA expressed by the APOC3 gene, the method comprising: administering to a subject in need thereof the siRNA, the pharmaceutical composition and/or the siRNA conjugate of the present disclosure.

In yet another aspect, the present disclosure also provides a method for inhibiting the expression level of the APOC3 gene in a cell, the method comprising: contacting an effective dose of the siRNA, the pharmaceutical composition and/or the siRNA conjugate of the present disclosure with the cell.

In addition, the present disclosure also provides a kit, comprising the siRNA, the pharmaceutical composition and/or the siRNA conjugate of the present disclosure.

## Incorporation by Reference

All publications, patents, and patent applications mentioned in this specification are incorporated herein by reference to the same extent as if each individual publication, patent, or patent application was specifically and individually indicated to be incorporated herein by reference.

## Beneficial Effects

The siRNA, pharmaceutical composition and/or siRNA conjugate of the present disclosure have good stability, low off-target effect, good APOC3 gene expression inhibition activity, and good blood lipid lowering effect. The details are as follows.

First, the siRNA, the pharmaceutical composition and/or the siRNA conjugate of the present disclosure can have lower off-target effects and/or toxicity caused by off-target effects, *in vitro* or *in vivo.* In particular, mice administered with the siRNA conjugates of the present disclosure show significantly lower blood biochemical results and a clear advantage in toxicity compared with a reference siRNA conjugate. For example, in a mice administered with the siRNA conjugate of the present disclosure at a dose of 100 mg/kg, the blood biochemical indicators are significantly reduced, and there is no obvious abnormality compared with the blank control group. In addition, compared with the reference siRNA conjugate, the mice administered with the siRNA conjugate of the present disclosure do not show moderate or severe inflammatory cell infiltration and necrosis, and show significantly lower toxicity in histopathology. In another example, even at a high dose of 300 mg/kg, there is no significant difference in serum ALT compared with the blank control group. Compared with the 6 of the mice administered with the reference conjugate and showing inflammatory cell infiltration in their histopathological sections, only 3 mice administered with the conjugate of the present disclosure show inflammatory cell infiltration, indicating the number of mice showing inflammatory cell infiltration is significantly reduced. In another example, the siRNA conjugate of the present disclosure has low off-target effects. In the *in vitro* sicheck system, the siRNA conjugate of the present disclosure exhibited excellent on-target inhibition activity against the target sequence, with an $IC_{50}$ value of 4.50 pM-11.3 pM. Meanwhile, the inhibition rate against off-target target sequences in all tested siRNA concentration ranges was less than 50%, indicating low off-target effects. In another example, in mice administered the siRNA conjugate of the present disclosure weekly at a high dose of 300 mg/kg for three consecutive weeks, their serum ALT and AST concentrations were comparable to those of the blank control group. Further, the pathological sections of the mice administered the siRNA conjugate of the present disclosure also showed similar responses to the blank control group in terms of hepatic steatosis and inflammation: there was no significant abnormality, indicating that the siRNA conjugate of the present disclosure has very low hepatotoxicity.

Second, the siRNA, the pharmaceutical composition and/or the siRNA conjugate of the present disclosure exhibited excellent APOC3 gene expression regulation activity in the *in vivo* and *in vitro* experiments. In another example, the siRNA conjugate provided by the present disclosure shows a very high target sequence inhibition activity in the *in vitro* sicheck system, with $IC_{50}$ of 6.89-8.55 pM. Meanwhile, the siRNA conjugate has a target sequence inhibition activity close to that of the reference siRNA conjugate containing no stabilizing modified nucleotides. In another example, the siRNA conjugate provided by the present disclosure has very high target sequence inhibition activity in the *in vitro* sicheck system. At a low concentration of 0.01 nM, the target sequence expression inhibition rate was at least 38.92 nM and could reach up to 67.54%. At a concentration of 0.1 nM, the target sequence expression inhibition rate may be as high as 84.73-89.35%. Meanwhile, the siRNA conjugate has a target sequence inhibition activity close to that of the reference siRNA conjugate containing no stabilizing modified nucleotides or not significantly reduced target sequence inhibition activity.

Third, the siRNA, the pharmaceutical composition and/or the siRNA conjugate of the present disclosure exhibited a better blood lipid TG reduction effect *in vivo.* For example, at different time points after administration, the siRNA conjugate of the present disclosure may significantly reduce the levels of TG and CHO in mouse serum, and shows a blood lipid level lowering effect similar to that of the corresponding reference siRNA conjugate containing no stabilizing modified nucleotides or not significantly reduced blood lipid level lowering effect. In particular, at a dose of 3 mg/kg, the siRNA conjugate of the present disclosure has always shown a very high blood lipid TG lowering effect throughout the

administration period of up to 50 days, and the highest inhibition rate can reach up to 90.2%. In another example, at different time points after administration, the siRNA conjugate of the present disclosure can significantly reduce the levels of TG and CHO in mouse serum, and showed a similar blood lipid level lowering effect to the corresponding reference siRNA conjugate containing no stabilizing modified nucleotides. In particular, at doses of 3 mg/kg and 1 mg/kg, the siRNA conjugate of the present disclosure has always shown a very high blood lipid TG lowering effect throughout the administration period of up to 50 days, and the highest inhibition rate can reach up to 92.0%. In another example, at different time points after administration, different concentrations of the siRNA conjugate of the present disclosure can all reduce the TG level in mouse serum. Especially, at a dose of 9 mg/kg, the siRNA conjugate of the present disclosure, after only one administration, can maintain a TG level inhibition rate of greater than 50% for as long as 64 days, and the highest inhibition rate can reach up to 89.5%, showing an excellent blood lipid inhibition ability. In another example, at different time points after administration, the siRNA conjugate of the present disclosure can significantly reduce the levels of TG and CHO in mouse serum. In addition, throughout the 43 days of the experiment, the inhibition effect was always kept high. Especially, the siRNA conjugates of the present disclosure in a dose of 3 mg/kg all showed excellent blood lipid inhibition effects in mice: the maximum serum TG inhibition rates were all higher than 88%, and the maximum serum CHO inhibition rates were 51.18%-57.41%. In another example, at different time points after administration, the siRNA conjugate of the present disclosure can significantly reduce the levels of TG and CHO in mouse serum, and maintain a high inhibition effect throughout the 22 days of the experiment. In addition, the conjugate showed a similar blood lipid level lowering effect to the corresponding reference siRNA conjugate containing no stabilizing modified nucleotides.

Therefore, the siRNA, the pharmaceutical composition and the siRNA conjugate provided by the present disclosure may have significantly lower off-target effects and toxic reactions caused by off-target effects, and may also effectively inhibit the expression of the APOC3 gene *in vivo* and *in vitro* and show good blood lipid lowering activity at the time of hepatotoxic reaction, and thus may effectively treat and/or prevent disease symptoms associated with the level of the mRNA expressed by the APOC3 gene, especially dyslipidemia, while having significantly higher safety, thus exhibiting a good application prospect.

## BRIEF DESCRIPTION OF THE DRAWINGS

FIGs. 1A and 1B are scatter plots showing ALT and AST concentrations in mouse serum after weekly administration of 300 mg/kg of siRNA conjugates of the present disclosure or PBS for three consecutive weeks.

FIG. 2 is a histogram of the relative expression level of the target sequence in an *in vitro* sicheck system after co-transfection of a plasmid containing a target sequence and a siRNA conjugate or a reference siRNA NC.

FIGs. 3A and 3B are line graphs showing changes in the level of serum TG or serum CHO over time after administration of a siRNA conjugate of the present disclosure, a reference siRNA conjugate or PBS.

FIGs. 4A and 4B are line graphs showing changes in the level of serum TG or serum CHO over time after administration of a siRNA conjugate of the present disclosure, a reference siRNA conjugate or PBS.

FIGs. 5A and 5B are line graphs showing changes in the level of serum TG or serum CHO over time after administration of siRNA conjugates of the present disclosure or PBS.

FIG. 6 is a line graph showing changes in the serum TG level over time after administration of different concentrations of a siRNA conjugate of the present disclosure or PBS.

FIGs. 7A and 7B are line graphs showing changes in the level of serum TG or serum CHO over time after administration of siRNA conjugates of the present disclosure or PBS.

FIGs. 8A and 8B are line graphs showing changes in the level of serum TG or serum CHO over time after administration of a siRNA conjugate of the present disclosure, a reference siRNA conjugate or PBS.

## DETAILED DESCRIPTION

Specific embodiments of the present disclosure are described in detail below. It will be appreciated that the specific embodiments described herein are intended to illustrate and explain the present disclosure only rather than limit

the present disclosure.

In the present disclosure, unless otherwise stated, APOC3 mRNA or "mRNA expressed by APOC3 gene" refers to an mRNA having a sequence set forth under Genbank accession number NM_000040.3, and APOC3 gene refers to a gene that transcribes the aforementioned APOC3 mRNA.

Definitions

In the text above and below, unless otherwise specified, the uppercase letters C, G, U, and A represent the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the lowercase letter s indicates that the two nucleotides adjacent to the letter s on the left and right sides are linked by a phosphorothioate group; P1 indicates that the nucleotide adjacent to the P1 on the right side is a 5'-phosphate nucleotide or 5'-phosphate analog-modified nucleotide. In some embodiments, P1 is VP, Ps or P that indicates a specific modification, wherein the letter combination VP indicates that the nucleotide adjacent to the letter combination VP on the right side is a vinylphosphate (5'-(E)-vinylphosphonate, E-VP)-modified nucleotide, the letter combination Ps indicates that the nucleotide adjacent to the letter combination Ps on the right side is a phosphorothioate-modified nucleotide, and the uppercase letter P indicates that the nucleotide adjacent to the letter P on the right side is a 5'-phosphate nucleotide.

In the text above and below, the "fluoro-modified nucleotide" refers to a nucleotide in which the hydroxy group at the 2' position of the ribosyl group of the nucleotide is substituted with fluorine, and "non-fluoro-modified nucleotide" refers to a nucleotide or a nucleotide analog in which the hydroxy group at the 2' position of the ribosyl group of the nucleotide is substituted with a non-fluorine group. "Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, e.g., an isonucleotide, a bridged nucleic acid (BNA for short) or an acyclic nucleotide. The "methoxy-modified nucleotide" refers to a nucleotide in which the 2'-hydroxy group of the ribosyl group is substituted with a methoxy group.

In the context of the present disclosure, the expressions "complementary" and "reversely complementary" are used interchangeably and have the meaning well known to those skilled in the art, that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (C) is always paired with the pyrimidine base cytosine (G). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases in the corresponding positions are not paired in a complementary manner.

In the text above and below, unless otherwise specified, "substantially reversely complementary" means that there are no more than 3 base mismatches between two nucleotide sequence segments involved; "virtually reversely complementary " means that there is no more than one base mismatch between two nucleotide sequence segments; "completely reversely complementary" means that there is no base mismatch between two nucleotide sequence segments.

In the text above and below, especially when the preparation methods for the siRNA, pharmaceutical composition or siRNA conjugate of the present disclosure are described, unless otherwise specified, the nucleoside monomer means modified or unmodified RNA phosphoramidites (sometimes RNA phosphoramidites are also known as nucleotide phosphoramidites) used in phosphoramidite solid-phase synthesis according to the type and sequence of nucleotides in an siRNA or an siRNA conjugate to be prepared. Solid-phase phosphoramidite synthesis is a method used in RNA synthesis well known to those skilled in the art. The nucleoside monomers used in the present disclosure are all commercially available.

Those skilled in the art will appreciate that for any group containing one or more substituents, these groups are not intended to introduce any substitution or substitution pattern that is sterically impractical, synthetically infeasible, and/or inherently unstable.

As used herein, "alkyl" refers to straight and branched chains having a specified number of carbon atoms, typically 1 to 20 carbon atoms, such as 1 to 10 carbon atoms, for example, 1 to 8 or 1 to 6 carbon atoms. For example, $C_1$-$C_6$ alkyl includes straight and branched chain alkyl groups of 1 to 6 carbon atoms. When reference is made to an alkyl residue having a specific number of carbons, all branched and straight chain forms having that number of carbons are intended to be encompassed. Therefore, for example, "butyl" is meant to include n-butyl, sec-butyl, isobutyl and tert-butyl; and "propyl" includes n-propyl and isopropyl. Alkylene is a subset of alkyl and refers to residues which are identical to alkyl but have two points of attachment.

As used herein, "alkenyl" refers to an unsaturated branched or straight chain alkyl group having at least one carbon-carbon double bond obtained by removing a molecule of hydrogen from adjacent carbon atoms of the parent alkyl group. The group may be in the cis or trans configuration of the double bond. Typical alkenyl groups include, but are not limited to: vinyl; propenyl, such as prop-1-en-1-yl, prop-1-en-2-yl, prop-2-en-1-yl (allyl), and prop-2-en-2-yl; and butenyl, such as but-1-en-1-yl, but-1-en-2-yl, 2-methylprop-1-en-1-yl, but-2-en-1-yl, but-2-en-2-yl, but-1,3-dien-1-yl and but-1,3-dien-2-yl. In certain embodiments, alkenyl groups have 2 to 20 carbon atoms, while in other embodiments, alkenyl groups have 2 to 10, 2 to 8, or 2 to 6 carbon atoms. Alkenylene is a subset of alkenyl and refers to residues which are identical to alkenyl but have two points of attachment.

As used herein, "alkynyl" refers to an unsaturated branched or straight chain alkyl group having at least one carbon-carbon triple bond obtained by removing two molecules of hydrogen from adjacent carbon atoms of the parent alkyl group. Typical alkynyl groups include, but are not limited to: ethynyl; propynyl, such as prop-1-yn-1-yl and prop-2-yn-1-yl; and butynyl, such as but-1-yn-1-yl, but-1-yn-3-yl and but-3-yn-1-yl. In certain embodiments, alkynyl groups have 2 to 20 carbon atoms, while in other embodiments, alkynyl groups have 2 to 10, 2 to 8, or 2 to 6 carbon atoms. Alkynylene is a subset of alkynyl and refers to residues which are identical to alkynyl but have two points of attachment.

As used herein, "alkoxy" refers to an alkyl group of a specified number of carbon atoms linked by an oxygen bridge, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy and 3-methylpentyloxy. An alkoxy group typically has 1 to 10, 1 to 8, 1 to 6, or 1 to 4 carbon atoms linked by an oxygen bridge.

As used herein, "aryl" refers to a group derived from an aromatic monocyclic or polycyclic hydrocarbon ring system by removing a hydrogen atom from a ring carbon atom. The aromatic monocyclic or polycyclic hydrocarbon ring system contains only hydrogen and carbons of 6 to 18 carbon atoms, wherein at least one ring in the ring system is fully unsaturated, i.e., a cyclic delocalized $(4n+2)\pi$-electron system according to the Hückel theory is included. Aryl groups include, but are not limited to, groups such as phenyl, fluorenyl, and naphthyl. Arylene is a subset of aryl and refers to residues which are identical to aryl but have two points of attachment.

"Heteroaryl" refers to a group derived from a 3- to 18-membered aromatic ring and contains 2 to 17 carbon atoms and 1 to 6 heteroatoms selected from nitrogen, oxygen and sulfur. As used herein, a heteroaryl group may be a monocyclic, bicyclic, tricyclic or tetracyclic ring system, wherein at least one ring in the ring system is fully unsaturated, i.e., a cyclic delocalized $(4n+2)$ $\pi$-electron system according to the Hückel theory is included. Heteroaryl groups include fused or bridged ring systems. In some embodiments, the heteroatoms in the heteroaryl group are oxidized heteroatoms. In some embodiments, one or more nitrogen atoms are included in the heteroaryl group. In some embodiments, one or more of the nitrogen atoms in the heteroaryl group are quaternized nitrogen atoms. A heteroaryl group is attached to the rest of the molecule via any ring atom. Examples of heteroaryl groups include, but are not limited to: azacycloheptatriene, acridinyl, benzimidazolyl, benzindolyl, 1,3-benzobisoxazolyl, benzofuranyl, benzooxazolyl, benzo[d]thiazolyl, benzothiadiazolyl, benzo[*b*][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-SH-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[H]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl.

A variety of hydroxy protecting groups may be used in the present disclosure. In general, protecting groups render a chemical functionality insensitive to specific reaction conditions and may be added and removed at that functionality in a molecule without substantially damaging the rest of the molecule. Representative hydroxyl protecting groups are disclosed in Beaucage et al., Tetrahedron 1992, 48, 2223-2311, and Greene and Wuts, Protective Groups in Organic Synthesis, Chapter 2, 2d ed, John Wiley & Sons, New York, 1991, each of which is hereby incorporated by reference in its entirety. In some embodiments, the protecting groups are stable under basic conditions and can be removed under acidic conditions. In some embodiments, non-exclusive examples of hydroxyl protecting groups used herein include dimethoxytrityl (DMT), monomethoxytrityl, 9-phenyloxanthene-9-yl (Pixyl) and 9-(p-methoxyphenyl)xanth-9-yl (Mox). In some embodiments, non-exclusive examples of hydroxyl protecting groups used herein include Tr (trityl), MMTr (4-methoxytrityl), DMTr (4,4'-dimethoxy Trityl) and TMTr (4,4',4"-trimethoxytrityl).

The term "subject", as used herein, refers to any animal, such as a mammal or a marsupial. Subjects of the present disclosure include, but are not limited to, humans, non-human primates (e.g., rhesus or other types of macaques), mice, pigs, horses, donkeys, cows, rabbits, sheep, rats, and any species of poultry.

As used herein, "treatment" refers to means of obtaining a beneficial or desired result, including but not limited to therapeutic benefit. The term "treatment benefit" means eradicating or ameliorating the underlying disorder being treated. Furthermore, therapeutic benefit is obtained by eradicating or ameliorating one or more physiological symptoms associated with the underlying disorder, whereby improvement is observed in the subject, although the subject may still be afflicted by the underlying disorder.

"Prevention" as used herein refers to the means of obtaining a beneficial or desired result, including but not limited to prophylactic benefit. For "prophylactic benefit", a double-stranded siRNA, a pharmaceutical composition, or an siRNA conjugate may be administered to a subject at risk for a particular disease, or a subject with one or more physiological symptoms of a reported disease, even though a diagnosis of the disease may not have been made.

siRNA of the present disclosure

In one aspect, the present disclosure provides an siRNA with a high APOC3 gene inhibition activity and a low off-target effect.

The siRNA of the present disclosure contains a nucleotide group as a basic structural unit. It is well known to those skilled in the art that the nucleotide group contains a phosphate group, a ribosyl group and a base, which are not detailed here.

The siRNA of the present disclosure comprises a sense strand and an antisense strand, wherein the sense strand comprises a nucleotide sequence I, and the antisense strand comprises a nucleotide sequence II; both the nucleotide sequence I and the nucleotide sequence II consist of 19 nucleotides, and each of the nucleotides in the nucleotide sequence I and the nucleotide sequence II is a modified or unmodified nucleotide; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence with 19 nucleotides in length in an mRNA expressed by an APOC3 gene; in the direction from the 5' end to the 3' end, at least one of the 3rd-6th nucleotides in the nucleotide sequence 2 is a stabilizing modified nucleotide; the stabilizing modified nucleotide refers to a nucleotide whose ribose 2' hydroxyl is substituted with a stabilizing modification group; compared with an siRNA whose nucleotides at the corresponding positions are unmodified nucleotides, the thermostability of the siRNA containing the stabilizing modified nucleotide is increased, and the steric hindrance of the stabilizing modification group is greater than that of 2'-O-methyl.

In some embodiments, in the direction from the 5' end to the 3' end, the 3rd or 5th nucleotide in the nucleotide sequence II is the stabilizing modified nucleotide. In some embodiments, in the direction from the 5' end to the 3' end, no more than 2 nucleotides of the 3rd-9th nucleotides in the nucleotide sequence II are the stabilizing modified nucleotides. By limiting the number of stabilizing modified nucleotides at a specific position, the siRNA of the present disclosure may achieve an optimal balance between pharmaceutical activity and low off-target effects, and also has excellent stability. In some embodiments, in the direction from the 5' end to the 3' end, the 3rd and/or 5th nucleotide(s) in the nucleotide sequence II are/is the stabilizing modified nucleotide(s). In some embodiments, in the direction from the 5' end to the 3' end, the 3rd nucleotide in the nucleotide sequence II is the stabilizing modified nucleotide. In some embodiments, in the direction from the 5' end to the 3' end, the 5th nucleotide in the nucleotide sequence II is the stabilizing modified nucleotide.

In the siRNA of the present disclosure, in the direction from the 5' end to the 3' end, the nucleotides other than the 3rd to 9th nucleotides in the nucleotide sequence II are not the stabilizing modified nucleotides. If at least one of the 3rd to 6th nucleotides in the nucleotide sequence II is the stabilizing modified nucleotide, and the sequence also contains a stabilizing modified nucleotide other than the 3rd to 9th nucleotides, the ability to regulate the expression level of the target sequence of the siRNA may be significantly affected.

In some embodiments, "siRNA has increased thermostability" means that the melting temperature (Tm) of the siRNA is increased. In some embodiments, "double-stranded siRNA has increased thermostability" means that the Tm of the siRNA is increased by at least 0.05 °C. In some embodiments, the expression means an increase of 0.1-6 °C. In some embodiments, the expression means an increase of the Tm of 0.5-4 °C. Without being limited by theoretical explanation, by including stabilizing modified nucleotides at specific positions, the ability of the antisense strand in the siRNA of the present disclosure to bind to the mRNA expressed by the APOC3 gene is substantially unaffected, while the binding to an off-target target mRNA is significantly reduced, thereby reducing or even eliminating off-target effects.

In some embodiments, each of the stabilizing modification groups independently has a structure represented by -X-R, wherein X is O, NR', S or $SiR'_2$; R is one of $C_2$-$C_6$ alkyl, substituted $C_2$-$C_6$ alkyl, $C_6$-$C_8$ aryl and substituted $C_6$-$C_8$ aryl; each R' is independently one of H, $C_1$-$C_6$ alkyl, substituted Ci-$C_6$ alkyl, $C_6$-$C_8$ aryl and substituted $C_6$-$C_8$ aryl; the substituted $C_2$-$C_6$ alkyl, substituted $C_6$-$C_8$ aryl or substituted $C_1$-$C_6$ alkyl refers to a group formed by replacing one or more hydrogen atoms on $C_2$-$C_6$ alkyl, $C_6$-$C_8$ aryl or $C_1$-$C_6$ alkyl by a substituent, and the substituent is selected

from one or more of the following substituents: $C_1$-$C_3$ alkyl, $C_6$-$C_8$ aryl, $C_1$-$C_3$ alkoxy, halogen, oxo and sulfanylidene. It should be noted that the present disclosure is not intended to cover all modified groups conforming to the structure, but only involves those stabilizing modification groups capable of increasing the thermostability of the siRNA. In some embodiments, each of the stabilizing modification groups is independently selected from one of 2'-O-methoxyethyl, 2'-O-allyl, 2'-C-allyl, 2'-O-2-N-methylamino-2-oxoethyl, 2'-O-2-N,N-dimethylaminoethyl, 2'-O-3-aminopropyl and 2'-O-2,4-dinitrophenyl. In some embodiments, each of the stabilizing modification groups is 2'-O-methoxyethyl.

In some embodiments, the siRNA with stabilized nucleotides of the present disclosure may be the following first, second, or third siRNA, and each siRNA will be described below.

First siRNA

In some embodiments, the siRNA of the present disclosure is the first siRNA. The nucleotide sequence I and the nucleotide sequence set forth in SEQ ID NO: 1 are equal in length and differ by no more than 3 nucleotides; the nucleotide sequence II and the nucleotide sequence set forth in SEQ ID NO: 2 are equal in length and differ by no more than 3 nucleotides:

5'- CAAUAAAGCUGGACAAGA$Z_1$ -3' (SEQ ID NO:1);

5'- $Z_2$UCUUGUCCAGCUUUAUUG -3' (SEQ ID NO:2),

wherein $Z_1$ is A, and $Z_2$ is U;

furthermore, the nucleotide sequence I contains nucleotide $Z_3$ corresponding to $Z_1$ in position, the nucleotide sequence II contains nucleotide $Z_4$ corresponding to $Z_2$ in position, and $Z_4$ is the first nucleotide at the 5' end of the antisense strand. The first nucleotide sequence segment is the nucleotide sequence set forth in SEQ ID NO: 1. Each U may be arbitrarily replaced by T. In the context of the present disclosure, "corresponding to ... in position" refers to being at the same position in the nucleotide sequence, counting from the same end of the nucleotide sequence. For example, the first nucleotide at the 3' end of nucleotide sequence I is the nucleotide whose position corresponds to the first nucleotide set forth in SEQ ID NO: 1.

In some embodiments, the sense strand only contains the nucleotide sequence I, and the antisense strand only contains the nucleotide sequence II.

In some embodiments, the nucleotide sequence I and the nucleotide sequence set forth in SEQ ID NO: 1 differ by no more than 1 nucleotide, and/or the nucleotide sequence II and the nucleotide sequence set forth in SEQ ID NO: 2 differ by no more than 1 nucleotide.

In some embodiments, the nucleotide difference between the nucleotide sequence II and the nucleotide sequence set forth in SEQ ID NO: 2 comprises the difference at $Z_4$ position, and $Z_4$ is selected from A, G or C. In some embodiments, the nucleotide difference is the difference at $Z_4$ position, and $Z_4$ is selected from A, G or C. In some embodiments, $Z_3$ is a nucleotide complementary to $Z_4$. These nucleotide differences will not significantly reduce the target gene inhibition ability of the siRNA or increase the off-target effect of the siRNA, and these siRNAs containing nucleotide differences are also within the protection scope of the present disclosure.

In some embodiments, the nucleotide sequence I is substantially reversely complementary, virtually reversely complementary or completely reversely complementary to the nucleotide sequence II; the substantially reversely complementary means that there are no more than 3 base mismatches between the two nucleotide sequences; the virtually reversely complementary means that there are no more than 1 base mismatch between the two nucleotide sequences; the completely reversely complementary means that there is no mismatch between the two nucleotide sequences.

In some embodiments, in the direction from the 5' end to the 3' end, the nucleotides at the 2nd-19th positions of the nucleotide sequence II are completely reversely complementary to the nucleotides at the 1st-18th positions of the first nucleotide sequence segment. In some embodiments, the nucleotide sequence II is completely reversely complementary to the nucleotide sequence I, or there is a base mismatch between the 2nd nucleotide in the nucleotide sequence II in the direction from the 5' end to the 3' end and the 2nd nucleotide in the nucleotide sequence I in the direction from the 3' end to the 5' end. By including the base mismatch, the target gene expression inhibition activity of the siRNA of the present disclosure may be further improved while maintaining a low off-target effect.

In some embodiments, the nucleotide sequence I is a nucleotide sequence set forth in SEQ ID NO: 3, and the nucleotide sequence II is a nucleotide sequence set forth in SEQ ID NO: 4:

5'- CAAUAAAGCUGGACAAGA$Z_3$ -3'(SEQ ID NO:3);

5'- $Z_4$UCUUGUCCAGCUUUAUUG -3'(SEQ ID NO:4),

wherein $Z_3$ is selected from A, U, G or C, and $Z_4$ is a nucleotide complementary to $Z_3$. In some embodiments, $Z_3$ is A and $Z_4$ is U.

Furthermore, the sense strand and the antisense strand have the same or different lengths; the length of the sense strand is 19-23 nucleotides; the length of the antisense strand is 19-26 nucleotides, thus, the length ratio of the sense strand to the antisense strand of the siRNA provided by the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the antisense strand of the siRNA is 19/21, 21/23 or 23/25.

In some embodiments, the sense strand also contains a nucleotide sequence III, and the antisense strand also contains a nucleotide sequence IV; each nucleotide in the nucleotide sequence III and the nucleotide sequence IV is independently one of non-fluoro-modified nucleotides and is not the stabilizing modified nucleotide; the lengths of the nucleotide sequence III and the nucleotide sequence IV are each 1-4 nucleotides; the nucleotide sequence IV and the nucleotide sequence III are equal in length, and the nucleotide sequence IV and the nucleotide sequence III are virtually reversely complementary or completely reversely complementary; the nucleotide sequence III is linked to the 5' end of the nucleotide sequence I, and the nucleotide sequence IV is linked to the 3' end of the nucleotide sequence II. Furthermore, the nucleotide sequence IV is virtually reversely complementary or completely reversely complementary to a second nucleotide sequence segment, and the second nucleotide sequence segment refers to a nucleotide sequence adjacent to the first nucleotide sequence segment and having the same length as the nucleotide sequence IV in the mRNA expressed by the APOC3 gene.

In some embodiments, the lengths of the nucleotide sequences III and IV are both 1 nucleotide, the base of the nucleotide sequence III is C, and the base of the nucleotide sequence IV is G; the base of the second nucleotide sequence segment is C; at this time, the length ratio of the sense strand to the antisense strand is 20/20; or the lengths of the nucleotide sequences III and IV are both 2 nucleotides, the base composition of the nucleotide sequence III is CC, and the base composition of the nucleotide sequence IV is GG; the composition of the second nucleotide sequence segment is CC; at this time, the length ratio of the sense strand to the antisense strand is 21/21; or the lengths of the nucleotide sequences III and IV are both 3 nucleotides, the base composition of the nucleotide sequence III is UCC, and the base composition of nucleotide IV is GGA; the composition of the second nucleotide sequence segment is UCC; at this time, the length ratio of the sense strand to the antisense strand is 22/22; or the lengths of the nucleotide sequences III and IV are both 4 nucleotides, the base composition of the nucleotide sequence III is CUCC, and the base composition of the nucleotide IV is GGAG; the base composition of the second nucleotide sequence segment is CUCC; at this time, the length ratio of the sense strand to the antisense strand is 21/21.

In some embodiments, the nucleotide sequence III and the nucleotide sequence IV are completely reversely complementary. Therefore, given the base composition of the nucleotide sequence III, the base composition of the nucleotide sequence IV is determined.

Second siRNA

In some embodiments, the siRNA of the present disclosure is the second siRNA. The nucleotide sequence I and the nucleotide sequence set forth in SEQ ID NO: 45 are equal in length and differ by no more than 3 nucleotides; the nucleotide sequence II and the nucleotide sequence set forth in SEQ ID NO: 46 are equal in length and differ by no more than 3 nucleotides:

5'- UUAAAAGGGACAGUAUUC$Z_5$ -3' (SEQ ID NO:45);

5'- $Z_6$GAAUACUGUCCCUUUUAA -3' (SEQ ID NO:46),

wherein $Z_5$ is U and $Z_6$ is A;

furthermore, the nucleotide sequence I contains nucleotide $Z_7$ corresponding to $Z_5$ in position, the nucleotide sequence II contains nucleotide $Z_8$ corresponding to $Z_6$ in position, and $Z_8$ is the first nucleotide at the 5' end of the antisense strand. The first nucleotide sequence segment is the nucleotide sequence set forth in SEQ ID NO: 45. Each U may be arbitrarily replaced by T.

In some embodiments, the sense strand only contains the nucleotide sequence I, and the antisense strand only contains the nucleotide sequence II.

In some embodiments, the nucleotide sequence I and the nucleotide sequence set forth in SEQ ID NO: 45 differ by no more than 1 nucleotide, and/or the nucleotide sequence II and the nucleotide sequence set forth in SEQ ID NO: 46 differ by no more than 1 nucleotide.

In some embodiments, the nucleotide difference between the nucleotide sequence II and the nucleotide sequence set forth in SEQ ID NO: 46 comprises the difference at $Z_8$ position, and $Z_8$ is selected from U, G or C. In some embodiments, the nucleotide difference is the difference at $Z_8$ position, and $Z_8$ is selected from U, G or C. In some embodiments, $Z_7$ is a nucleotide complementary to $Z_8$. These nucleotide differences will not significantly reduce the target gene inhibition ability of the siRNA or increase the off-target effect of the siRNA, and these siRNAs containing nucleotide differences are also within the protection scope of the present disclosure.

In some embodiments, the nucleotide sequence I is substantially reversely complementary, virtually reversely complementary or completely reversely complementary to the nucleotide sequence II; the substantially reversely complementary means that there are no more than 3 base mismatches between the two nucleotide sequences; the virtually reversely complementary means that there are no more than 1 base mismatch between the two nucleotide sequences; the completely reversely complementary means that there is no mismatch between the two nucleotide sequences.

In some embodiments, in the direction from the 5' end to the 3' end, the nucleotides at the 2nd-19th positions of the nucleotide sequence II are completely reversely complementary to the nucleotides at the 1st-18th positions of the first nucleotide sequence segment. In some embodiments, the nucleotide sequence II is completely reversely complementary to the nucleotide sequence I, or there is a base mismatch between the 2nd nucleotide in the nucleotide sequence II in the direction from the 5' end to the 3' end and the 2nd nucleotide in the nucleotide sequence I in the direction from the 3' end to the 5' end. By including the base mismatch, the target gene expression inhibition activity of the siRNA of the present disclosure may be further improved while maintaining a low off-target effect.

In some embodiments, the nucleotide sequence I is a nucleotide sequence set forth in SEQ ID NO: 47, and the nucleotide sequence II is a nucleotide sequence set forth in SEQ ID NO: 48:

5'- UUAAAAGGGACAGUAUUC$Z_7$ -3'(SEQ ID NO:47);

5'- $Z_8$GAAUACUGUCCCUUUUAA -3'(SEQ ID NO:48),

wherein $Z_7$ is selected from A, U, G or C, and $Z_8$ is a nucleotide complementary to $Z_7$. In some embodiments, $Z_7$ is U and $Z_8$ is A.

Furthermore, the sense strand and the antisense strand have the same or different lengths; the length of the sense strand is 19-23 nucleotides; the length of the antisense strand is 19-26 nucleotides, thus, the length ratio of the sense strand to the antisense strand of the siRNA provided by the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the antisense strand of the siRNA is 19/21, 21/23 or 23/25.

In some embodiments, the sense strand also contains a nucleotide sequence III, and the antisense strand also contains a nucleotide sequence IV; each nucleotide in the nucleotide sequence III and the nucleotide sequence IV is independently one of non-fluoro-modified nucleotides and is not the stabilizing modified nucleotide; the lengths of the nucleotide sequence III and the nucleotide sequence IV are each 1-4 nucleotides; the nucleotide sequence IV and the nucleotide sequence III are equal in length, and the nucleotide sequence IV and the nucleotide sequence III are virtually reversely complementary or completely reversely complementary; the nucleotide sequence III is linked to the 5' end of the nucleotide sequence I, and the nucleotide sequence IV is linked to the 3' end of the nucleotide sequence II. Furthermore, the nucleotide sequence IV is virtually reversely complementary or completely reversely complementary to a second nucleotide sequence segment, and the second nucleotide sequence segment refers to a nucleotide sequence adjacent to the first nucleotide sequence segment and having the same length as the nucleotide sequence IV in the mRNA expressed by the APOC3 gene.

In some embodiments, the lengths of the nucleotide sequences III and IV are both 1 nucleotide, the base of the nucleotide sequence III is C, and the base of the nucleotide sequence IV is G; the base of the second nucleotide sequence segment is C; at this time, the length ratio of the sense strand to the antisense strand is 20/20; or the lengths of the nucleotide sequences III and IV are both 2 nucleotides, the base composition of the nucleotide sequence III is GC, and the base composition of the nucleotide sequence IV is GC; the composition of the second nucleotide sequence segment is GC; at this time, the length ratio of the sense strand to the antisense strand is 21/21; or the lengths of the nucleotide sequences III and IV are both 3 nucleotides, the base composition of the nucleotide sequence III is UGC, and the base composition of nucleotide IV is GCA; the composition of the second nucleotide sequence segment is GCA; at this time, the length ratio of the sense strand to the antisense strand is 22/22; or the lengths of the nucleotide sequences III and IV are both 4 nucleotides, the base composition of the nucleotide sequence III is UUGC, and the base composition

of the nucleotide IV is GCAA; the base composition of the second nucleotide sequence segment is GCAA; at this time, the length ratio of the sense strand to the antisense strand is 21/21.

In some embodiments, the nucleotide sequence III and the nucleotide sequence IV are completely reversely complementary. Therefore, given the base composition of the nucleotide sequence III, the base composition of the nucleotide sequence IV is determined.

Third siRNA

In some embodiments, the siRNA of the present disclosure is the second siRNA. The nucleotide sequence I and the nucleotide sequence set forth in SEQ ID NO: 105 are equal in length and differ by no more than 3 nucleotides; the nucleotide sequence II and the nucleotide sequence set forth in SEQ ID NO: 106 are equal in length and differ by no more than 3 nucleotides:

5'- GGACAGUAUUCUCAGUGC$Z_9$ -3'(SEQ ID NO: 105);

5'- $Z_{10}$GCACUGAGAAUACUGUCC -3'(SEQ ID NO: 106),

wherein $Z_9$ is U and $Z_{10}$ is A;

furthermore, the nucleotide sequence I contains nucleotide $Z_{11}$ corresponding to $Z_9$ in position, the nucleotide sequence II contains nucleotide $Z_{12}$ corresponding to $Z_{10}$ in position, and $Z_8$ is the first nucleotide at the 5' end of the antisense strand. The first nucleotide sequence segment is the nucleotide sequence set forth in SEQ ID NO: 105. Each U may be arbitrarily replaced by T.

In some embodiments, the sense strand only contains the nucleotide sequence I, and the antisense strand only contains the nucleotide sequence II.

In some embodiments, the nucleotide sequence I and the nucleotide sequence set forth in SEQ ID NO: 105 differ by no more than 1 nucleotide, and/or the nucleotide sequence II and the nucleotide sequence set forth in SEQ ID NO: 106 differ by no more than 1 nucleotide.

In some embodiments, the nucleotide difference between the nucleotide sequence II and the nucleotide sequence set forth in SEQ ID NO: 106 comprises the difference at $Z_{12}$ position, and $Z_{12}$ is selected from G, C or U. In some embodiments, the nucleotide difference is the difference at $Z_{12}$ position, and $Z_{12}$ is selected from G, C or U. In some embodiments, $Z_{11}$ is a nucleotide complementary to $Z_{12}$. These nucleotide differences will not significantly reduce the target gene inhibition ability of the siRNA or increase the off-target effect of the siRNA, and these siRNAs containing nucleotide differences are also within the protection scope of the present disclosure.

In some embodiments, the nucleotide sequence I is substantially reversely complementary, virtually reversely complementary or completely reversely complementary to the nucleotide sequence II; the substantially reversely complementary means that there are no more than 3 base mismatches between the two nucleotide sequences; the virtually reversely complementary means that there are no more than 1 base mismatch between the two nucleotide sequences; the completely reversely complementary means that there is no mismatch between the two nucleotide sequences.

In some embodiments, in the direction from the 5' end to the 3' end, the nucleotides at the 2nd-19th positions of the nucleotide sequence II are completely reversely complementary to the nucleotides at the 1st-18th positions of the first nucleotide sequence segment. In some embodiments, the nucleotide sequence II is completely reversely complementary to the nucleotide sequence I, or there is a base mismatch between the 2nd nucleotide in the nucleotide sequence II in the direction from the 5' end to the 3' end and the 2nd nucleotide in the nucleotide sequence I in the direction from the 3' end to the 5' end. By including the base mismatch, the target gene expression inhibition activity of the siRNA of the present disclosure may be further improved while maintaining a low off-target effect.

In some embodiments, the nucleotide sequence I is a nucleotide sequence set forth in SEQ ID NO: 107, and the nucleotide sequence II is a nucleotide sequence set forth in SEQ ID NO: 108:

5'- GGACAGUAUUCUCAGUGCZ 11 -3'(SEQ ID NO:107);

5'- Z 12GCACUGAGAAUACUGUCC -3'(SEQ ID NO: 108),

wherein $Z_{11}$ is selected from A, U, G or C, and $Z_{12}$ is a nucleotide complementary to $Z_{11}$. In some embodiments, $Z_{11}$ is U and $Z_{12}$ is A.

Furthermore, the sense strand and the antisense strand have the same or different lengths; the length of the

sense strand is 19-23 nucleotides; the length of the antisense strand is 19-26 nucleotides, thus, the length ratio of the sense strand to the antisense strand of the siRNA provided by the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the antisense strand of the siRNA is 19/21, 21/23 or 23/25.

In some embodiments, the sense strand also contains a nucleotide sequence III, and the antisense strand also contains a nucleotide sequence IV; each nucleotide in the nucleotide sequence III and the nucleotide sequence IV is independently one of non-fluoro-modified nucleotides and is not the stabilizing modified nucleotide; the lengths of the nucleotide sequence III and the nucleotide sequence IV are each 1-4 nucleotides; the nucleotide sequence IV and the nucleotide sequence III are equal in length, and the nucleotide sequence IV and the nucleotide sequence III are virtually reversely complementary or completely reversely complementary; the nucleotide sequence III is linked to the 5' end of the nucleotide sequence I, and the nucleotide sequence IV is linked to the 3' end of the nucleotide sequence II. Furthermore, the nucleotide sequence IV is virtually reversely complementary or completely reversely complementary to a second nucleotide sequence segment, and the second nucleotide sequence segment refers to a nucleotide sequence adjacent to the first nucleotide sequence segment and having the same length as the nucleotide sequence IV in the mRNA expressed by the APOC3 gene.

In some embodiments, the lengths of the nucleotide sequences III and IV are both 1 nucleotide, the base of the nucleotide sequence III is C, and the base of the nucleotide sequence IV is G; the base of the second nucleotide sequence segment is C; at this time, the length ratio of the sense strand to the antisense strand is 20/20; or the lengths of the nucleotide sequences III and IV are both 2 nucleotides, the base composition of the nucleotide sequence III is AG, and the base composition of the nucleotide sequence IV is CU; the composition of the second nucleotide sequence segment is AG; at this time, the length ratio of the sense strand to the antisense strand is 21/21; or the lengths of the nucleotide sequences III and IV are both 3 nucleotides, the base composition of the nucleotide sequence III is AAG, and the base composition of nucleotide IV is CUU; the composition of the second nucleotide sequence segment is AAG; at this time, the length ratio of the sense strand to the antisense strand is 22/22; or the lengths of the nucleotide sequences III and IV are both 4 nucleotides, the base composition of the nucleotide sequence III is AAAG, and the base composition of the nucleotide IV is CUUU; the base composition of the second nucleotide sequence segment is AAAG; at this time, the length ratio of the sense strand to the antisense strand is 21/21.

In some embodiments, the nucleotide sequence III and the nucleotide sequence IV are completely reversely complementary. Therefore, given the base of the nucleotide sequence III, the base of the nucleotide sequence IV is determined.

Hereinafter, descriptions of nucleotide sequence V, nucleotide modification in siRNA, and modified sequence are applicable to the above-mentioned siRNA of the present disclosure, such as the first siRNA, the second siRNA or the third siRNA. That is, if there is no specific description, the following description of siRNA should be regarded as describing the above-mentioned siRNAs of the present disclosure, such as the first siRNA, the second siRNA and the third siRNA one by one. For example, if the specific siRNA is not specified, "the siRNA also contains the nucleotide sequence V" means "the siRNA of the present disclosure, such as the first siRNA, the second siRNA or the third siRNA described above, also contains the nucleotide sequence V".

In some embodiments, the sense strand and the antisense strand have different lengths, the antisense strand also contains a nucleotide sequence V, each nucleotide in the nucleotide sequence V is independently one of the non-fluoro-modified nucleotides and is not the stabilizing modified nucleotide, the length of the nucleotide sequence V is 1 to 3 nucleotides, and the nucleotide sequence V is linked to the 3' end of the antisense strand, constituting the 3' overhang of the antisense strand. Thus, the length ratio of the sense strand to the antisense strand of the siRNA provided by the present disclosure may be 19/20, 19/21, 19/22, 20/21, 20/22, 20/23, 21/22, 21/23, 21/24, 22/23, 22/24, 22/25, 23/24, 23/25 or 23/26. In some embodiments, the nucleotide sequence V is 2 nucleotides in length, and thus, the length ratio of the sense strand to the antisense strand of the siRNA provided by the present disclosure may be 19/21, 21/23 or 23/25.

Each nucleotide in the nucleotide sequence V may be any nucleotide. For the convenience of synthesis and cost saving, the nucleotide sequence V is 2 consecutive thymine deoxyribonucleotides (dTdT) or 2 consecutive uracil ribonucleotides (UU); or, in order to improve the affinity of the siRNA antisense strand to the target mRNA, the nucleotide sequence V is completely reversely complementary to a third nucleotide sequence segment. The third nucleotide sequence segment refers to a nucleotide sequence adjacent to the first nucleotide sequence segment or the second nucleotide sequence segment and having the same length as the nucleotide sequence V in the mRNA expressed by an APOC3 gene. Therefore, in some embodiments, the length ratio of the sense strand to the antisense strand of the siRNA of the present disclosure is 19/21 or 21/23, at this time, the siRNA of the present disclosure has better mRNA silencing activity.

In some embodiments, for the first siRNA, the first nucleotide sequence segment has the nucleotide sequence set forth in SEQ ID NO: 1, and the base composition of the third nucleotide sequence segment is CC; the sense strand of the siRNA contains the nucleotide sequence set forth in SEQ ID NO: 5, and the antisense strand of the siRNA contains

the nucleotide sequence set forth in SEQ ID NO: 6:

5'- CAAUAAAGCUGGACAAGA$Z_3$ -3'(SEQ ID NO:5);

5'- $Z_4$UCUUGUCCAGCUUUAUUGGG -3'(SEQ ID NO:6),

or the sense strand of the siRNA contains the nucleotide sequence set forth in SEQ ID NO: 7, and the antisense strand of the siRNA contains the nucleotide sequence set forth in SEQ ID NO: 8:

5'- CCCAAUAAAGCUGGACAAGA$Z_3$ -3'(SEQ ID NO:7);

5'- $Z_4$UCUUGUCCAGCUUUAUUGGGAG -3'(SEQ ID NO:8),

wherein $Z_4$ is the first nucleotide at the 5' end of the antisense strand, $Z_3$ is selected from A, U, G or C, and $Z_4$ is a nucleotide complementary to $Z_3$.

In some embodiments, the sense strand of the siRNA contains the nucleotide sequence set forth in SEQ ID NO: 9, and the antisense strand of the siRNA contains the nucleotide sequence set forth in SEQ ID NO: 10:

5'- CAAUAAAGCUGGACAAGAA -3'(SEQ ID NO:9);

5'- UUCUUGUCCAGCUUUAUUGGG -3'(SEQ ID NO:10),

or the sense strand of the siRNA contains the nucleotide sequence set forth in SEQ ID NO: 11, and the antisense strand of the siRNA contains the nucleotide sequence set forth in SEQ ID NO: 12:

5'- CCCAAUAAAGCUGGACAAGAA -3'(SEQ ID NO: 11);

5'- UUCUUGUCCAGCUUUAUUGGGAG -3'(SEQ ID NO:12).

In some embodiments, for the second siRNA, the first nucleotide sequence segment has the nucleotide sequence set forth in SEQ ID NO: 45, and the base composition of the third nucleotide sequence segment is GC. The sense strand of the siRNA contains the nucleotide sequence set forth in SEQ ID NO: 49, and the antisense strand of the siRNA contains the nucleotide sequence set forth in SEQ ID NO: 50:

5'- UUAAAAGGGACAGUAUUC$Z_7$ -3'(SEQ ID NO:49);

5'- $Z_8$GAAUACUGUCCCUUUUAAGC -3'(SEQ ID NO:50),

or the sense strand of the siRNA contains the nucleotide sequence set forth in SEQ ID NO: 51, and the antisense strand of the siRNA contains the nucleotide sequence set forth in SEQ ID NO: 52:

5'- GCUUAAAAGGGACAGUAUUC$Z_7$ -3'(SEQ ID NO:51);

5'- $Z_8$GAAUACUGUCCCUUUUAAGCAA -3'(SEQ ID NO:52),

or the sense strand of the siRNA contains the nucleotide sequence set forth in SEQ ID NO: 49, and the antisense strand contains the nucleotide sequence set forth in SEQ ID NO: 149:

5'- UUAAAAGGGACAGUAUUC$Z_7$ -3'(SEQ ID NO:49);

5'- $Z_8$GAAUACUGUCCCUUUUAAUU -3'(SEQ ID NO: 149),

or the sense strand of the siRNA contains the nucleotide sequence set forth in SEQ ID NO: 51, and the antisense strand contains the nucleotide sequence set forth in SEQ ID NO: 150:

5'- GCUUAAAAGGGACAGUAUUC$Z_7$ -3'(SEQ ID NO:51);

5'- $Z_8$GAAUACUGUCCCUUUUAAGCUU -3'(SEQ ID NO: 150),

wherein $Z_8$ is the first nucleotide at the 5' end of the antisense strand, $Z_7$ is selected from A, U, G or C, and $Z_8$ is a nucleotide complementary to $Z_7$.

In some embodiments, the sense strand of the siRNA contains the nucleotide sequence set forth in SEQ ID NO: 53, and the antisense strand of the siRNA contains the nucleotide sequence set forth in SEQ ID NO: 54:

5'- UUAAAAGGGACAGUAUUCU -3'(SEQ ID NO:53);

5'- AGAAUACUGUCCCUUUUAAGC -3'(SEQ ID NO:54),

or the sense strand of the siRNA contains the nucleotide sequence set forth in SEQ ID NO: 55, and the antisense strand of the siRNA contains the nucleotide sequence set forth in SEQ ID NO: 56:

5'- GCUUAAAAGGGACAGUAUUCU -3'(SEQ ID NO:55);

5'- AGAAUACUGUCCCUUUUAAGCAA -3'(SEQ ID NO:56).

In some embodiments, for the third siRNA, the first nucleotide sequence segment has the nucleotide sequence set forth in SEQ ID NO: 105, and the base composition of the third nucleotide sequence segment is AG. The sense strand of the siRNA contains the nucleotide sequence set forth in SEQ ID NO: 109, and the antisense strand contains the nucleotide sequence set forth in SEQ ID NO: 110:

5'- GGACAGUAUUCUCAGUGC$Z_{11}$ -3'(SEQ ID NO:109);

5'- $Z_{12}$GCACUGAGAAUACUGUCCCU -3'(SEQ ID NO:110),

or the sense strand of the siRNA contains the nucleotide sequence set forth in SEQ ID NO: 111, and the antisense strand contains the nucleotide sequence set forth in SEQ ID NO: 112:

5'- AGGGACAGUAUUCUCAGUGC$Z_{11}$ -3'(SEQ ID NO:111);

5'- $Z_{12}$GCACUGAGAAUACUGUCCCUUU -3'(SEQ ID NO:112),

wherein $Z_{12}$ is the first nucleotide at the 5' end of the antisense strand, $Z_{11}$ is selected from A, U, G or C, and $Z_{12}$ is a nucleotide complementary to $Z_{11}$.

In some embodiments, the sense strand of the siRNA contains the nucleotide sequence set forth in SEQ ID NO: 113, and the antisense strand of the siRNA contains the nucleotide sequence set forth in SEQ ID NO: 114:

5'- GGACAGUAUUCUCAGUGCU -3'(SEQ ID NO:113);

5'- AGCACUGAGAAUACUGUCCCU -3'(SEQ ID NO:114),

or the sense strand of the siRNA contains the nucleotide sequence set forth in SEQ ID NO: 115, and the antisense strand of the siRNA contains the nucleotide sequence set forth in SEQ ID NO: 116:

5'- AGGGACAGUAUUCUCAGUGCU -3'(SEQ ID NO:115);

5'- AGCACUGAGAAUACUGUCCCUUU -3'(SEQ ID NO:116).

As mentioned above, the nucleotides in the siRNA of the present disclosure are each independently modified or unmodified nucleotides. In some embodiments, some or all of the nucleotides in the siRNA of the present disclosure are modified nucleotides. These modifications on the nucleotide group will not lead to the obvious weakening or loss of the function of the siRNA of the present disclosure to inhibit the expression of the APOC3 gene.

In the context of the present disclosure, the term "modified nucleotide" as used refers to a nucleotide or nucleotide analog formed by substituting the hydroxyl group at the 2' position of the ribosyl group of a nucleotide with other groups,

or a nucleotide in which the base is a modified base. The modified nucleotides will not cause obvious weakening or loss of the function of the siRNA to inhibit gene expression. For example, the modified nucleotides disclosed in J.K. Watts, G.F. Deleavey, and M.J. Damha, Chemically modified siRNA: tools and applications. Drug Discov Today, 2008, 13(19-20): 842-55 may be selected.

In some embodiments, in the direction from the 5' end to the 3' end, if the 2nd, 6th, 14th, and 16th nucleotides in the nucleotide sequence II are not the stabilizing modified nucleotides, the same are 2'-fluoro-modified nucleotides. In some embodiments, all nucleotides in the nucleotide sequence II are modified nucleotides; in the direction from the 5' end to the 3' end, if the 2nd, 6th, 14th, and 16th nucleotides in the nucleotide sequence II are not the stabilizing modified nucleotides, the same are 2'-fluoro-modified nucleotides, and the other nucleotides in the nucleotide sequence II are each independently one of non-fluoro-modified nucleotides. In some embodiments, in the direction from the 5' end to the 3' end, the 7th-9th nucleotides in the nucleotide sequence I are 2'-fluoro-modified nucleotides. In some embodiments, all nucleotides in the nucleotide sequence I are modified nucleotides; in the direction from the 5' end to the 3' end, the 7th-9th nucleotides in the nucleotide sequence I are 2'-fluoro-modified nucleotides, and the other nucleotides in the nucleotide sequence I are each independently one of non-fluoro-modified nucleotides. With the modification, the siRNA of the present disclosure may achieve a good balance between gene expression regulation activity and *in vivo* stability.

In the context of the present disclosure, "fluoro-modified nucleotide" refers to a nucleotide formed by substituting the hydroxyl group at the 2' position of the ribosyl group of a nucleotide with fluorine, which has the structure represented by the following formula (7). "Non-fluoro-modified nucleotide" refers to a nucleotide or nucleotide analog formed by substituting the hydroxyl group at the 2' position of the ribosyl group of a nucleotide with a non-fluorine group. In some embodiments, each of the non-fluoro-modified nucleotides is independently selected from one of a nucleotide or nucleotide analog formed by substituting the hydroxyl group at the 2' position of the ribosyl group of a nucleotide with a non-fluorine group.

Nucleotides in which the hydroxyl at the 2' position of the ribosyl group is substituted with a non-fluorine group are well known to those skilled in the art. These nucleotides may be selected from 2'-alkoxy-modified nucleotides, 2'-alkyl-modified nucleotides, 2'-substituted alkyl-modified nucleotides, 2'-amino-modified nucleotides, 2'-substituted amino-modified nucleotides and 2'-deoxynucleotides.

In some embodiments, the 2'-alkoxy-modified nucleotide is a methoxy-modified nucleotide (2'-OMe), as represented by formula (8). In some embodiments, the 2'-amino-modified nucleotide (2'-$NH_2$) is represented by formula (9). In some embodiments, the 2'-deoxynucleotide (DNA) is represented by formula (10):

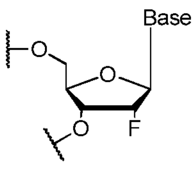 , 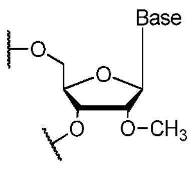 , 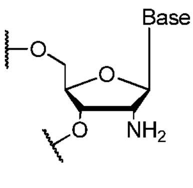 , 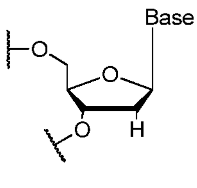 .

formula (7) formula (8) formula (9) formula (10)

The word "nucleotide analog" refers to a group capable of substituting the nucleotide in nucleic acid, but structurally different from adenine ribonucleotides, guanine ribonucleotides, cytosine ribonucleotides, uracil ribonucleotides, or thymus pyrimidine deoxyribonucleotides. In some embodiments, the nucleotide analog may be isonucleotide, bridged nucleic acid (BNA for short) or acyclic nucleotide.

A BNA refers to constrained or inaccessible nucleotide. A BNA may contain five-membered, six-membered, or seven-membered ring bridge structures with "fixed" C3'-endosugar constrictions. Typically, the bridge is incorporated at the 2'- and 4'-positions of the ribose to provide a 2',4'-BNA nucleotide. In some embodiments, BNA may be LNA, ENA, cET BNA, etc., wherein LNA is represented by formula (12), ENA is represented by formula (13), and cET BNA is represented by formula (14):

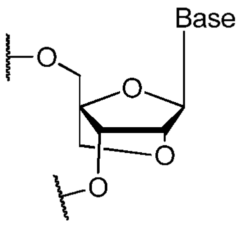 , 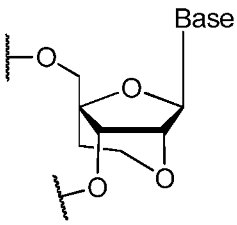 , 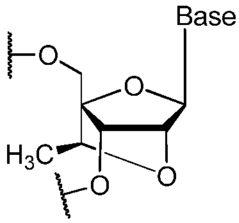 .

formula (12) formula (13) formula (14)

Acyclic nucleotide is a type of nucleotide formed by opening the sugar ring of the nucleotide. In some embodiments, the acyclic nucleotide may be unlocked nucleic acid (UNA) or glycerol nucleic acid (GNA), wherein UNA is represented by formula (15), and GNAis represented by formula (16):

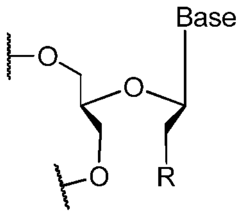 , 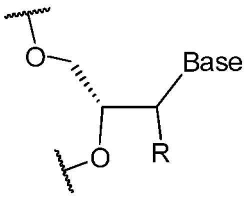 .

formula (15) formula (16)

In formula (15) and formula (16), R is selected from H, OH or alkoxy (O-alkyl).

Isonucleotide refers to a compound formed by changing the position of the base in the nucleotide on the ribose ring. In some embodiments, the isonucleotide may be a compound formed by moving a base from the 1'-position to the 2'-position or 3'-position of the ribose ring, as represented by formula (17) or (18).

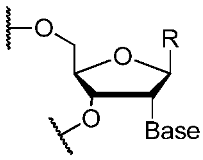 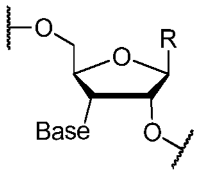

formula (17) formula (18)

In the compounds of formulas (17)-(18), Base represents a nucleic acid base, such as A, U, G, C or T; R is selected from H, OH, F or non-fluorine groups as described above.

In some embodiments, the nucleotide analog is selected from one of isonucleotides, LNA, ENA, cET, UNA and GNA. In some embodiments, each of the non-fluoro-modified nucleotides is a methoxy-modified nucleotide. In the text above and below, the methoxy-modified nucleotide refers to a nucleotide formed by substituting the 2'-hydroxyl of the ribosyl group with methoxy.

In the text above and below, "fluoro-modified nucleotide", "2'-fluorine-modified nucleotide", "nucleotide in which the 2'-hydroxyl group of the ribosyl group is substituted with fluorine" and "nucleotide with 2'-fluororibosyl group" have the same meaning, all of which refer to a compound which is formed by substituting the 2'-hydroxyl group of the nucleotide with fluorine and having a structure represented by formula (7); "methoxy-modified nucleotide", "2'-methoxy-modified nucleotide", "nucleotide in which the 2'-hydroxyl group of the ribosyl group is substituted with methoxy" and "nucleotide with 2'-methoxyribosyl group" have the same meaning, all of which refer to a compound which is formed by substituting the 2'-hydroxyl group of the ribosyl group of the nucleotide with methoxy and having a structure represented by formula (8).

In some embodiments, the siRNA containing the stabilizing modified nucleotide of the present disclosure is an siRNA having the following modifications: in the direction from the 5' end to the 3' end, in the sense strand, the nucleotides at the 7th, 8th, and 9th or 5th, 7th, 8th, and 9th positions of the nucleotide sequence I are fluoro-modified nucleotides,

and the nucleotides at the remaining positions in the sense strand are methoxy-modified nucleotides; in the antisense strand, the nucleotides at the 2nd, 6th, 14th, 16th or 2nd, 6th, 8th, 9th, 14th, 16th positions of the nucleotide sequence II are fluoro-modified nucleotides, the nucleotide at the 3rd or 5th position in the antisense strand is a stabilizing modified nucleotide, and the nucleotides at the remaining positions in the antisense strand are methoxy-modified nucleotides.

The siRNA with the modification above is not only low in cost, but also makes it difficult for ribonuclease in the blood to cut the siRNA, thereby increasing the stability of the siRNA and making it have stronger resistance to nuclease hydrolysis. Meanwhile, the modification above reduces the off-target effect of the siRNA without significantly reducing the inhibition performance of the siRNA.

In some embodiments, the siRNA provided by the present disclosure is one of siAPOC3a1-M1, siAPOC3a1-M2, siAPOC3a2-M1, siAPOC3a2-M2, siAPOC3b1-M1, siAPOC3b1-M2, siAPOC3b2-M1, siAPOC3b2-M2, siAPOC3b3-M1, siAPOC3b3-M2, siAPOC3b4-M1, siAPOC3b4-M2, siAPOC3c1-M1, siAPOC3c1-M2, siAPOC3c2-M1, and siAPOC3c2-M2.

In some embodiments, at least part of the phosphoester groups in the phosphate-sugar backbone of at least one single strand in the sense strand and the antisense strand of the siRNA provided by the present disclosure are phosphoester groups with a modified group. In some embodiments, the phosphate group with the modified group is a phosphorothioate group formed by substituting at least one oxygen atom in a phosphodiester bond in the phosphate group with a sulfur atom. In some embodiments, the phosphate group with the modified group is a phosphorothioate group with a structure represented by formula (1):

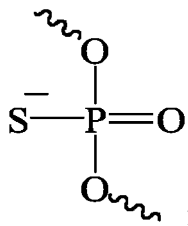
formula (1).

This modification may stabilize the double-stranded structure of the siRNA and maintain high specificity and high affinity of base pairing.

In some embodiments, in the siRNA provided by the present disclosure, the phosphorothioate group linkage exists in at least one of the following positions: between the first and second nucleotides at either end of the sense strand or the antisense strand; between the second and third nucleotides at either end of the sense strand or the antisense strand; or any combination of the above. In some embodiments, the phosphorothioate group linkage exists in all of the above positions except the 5' end of the sense strand. In some embodiments, the phosphorothioate group linkage exists in all of the above positions except the 3' end of the sense strand. In some embodiments, the phosphorothioate group linkage exists in at least one of the following positions:

between the 1st nucleotide and the 2nd nucleotide at the 5' end of the sense strand;

between the 2nd nucleotide and the 3rd nucleotide at the 5' end of the sense strand;

between the 1st nucleotide and the 2nd nucleotide at the 3' end of the sense strand;

between the 2nd nucleotide and the 3rd nucleotide at the 3' end of the sense strand;

between the 1st nucleotide and the 2nd nucleotide at the 5' end of the antisense strand;

between the 2nd nucleotide and the 3rd nucleotide at the 5' end of the antisense strand;

between the 1st nucleotide and the 2nd nucleotide at the 3' end of the antisense strand; and

between the 2nd nucleotide and the 3rd nucleotide at the 3' end of the antisense strand.

In some embodiments, the siRNA provided by the present disclosure is one of siAPOC3a1-M1S, siAPOC3a1-M2S, siAPOC3a2-M1S, siAPOC3a2-M2S, siAPOC3b1-M1S, siAPOC3b1-M2S, siAPOC3b2-M1S, siAPOC3b2-M2S, siAPOC3b3-M1S, siAPOC3b3-M2S, siAPOC3b4-M1S, siAPOC3b4-M2S, siAPOC3c1-M1S, siAPOC3c1-M2S, siAPOC3c2-M1S, and siAPOC3c2-M2S as listed in Table 1a to Table 1c.

In some embodiments, a 5' end nucleotide of the antisense strand of the siRNA is a 5'-phosphate nucleotide or a nucleotide modified by 5'-phosphate analog.

Commonly used 5'-phosphate nucleotides or 5'-phosphate analog-modified nucleotides are well known to those skilled in the art, for example, the 5'-phosphate nucleotides may have the following structure:

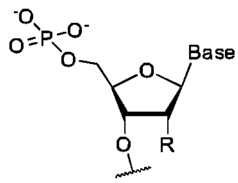

formula (2);

In another example, Anastasia Khvorova and Jonathan K. Watts, The chemical evolution of oligonucleotide therapies of clinical utility. Nature Biotechnology, 2017,35(3):238-48 discloses the following four 5'-phosphate analog modified nucleotides:

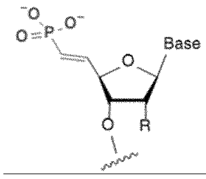

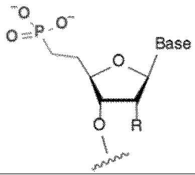

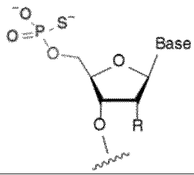

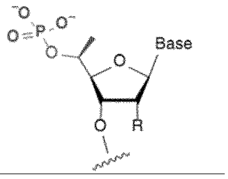

formula (3) formula (4) formula (5) formula (6)

wherein R is selected from H, OH, methoxy, fluorine; and the base represents a nucleic acid base, selected from A, U, C, G or T.

In some embodiments, the 5'-phosphate nucleotide is a nucleotide containing a 5'-phosphate modified nucleotide represented by formula (2), and the 5'-phosphate analog-modified nucleotide is a vinylphosphate (5'-(E)-vinylphosphonate, E-VP) modified nucleotide as represented by formula (3), or phosphorothioate modified nucleotide as represented by formula (5).

In some embodiments, the siRNA of the present disclosure is one of siAPOC3a1-M1P1, siAPOC3a1-M2P1, siAPOC3a2-M1P1, siAPOC3a2-M2P1, siAPOC3a1-M1SP1, siAPOC3a1-M2SP1, siAPOC3a2-M1SP1, siAPOC3a2-M2SP1, siAPOC3b1-M1P1, siAPOC3b1-M2P1, siAPOC3b2-M1P1, siAPOC3b2-M2P1, siAPOC3b1-M1SP1, siAPOC3b1-M2SP1, siAPOC3b2-M1SP1, siAPOC3b2-M2SP1, siAPOC3b3-M1P1, siAPOC3b3-M2P1, siAPOC3b4-M1P1, siAPOC3b4-M2P1, siAPOC3b3-M1SP1, siAPOC3b3-M2SP1, siAPOC3b4-M1SP1, siAPOC3b4-M2SP1, siAPOC3c1-M1P1, siAPOC3c1-M2P1, siAPOC3c2-M1P1, siAPOC3c2-M2P1, siAPOC3c1-M1SP1, siAPOC3c1-M2SP1, siAPOC3c2-M1SP1, and siAPOC3c2-M2SP1 as listed in Table 1a to Table 1c.

The inventors of the present disclosure unexpectedly found that the siRNA provided by the present disclosure not only has significantly enhanced plasma and lysosome stability and significantly low off-target effects, but also retains high gene inhibition activity.

The siRNA provided by the present disclosure may be obtained by conventional siRNA preparation methods (such as solid-phase synthesis and liquid-phase synthesis methods) in the art. Among them, the solid-phase synthesis has commercialized customized services. The modified nucleotide groups may be introduced into the siRNA of the present disclosure by using nucleoside monomers with corresponding modifications. The method for preparing the nucleoside monomers with corresponding modifications and the method for introducing the modified nucleotide groups into the siRNA are also well known to those skilled in the art.

Pharmaceutical composition

The present disclosure provides a pharmaceutical composition, containing the siRNA as described above as an active ingredient and a pharmaceutically acceptable carrier.

The pharmaceutically acceptable carrier may be a carrier commonly used in the field of siRNA administration, such as but not limited to magnetic nanoparticles (such as nanoparticles based on $Fe_3O_4$ or $Fe_2O_3$), carbon nanotubes, mesoporous silicon, calcium phosphate nanoparticles, polyethyleneimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid)copolymer (PLGA), poly(2-aminoethylethylene phosphate) (PPEEA) and poly(2-dimethylaminoethyl methacrylate) (PDMAEMA) as well as derivatives thereof.

In some embodiments, in the pharmaceutical composition, there is no special requirement on the contents of the siRNA and the pharmaceutically acceptable carrier. In some embodiments, the weight ratio of the siRNA to the pharmaceutically acceptable carrier may be 1:(1-500), and in some embodiments, the weight ratio described above is 1:(1-50).

In some embodiments, the pharmaceutical composition may further comprise other pharmaceutically acceptable excipients, which may be one or more of various preparations or compounds routinely used in the art. For example, the other pharmaceutically acceptable excipients may include at least one of a pH buffer, a protective agent and an osmotic pressure regulator.

The pH buffer may be a Tris hydrochloride buffer with a pH value of 7.5-8.5 and/or a phosphate buffer with a pH value of 5.5-8.5, for example, a phosphate buffer with a pH value of 5.5-8.5.

The protective agent may be at least one of inositol, sorbitol, sucrose, trehalose, mannose, maltose, lactose and glucose. Based on the total weight of the pharmaceutical composition, the content of the protective agent may be 0.01-30 wt%.

The osmotic pressure regulator may be sodium chloride and/or potassium chloride. The content of the osmotic pressure regulator makes the osmotic pressure of the pharmaceutical composition 200-700 milliosmol/kg (mOsm/kg). According to the desired osmotic pressure, those skilled in the art may easily determine the content of the osmotic pressure regulator. In some embodiments, the dose of the preparation made from the pharmaceutical composition will be adjusted due to different administration methods during administration.

In some embodiments, the pharmaceutical composition may be a liquid preparation, such as an injection; it may also be a freeze-dried powder injection, which is mixed with a liquid excipient during administration to prepare a liquid preparation. The liquid preparation may be used for subcutaneous, intramuscular or intravenous injection administration, but may also be administered to the lungs by spraying, or administered to other organs (such as the liver) through the lungs by spraying, or deliver the pharmaceutical composition through oropharyngeal inhalation or nasal administration. In some embodiments, the pharmaceutical composition is for spray administration.

In some embodiments, the pharmaceutical composition may be in the form of a liposomal formulation. In some embodiments, the pharmaceutically acceptable carrier used in the liposome formulation comprises an amine-containing transfection compound (hereinafter also referred to as an organic amine), a helper lipid and/or a PEGylated lipid. The organic amine, helper lipid and pegylated lipid may be selected from one or more of the amine-containing transfection compounds described in Chinese patent application CN103380113A (which is incorporated herein by reference in its entirety) or pharmaceutically acceptable salts or derivatives, helper lipids and PEGylated lipids thereof.

In some embodiments, the organic amine may be a compound represented by formula (201) described in Chinese patent application CN103380113A or a pharmaceutically acceptable salt thereof:

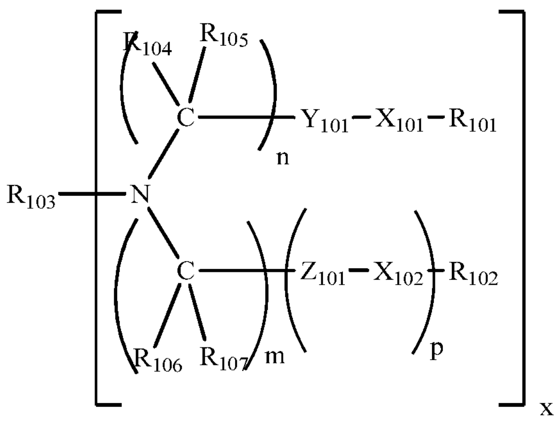

formula (201),

wherein:

$X_{101}$ and $X_{102}$ are each independently O, S, N-A or C-A, wherein A is hydrogen or a $C_1$-$C_{20}$ hydrocarbon chain;

$Y_{101}$ and $Z_{101}$ are each independently C=O, C=S, S=O, CH-OH or SOz;

$R_{101}$, $R_{102}$, $R_{103}$, $R_{104}$, $R_{105}$, $R_{106}$ and $R_{107}$ are each independently hydrogen, cyclic or acyclic, substituted or unsubstituted, branched or straight-chain aliphatic group, cyclic or acyclic, substituted or unsubstituted, branched

or straight-chain heteroaliphatic, substituted or unsubstituted, branched or straight-chain acyl, substituted or unsubstituted, branched or straight-chain aryl, substituted or unsubstituted, branched or straight-chain heteroaryl;

x is an integer of 1 to 10;

n is an integer of 1-3, m is an integer of 0-20, p is 0 or 1; wherein if m = p = 0, $R_{102}$ is hydrogen;

and, if at least one of n or m is 2, $R_{103}$ and nitrogen in formula (201) form a structure as represented by formula (202) or formula (203):

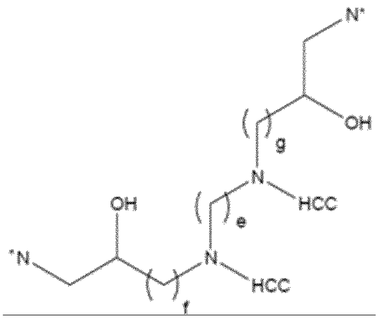 formula (202), 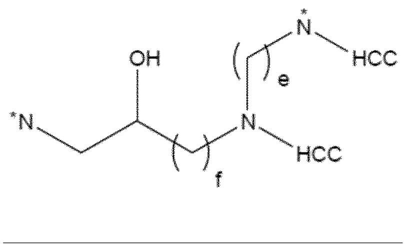 formula (203);

wherein g, e, and f are each independently an integer of 1-6, "HCC" represents a hydrocarbon chain, and each *N represents a nitrogen atom in formula (201).

In some embodiments, $R_{103}$ is a polyamine. In other embodiments, $R_{103}$ is a ketal. In some embodiments, each of $R_{101}$ and $R_{102}$ in formula (201) is independently any substituted or unsubstituted, branched or straight-chain alkyl or alkenyl, the alkyl or alkenyl group has 3 to about 20 carbon atoms, for example, 8 to about 18 carbon atoms, and 0 to 4 double bonds, for example, 0 to 2 double bonds.

In some embodiments, if each of n and m independently has a value of 1 or 3, $R_{103}$ may be any of formulas (204)-(213):

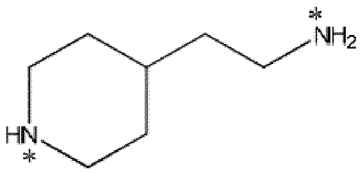 formula (204), 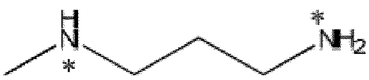 formula (205),

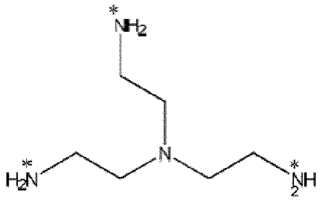 formula (206), 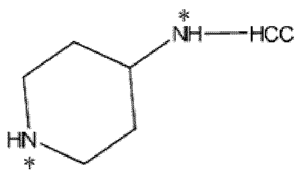 formula (207),

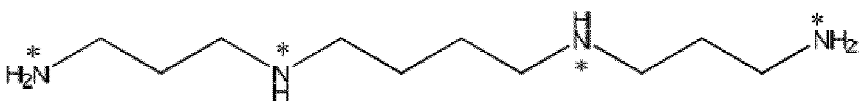 formula (208),

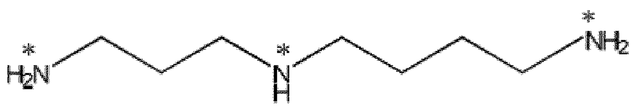 formula (209),

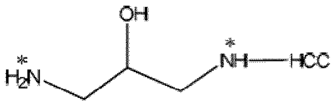 formula (210), 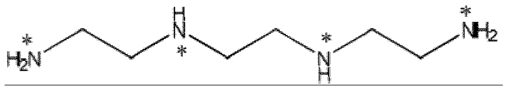 formula (211),

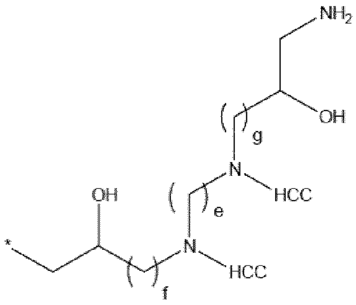 formula (212) and 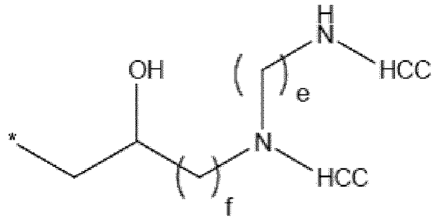 formula (213);

wherein in formula (204)-formula (213), g, e and f are each independently an integer of 1-6, each "HCC" represents a hydrocarbon chain, and each * shows possible points of attachment of $R_{103}$ to the nitrogen atom in formula (201), wherein each H at any * position may be substituted to achieve linkage to the nitrogen atom in formula (201).

The compound represented by formula (201) may be prepared according to the description in Chinese patent application CN103380113A.

In some embodiments, the organic amine is an organic amine represented by formula (214) and/or an organic amine represented by formula (215):

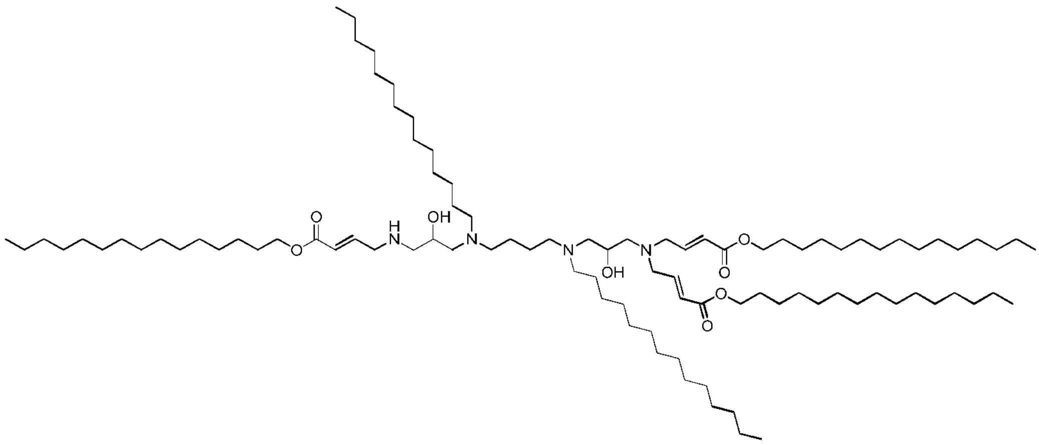 formula (214),

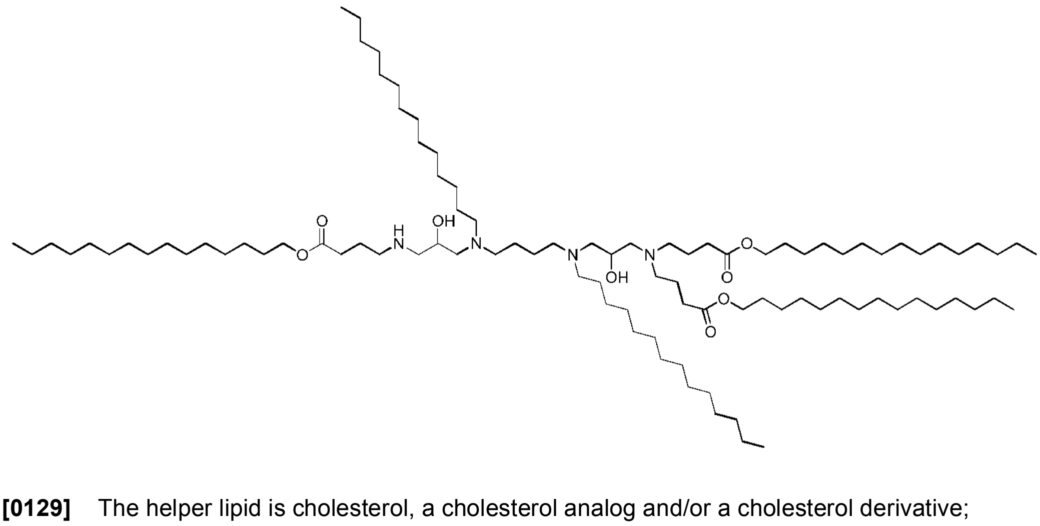 formula (215);

The helper lipid is cholesterol, a cholesterol analog and/or a cholesterol derivative;

the PEGylated lipid is 1,2-dipalmitamide-sn-glycerol-3-phosphatidylethanolamine-*N*-[methoxy(polyethylene glycol)]-2000.

In some embodiments, in the pharmaceutical composition, the molar ratio among the organic amine, the helper lipid and the PEGylated lipid is (19.7-80):(19.7-80):(0.3-50), such as (50-70):(20-40):(3-20).

In some embodiments, the particles of the pharmaceutical composition formed from the siRNA of the present disclosure and the amine-containing transfection reagent described above have an average diameter of about 30 nm to about 200 nm, typically about 40 nm to about 135 nm; more typically, the average diameter of the liposome particles is about 50 nm to about 120 nm, about 50 nm to about 100 nm, about 60 nm to about 90 nm or about 70 nm to about 90 nm. For example, the average diameter of the liposome particles is about 30, 40, 50, 60, 70, 75, 80, 85, 90, 100, 110, 120, 130, 140, 150 or 160 nm.

In some embodiments, in the pharmaceutical composition formed from the siRNA of the present disclosure and the amine-containing transfection reagent, the weight ratio (weight/weight ratio) of the siRNA to all lipids (such as the organic amines, helper lipids and/or PEGylated lipids) is within the range of from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12 or from about 1:6 to about 1:10. For example, the weight ratio of the siRNA of the present disclosure to all lipids is about 1:5, 1:6, 1:7. 1:8, 1:9, 1:10, 1:11, 1:12, 1:13, 1:14, 1:15, 1:16, 1:17, or 1:18.

In some embodiments, each component of the pharmaceutical composition may exist independently when sold, and may exist in the form of a liquid formulation when used. In some embodiments, the pharmaceutical composition formed from the siRNA provided by the present disclosure and the pharmaceutically acceptable carrier described above may be prepared according to various known methods, and may be formed by substituting the existing siRNA with the siRNA provided by the present disclosure. In some embodiments, the composition may be prepared as follows:
the organic amine, helper lipid and PEGylated lipid are suspended in the alcohol according to the molar ratio and mixed to obtain a lipid solution, wherein the amount of alcohol is such that the total mass concentration of the obtained lipid solution is 2-25 mg/mL, for example, it may be 8-18 mg/mL.

The alcohol is selected from pharmaceutically acceptable alcohols, such as alcohols that are liquid at about room temperature, for example, one or more of ethanol, propylene glycol, benzyl alcohol, glycerin, polyethylene glycol 200, polyethylene glycol 300, polyethylene glycol 400, for example, it may be ethanol.

The siRNA provided by the present disclosure is dissolved in a buffered saline solution to obtain an siRNA aqueous solution. The concentration of the buffered saline solution is 0.05-0.5 M, for example, 0.1-0.2 M; the pH of the buffered saline solution is adjusted to 4.0-5.5, for example, 5.0-5.2; the amount of the buffered saline solution makes the concentration of the siRNA do not exceed 0.6 mg/mL, for example, 0.2-0.4 mg/mL. The buffer salt is selected from one or more of soluble acetate and soluble citrate, for example, sodium acetate and/or potassium acetate.

The lipid solution and the siRNA aqueous solution are mixed, and the mixed product is incubated at 40-60 °C for at least 2 min, for example, 5-30 min, to obtain an incubated liposome preparation. The volume ratio of the lipid solution to the siRNA aqueous solution is 1 :(2-5), for example, 1:4.

The incubated liposome preparation is concentrated or diluted, impurities are removed, and the preparation is sterilized to obtain the pharmaceutical composition provided by the present disclosure, whose physical and chemical parameters are pH of 6.5-8, encapsulation efficiency of not less than 80%, particle size of 40-200 nm, polydispersity index of not higher than 0.30, and osmotic pressure of 250-400 mOsm/kg. For example, the physical and chemical parameters may be pH of 7.2-7.6, encapsulation efficiency of not less than 90%, particle size of 60-100nm, polydispersity index of not higher than 0.20, and osmotic pressure of 300-400 mOsm/kg.

The concentration or dilution may be performed before, after or simultaneously with the removal of impurities. Various existing methods may be used to remove impurities, for example, a tangential flow system and a hollow fiber column may be used for ultrafiltration at 100K Da, wherein the ultrafiltration exchange solution is phosphate buffered saline (PBS) with pH of 7.4. Various existing methods may be used for the sterilization, for example, filtration sterilization on a 0.22 $\mu$m filter may be used.

<u>siRNA conjugate</u>

The present disclosure provides an siRNA conjugate, comprising the siRNA provided by the present disclosure and a conjugated group conjugated to the siRNA. In some embodiments, the conjugated group comprises a linker and a pharmaceutically acceptable targeting group and/or a delivery auxiliary group, and the siRNA, the linker and the targeting group or the delivery auxiliary group are sequentially linked covalently or non-covalently; each of the targeting groups is selected from ligands capable of binding to a cell surface receptor, and each delivery auxiliary group is selected from groups capable of increasing the biocompatibility of the siRNA conjugate in a delivery target organ or tissue.

In the context of the present disclosure, unless otherwise stated, "conjugation" means that two or more chemical moieties each having a specific function are covalently linked to each other; accordingly, "conjugate" refers to a compound

formed by covalent linkage between the various chemical moieties. Further, "siRNA conjugate" refers to a compound formed by covalently linking one or more chemical moieties with specific functions to an siRNA. The siRNA should be understood as a general term of multiple siRNA conjugates or an siRNA conjugate represented by a certain chemical formula according to the context. In the context of the present disclosure, a "conjugate molecule" should be understood as a specific compound that may be conjugated to an siRNA through a reaction, ultimately forming an siRNA conjugate of the present disclosure.

Generally, the conjugated group comprises at least one pharmaceutically acceptable targeting group and optionally a linker, and the siRNA, the linker and the targeting group are linked sequentially. In some embodiments, the number of the targeting group is 1-6. In some embodiments, the number of the targeting group is 2-4. The siRNA molecule may be non-covalently or covalently conjugated to the conjugated group. For example, the same may be covalently conjugated to the conjugated group. The conjugation site between the siRNA and the conjugated group may be at the 3' end or 5' end of the sense strand of the siRNA, or at the 5' end of the antisense strand, or in the internal sequence of the siRNA. In some embodiments, the conjugation site between the siRNA and the conjugated group is at the 3' end of the sense strand of the siRNA.

In some embodiments, the conjugated group may be linked to the phosphate group, the 2'-position hydroxyl or the base of the nucleotide. In some embodiments, the conjugated group may be linked to the 3'-position hydroxyl, and at this time, the nucleotides are linked by a 2'-5' phosphodiester bond linkage. When the conjugated group is linked to the end of the siRNA chain, the conjugated group is usually linked to the phosphate group of the nucleotide. When the conjugated group is linked to the internal sequence of the siRNA, the conjugated group is usually linked to the ribose sugar ring or the base. For various linkage methods, please refer to Muthiah Manoharan et.al. siRNA conjugates carrying sequentially assembled trivalent N-acetylgalactosamine linked through nucleotides elicit robust gene silencing *in vivo* in hepatocytes.ACS Chemical biology,2015,10(5):1181-7.

The targeting group may be linked to the siRNA molecule through a suitable linker, and those skilled in the art may select a suitable linker according to the specific type of the targeting group. The types of these linkers, targeting groups and the linkage methods with the siRNA may be found in the disclosure of WO2015006740A2, the entire contents of which are incorporated herein by reference.

In some embodiments, the targeting group may be a ligand commonly used in the field of siRNA administration, such as various ligands described in WO2009082607A2, the entire disclosure of which is incorporated herein by reference.

In some embodiments, at least one or each of the targeting groups is selected from ligands capable of binding to receptors on the surfaces of cells expressing the APOC3 gene.

In some embodiments, at least one or each of the targeting groups is selected from ligands capable of binding to receptors on the surfaces of mammalian hepatocytes (ASGPR). In some embodiments, each of the targeting groups is independently a ligand that has an affinity for an asialoglycoprotein receptor on the surface of mammalian hepatocytes. In some embodiments, each of the targeting groups is independently an asialoglycoprotein or a sugar. In some embodiments, each of the targeting groups is independently an asialoglycoprotein, such as asialoorosomucoid (ASOR) or asialofetuin (ASF). In some embodiments, each of the targeting groups is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucopyranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, 2,3,4-tri-O-acetyl-1-thio-6-O-trityl-α-D-glucopyranoside methyl ester, 4-thio-β-D-galactopyranose, 3,4,6,7-tetra-O-acetyl-2-deoxy-1,5-dithio-α-D-glucopyranoside ethyl ester, 2,5-anhydro-D-allose nitrile, ribose, D-ribose, D-4-thioribose, L-ribose, and L-4-thioribose. In some embodiments, at least one or each of the targeting groups is galactose or N-acetylgalactosamine.

In some embodiments, the linker in the siRNA conjugate of the present disclosure has a structure as represented by formula (301):

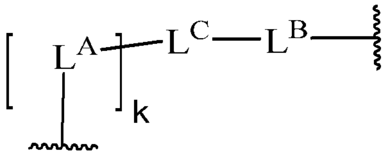

formula (301),

wherein k is an integer of 1 to 3;

$L^A$ has a structure containing an amide bond as represented by formula (302), $L^B$ has a structure containing N-acylpyrrolidine as represented by formula (303), containing carbonyl and oxygen atoms, and $L^C$ is a linking group based on hydroxymethylaminomethane, dimethylolaminomethane or trishydroxymethylaminomethane;

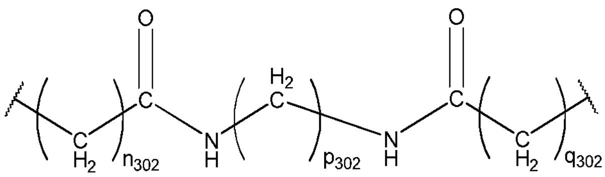 formula (302);

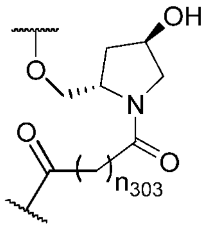

formula (303);

wherein $n_{302}$, $q_{302}$ and $p_{302}$ are each independently an integer of 2-6, and optionally, $n_{302}$, $q_{302}$ and $p_{302}$ are each independently 2 or 3; and $n_{303}$ is an integer of 4-16, and optionally, $n_{303}$ is an integer of 8-12. - indicates the point at which the group is covalently linked.

In the linker, each $L^A$ is linked to one of the targeting groups through an ether bond, and is linked to the $L^C$ part through the oxygen atom of the carbonyl group in the $L^C$ part by forming an ether bond. $L^B$ is linked to the nitrogen atom in the $L^C$ part through the carbonyl group in formula (303) by forming an amido bond, and is linked to the siRNA through the oxygen atom in formula (303) by forming a phosphoester bond or a phosphorothioate bond.

In some embodiments, the siRNA conjugate provided by the present disclosure has a structure as represented by formula (305):

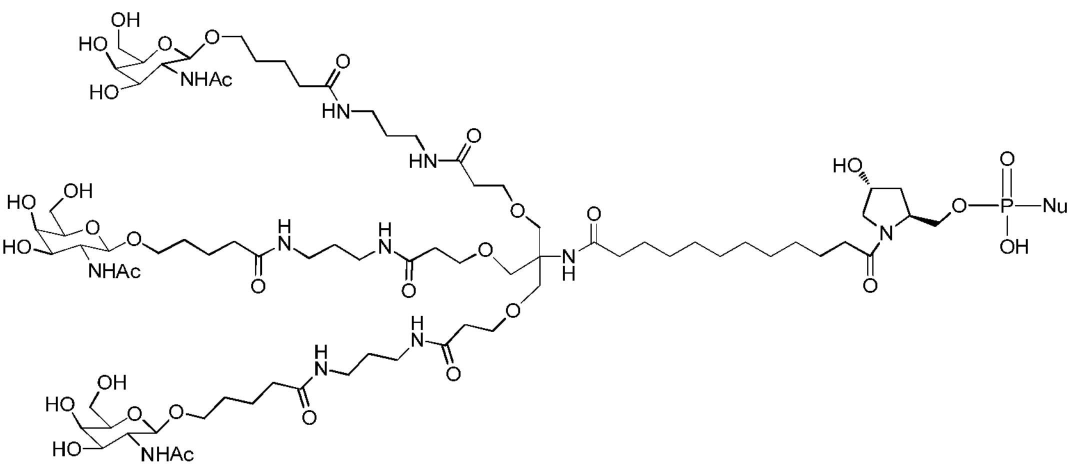

,

formula (305)

wherein Nu represents the siRNA provided by the present disclosure.

In some embodiments, the linker in the siRNA conjugate of the present disclosure has a structure as represented by formula (306):

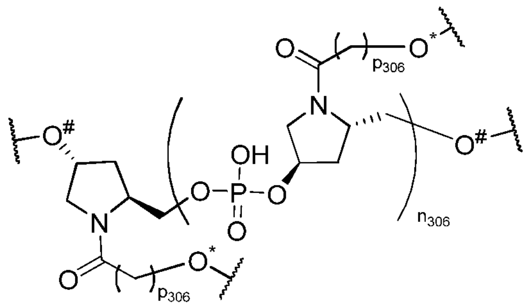

formula (306),

wherein $n_{306}$ is an integer of 0-3, each $p_{306}$ is independently an integer of 1-6, and represents the site where the group is covalently linked; the linking group forms a ether bond linkage to the targeting group through the oxygen atom marked by *. The linking group forms a phosphoester bond or a phosphorothioate bond linkage through at least one of the oxygen atoms marked by # with the siRNA, and the rest are linked to a hydrogen atom through the oxygen atom marked by # to form a hydroxyl, or linked to a Ci-Cs alkyl group to form a Ci-Cs alkoxy group.

In some embodiments, the siRNA conjugate of the present disclosure has a structure as represented by formula (307):

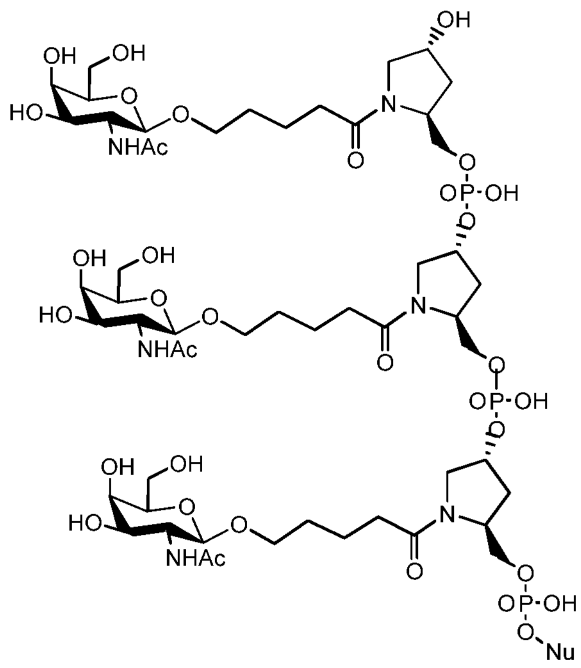

formula (307)

wherein Nu represents the siRNA provided by the present disclosure.

In some embodiments, the siRNA conjugate of the present disclosure has a structure as represented by formula (308):

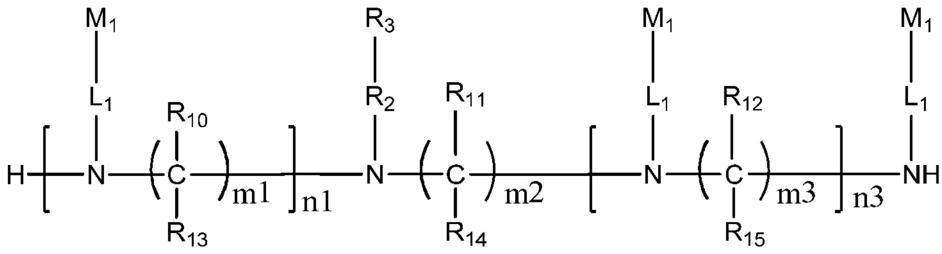

formula (308),

wherein,

n1 is an integer selected from 1 to 3, and n3 is an integer selected from 0 to 4;

each m1, m2, or m3 is independently an integer selected from 2 to 10;

$R_{10}$, $R_{11}$, $R_{12}$, $R_{13}$, $R_{14}$ or $R_{15}$ are each independently H, or are selected from the group consisting of the following groups: $C_1$-$C_{10}$ alkyl, $C_1$-$C_{10}$ haloalkyl and $C_1$-$C_{10}$ alkoxy;

$R_3$ has a structure represented by formula A59:

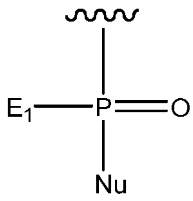

(A59)

wherein Ei is OH, SH or $BH_2$, and Nu represents the siRNA provided by the present disclosure;

$R_2$ is a straight-chain alkylene group with a length of 1-20 carbon atoms, wherein one or more carbon atoms are optionally replaced by any one or more selected from the group consisting of the following groups: C(O), NH, O, S, CH=N, S(O)z, $C_2$-$C_{10}$ alkenylene, $C_2$-$C_{10}$ alkynylene, $C_6$-$C_{10}$ arylene, $C_3$-$C_{18}$ heterocyclylene and $C_5$-$C_{10}$ heteroarylene; and wherein $R_2$ may optionally have any one or more substituents from the group consisting of the following groups: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituents, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -COzH, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl),-C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -SOz(phenyl), -$SO_2$($C_1$-$C_{10}$ haloalkyl), -$SO_2NH_2$, -$SO_2$NH($C_1$-$C_{10}$ alkyl),-SOzNH(phenyl), -$NHSO_2$($C_1$-$C_{10}$ alkyl), -NHSOz(phenyl) and -$NHSO_2$($C_1$-$C_{10}$ haloalkyl);

each Li is independently a straight-chain alkylene group with a length of 1-70 carbon atoms, wherein one or more carbon atoms are optionally replaced by any one or more selected from the group consisting of the following groups: C(O), NH, O, S, CH=N, S(O)z, $C_2$-$C_{10}$ alkenylene, $C_2$-$C_{10}$ alkynylene, $C_6$-$C_{10}$ arylene, $C_3$-$C_{18}$ heterocyclylene and $C_5$-$C_{10}$ heteroarylene; and wherein Li may optionally have any one or more substituents from the group consisting of the following groups: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl,-$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituents, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -COzH, -C(O)O($C_1$-$C_{10}$ alkyl),

-CON($C_1$-$C_{10}$ alkyl)($C_1$-$C_{10}$ alkyl), -CONH($C_1$-$C_{10}$ alkyl),-$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -SOz(phenyl), -$SO_2$($C_1$-$C_{10}$ haloalkyl), -SOzNHz, -$SO_2$NH($C_1$-$C_{10}$ alkyl), -SOzNH(phenyl), -NHSOz ($C_1$-$C_{10}$ alkyl), -NHSOz(phenyl) and -$NHSO_2$($C_1$-$C_{10}$ haloalkyl);



indicates the site where the group is covalently linked;

$M_1$ represents a targeting group, and its definition and optional range are the same as above. In some embodiments, each $M_1$ is independently selected from one of the ligands that have an affinity for the asialoglycoprotein receptor on the surface of mammalian hepatocytes.

Those skilled in the art will appreciate that although Li is defined as a straight-chain alkyl group for convenience, it may not be a linear group or have a different name, such as amine or alkenyl as a result of the substitutions and/or substitutions described above. For the purposes of the present disclosure, the length of Li is the number of atoms in the chain connecting the two points of attachment. For such purpose, a ring obtained by substituting a carbon atom of the straight-chain alkylene group, such as a heterocyclylene or heteroarylene, is counted as one atom.

When $M_1$ is a ligand having affinity for asialoglycoprotein receptors on the surface of mammalian hepatocytes, in some embodiments, n1 may be an integer from 1 to 3, and n3 may be an integer from 0 to 4, such that the number of $M_1$ ligands in the conjugate is at least 2. In some embodiments, $n1+ n3 \geq 2$, so that the number of $M_1$ ligands is at least 3, and the $M_1$ ligands more readily bind to the hepatic surface asialoglycoprotein receptor, which in turn facilitates the entry of the conjugate into the cell by endocytosis. Experiments have shown that when the number of $M_1$ ligands is greater than 3, the ease of binding of $M_1$ ligands to asialoglycoprotein receptors on the liver surface does not increase significantly. Therefore, from the perspective of ease of synthesis, structure/process cost and delivery efficiency and other aspects into consideration, in some embodiments, n1 is an integer of 1-2, n3 is an integer of 0-1, and n1 + n3 = 2-3.

In some embodiments, when m1, m2, and m3 are independently selected from an integer of 2-10, the spatial positions between multiple $M_1$ ligands may be suitable for the binding of $M_1$ ligands to asialoglycoprotein receptors on the liver surface. In order to make the conjugates provided by the present disclosure simpler, easier to synthesize and/or reduce costs, in some embodiments, m1, m2 and m3 are each independently an integer of 2-5, and in some embodiments, m1 = m2 = m3.

Those skilled in the art will understand that when $R_{10}$, $R_{11}$, $R_{12}$, $R_{13}$, $R_{14}$ and $R_{15}$ are each independently selected from one of H, $C_1$-$C_{10}$ alkyl, $C_1$-$C_{10}$ haloalkyl, and $C_1$-$C_{10}$ alkoxy, the purpose of the present disclosure may be achieved without changing the properties of the conjugates disclosed herein. In some embodiments, $R_{10}$, $R_{11}$, $R_{12}$, $R_{13}$, $R_{14}$, and $R_{15}$ are each independently selected from H, methyl, and ethyl. In some embodiments, $R_{10}$, $R_{11}$, $R_{12}$, $R_{13}$, $R_{14}$, and $R_{15}$ are all H.

According to the siRNA conjugate provided by the present disclosure, $R_3$ is a group with the structure represented by formula A59, wherein Ei is OH, SH or BHz. On the basis of the consideration of the availability of raw materials for preparation, Ei is OH or SH in some embodiments.

In some embodiments, $R_2$ is selected to achieve linkage to N and A59 on the nitrogen-containing backbone. In the context of the present disclosure, "nitrogen-containing skeleton" refers to a chain structure in which carbon atoms linked to $R_{10}$, $R_{11}$, $R_{12}$, $R_{13}$, $R_{14}$ and $R_{15}$ are linked to N. Thus, $R_2$ may be any linking group capable of linking the A59 group to the N of the nitrogen-containing backbone in an appropriate manner. In some embodiments, in the case of preparing the siRNA conjugate of the present disclosure by a solid-phase synthesis process, the $R_2$ group needs to contain both the linking site linked to the N on the nitrogen-containing backbone and the linking site linked to the P in the $R_3$. In some embodiments, the site linked to N on the nitrogen-containing skeleton in the $R_2$ group forms an amide bond with N, and the site linked to P on the $R_3$ group forms a phosphoester bond with P. In some embodiments, $R_2$ is B5, B6, B5' or B6':

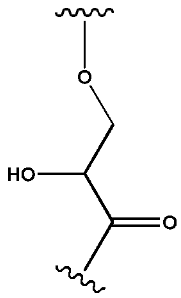,
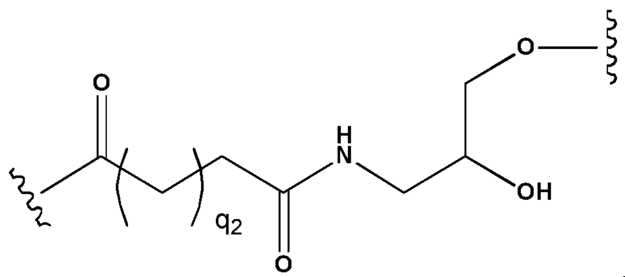,

(B5) (B6)

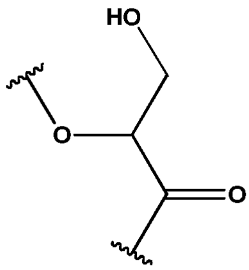,
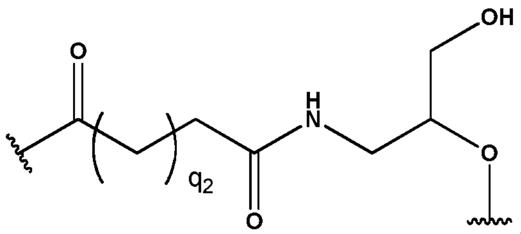;

(B5') (B6')

wherein 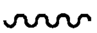 represents the site where the group is covalently linked.

The value range of $q_2$ may be an integer of 1-10, and in some embodiments, $q_2$ is an integer of 1-5.

The function of Li is to link the $M_1$ ligand to N on the nitrogen-containing backbone, providing a targeting function for the siRNA conjugate of the present disclosure. In some embodiments, Li is selected from a combination of one or more of the groups in formulas A1-A26. In some embodiments, Li is selected from one of or a combination of two or more of the linkages A1, A4, A5, A6, A8, A10, A11 and A13. In some embodiments, Li is selected from a combination of at least 2 of the linkages A1, A4, A8, A10 and A11. In some embodiments, Li is selected from a combination of at least 2 of the linkages A1, A8, and A10.

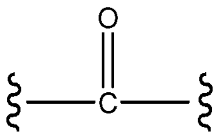,
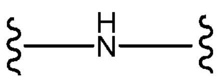,
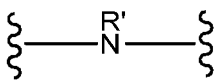,
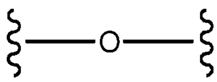,

(A1) (A2) (A3) (A4)

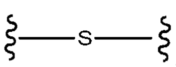,
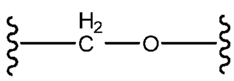,
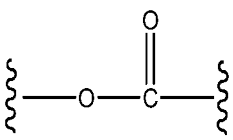,
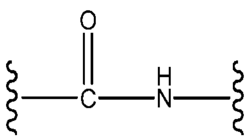,

(A5) (A6) (A7) (A8)

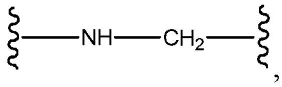, 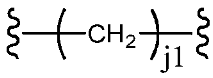, 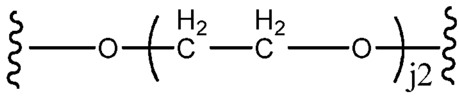,

(A9) (A10) (A11)

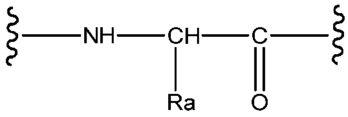, 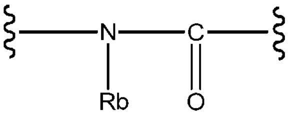, 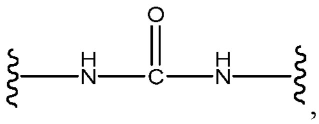,

(A12) (A13) (A14)

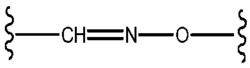, 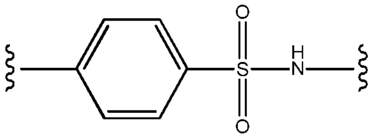, 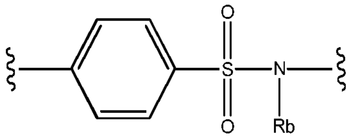,

(A15) (A16) (A17)

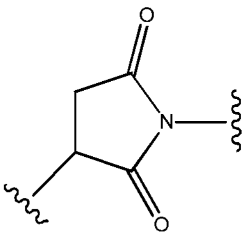, 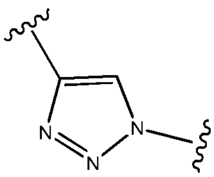, 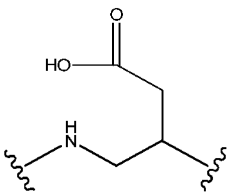, 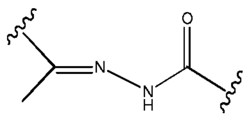,

(A18) (A19) (A20) (A21)

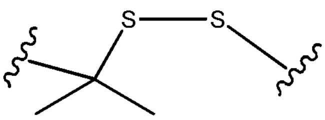, 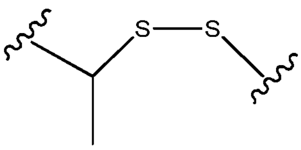, 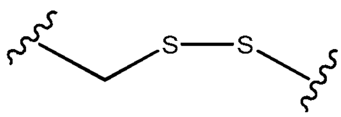,

(A22) (A23) (A24)

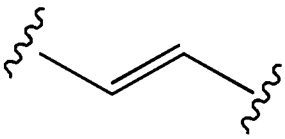 and 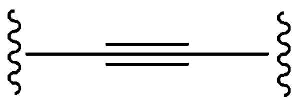;

(A25) (A26)

In some embodiments, the length of $L_1$ may be 3-25 atoms, 3-20 atoms, 4-15 atoms, or 5-12 atoms. In some embodiments, the length of $L_1$ is 3, 4, 5, 6, 7, 8, 9, 10, 11, 12, 13, 14, 15, 16, 17, 18, 19, 20, 21, 22, 23, 24, 25, 30, 35, 40, 45, 50, 55 or 60 atoms.

In some embodiments, j 1 is an integer of 2-10, and in some embodiments, j 1 is an integer of 3-5. In some embodiments, j2 is an integer of 2-10, and in some embodiments, j2 is an integer of 3-5. R' is C1-C4 alkyl, and in some embodiments, R' is one of methyl, ethyl and isopropyl. Ra is one of A27, A28, A29, A30 and A31, and in some embodiments, Ra is A27 or A28. Rb is C1-C5 alkyl, and in some embodiments, Rb is one of methyl, ethyl, isopropyl and butyl. In some embodiments, each of j 1, j2, R', Ra, and Rb in formulas A1-A26 is selected to achieve the linkage of the $M_1$ ligand to the N on the nitrogen-containing backbone, and to make the spatial position between the $M_1$ ligands more suitable for the $M_1$ ligand to bind to the liver surface asialoglycoprotein receptor.

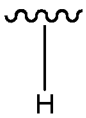 , 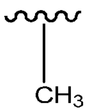 , 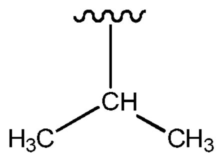 , 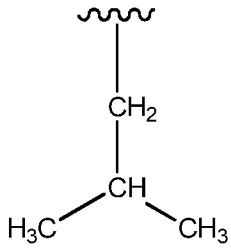 , 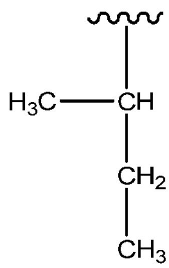

(A27) (A28) (A29) (A30) (A31)

In some embodiments, the siRNA conjugate of the present disclosure has the structure represented by formula (403), (404), (405), (406), (407), (408), (409), (410), (411), (412), (413), (414), (415), (416), (417), (418), (419), (420), (421) or (422).

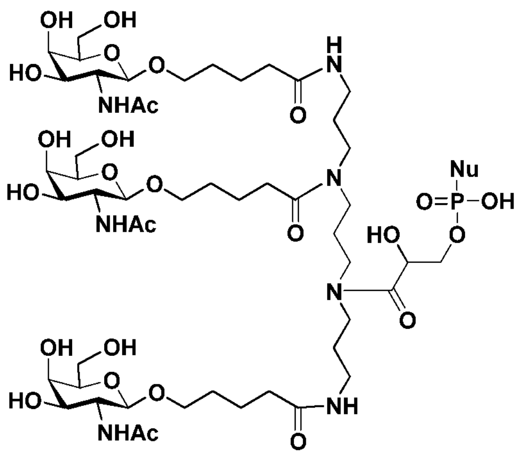

formula (403)

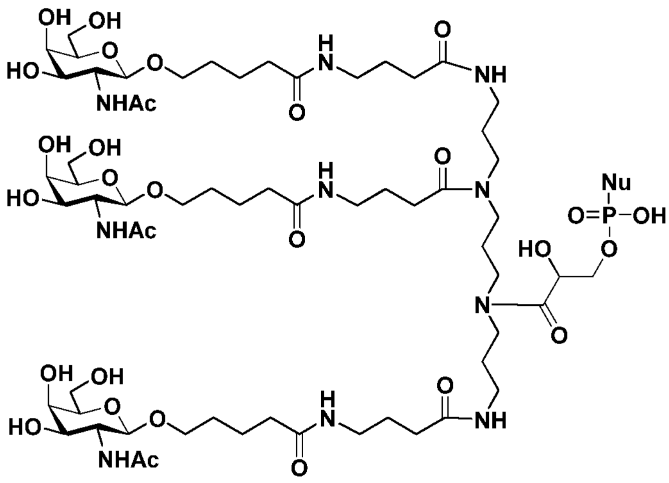

formula (404)

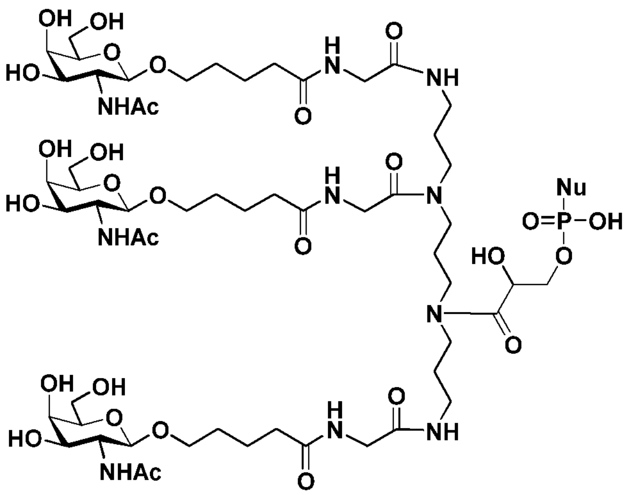

formula (405)

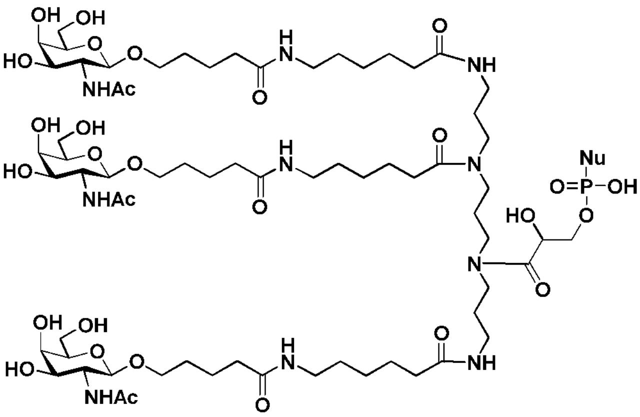

formula (406)

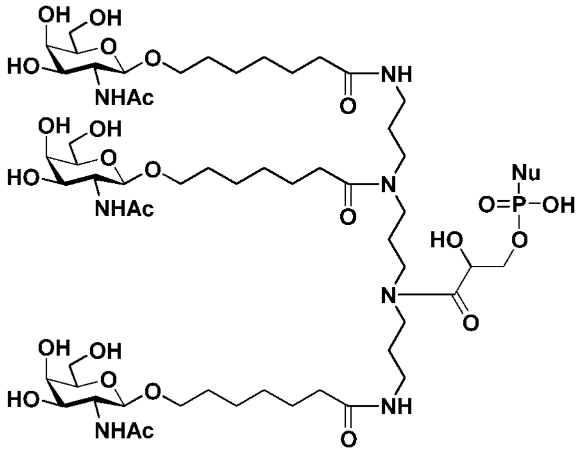

formula (407)

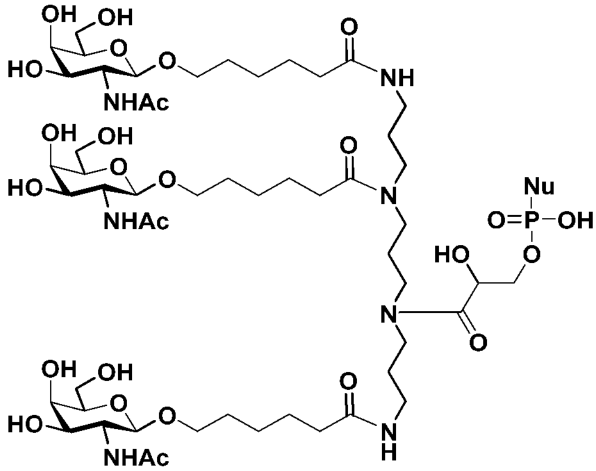

formula (408)

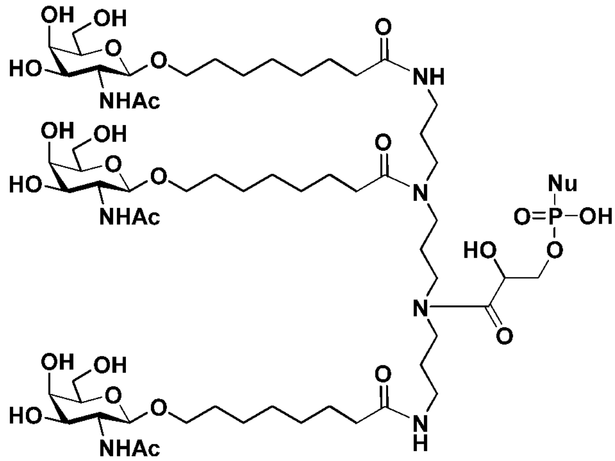

formula (409)

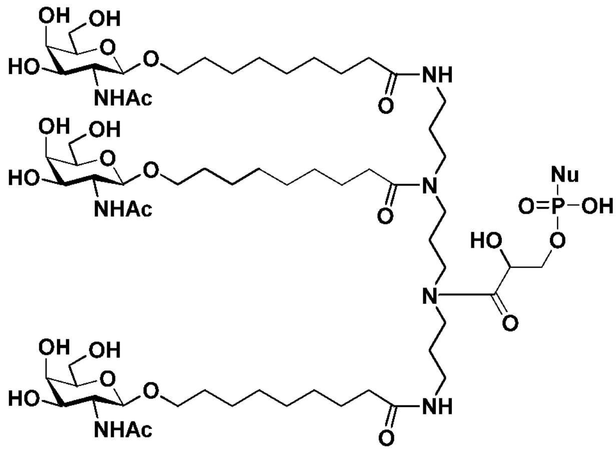

formula (410)

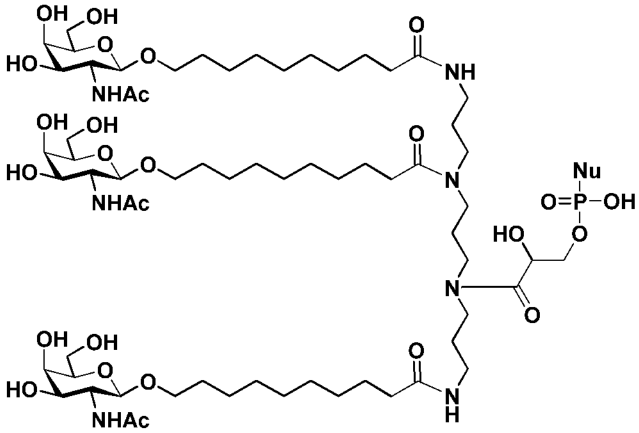

formula (411)

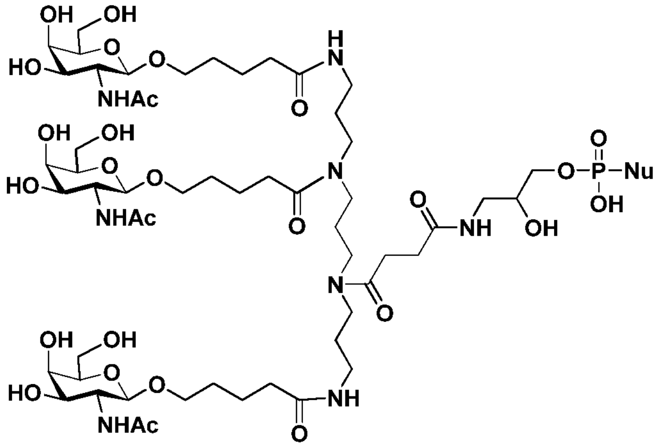

formula (412)

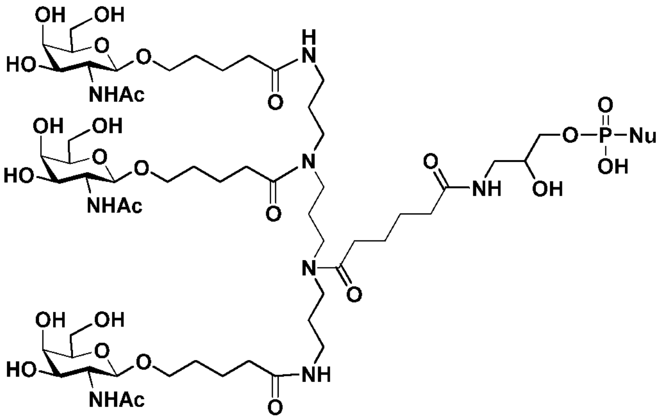

formula (413)

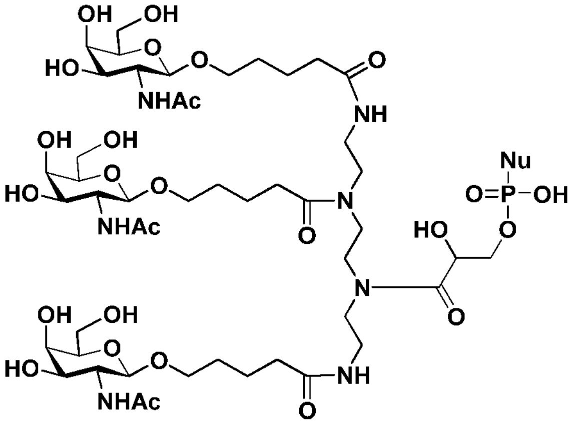

formula (414)

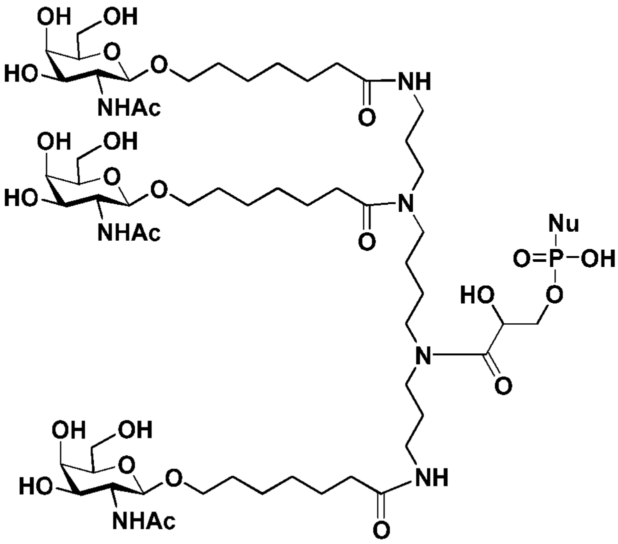

formula (415)

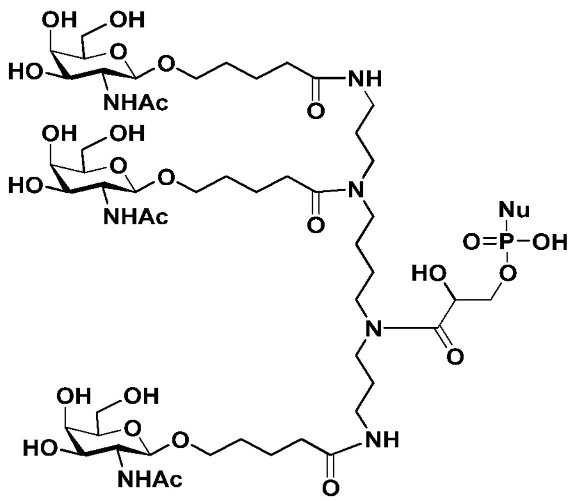

formula (416)

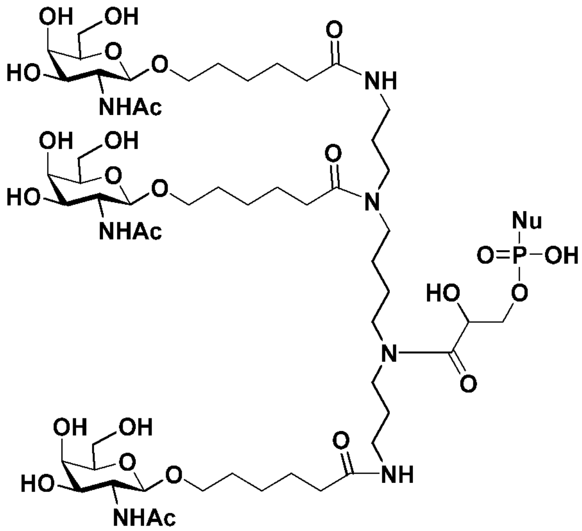

formula (417)

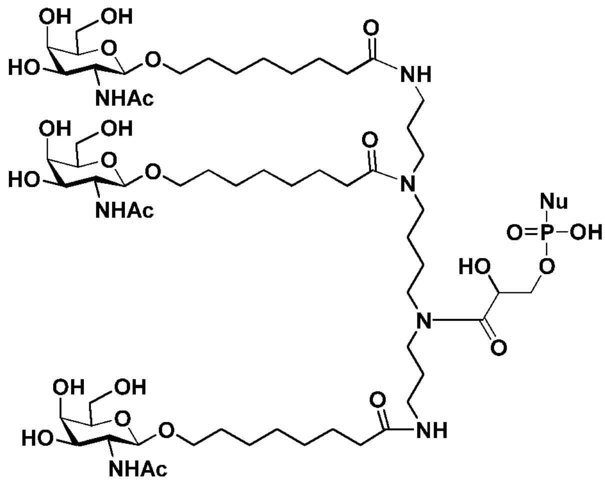

formula (418)

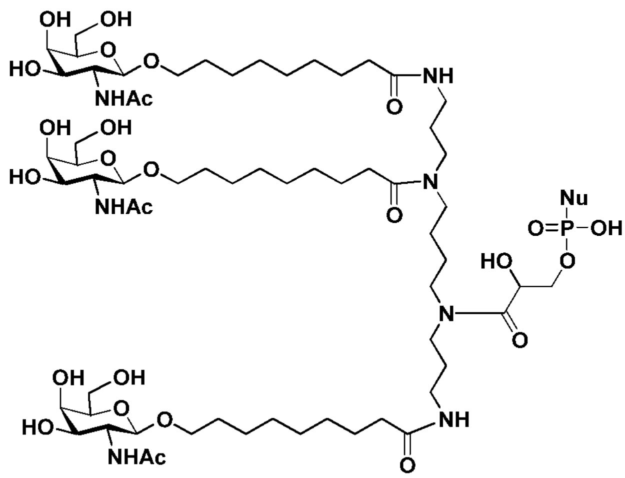

formula (419)

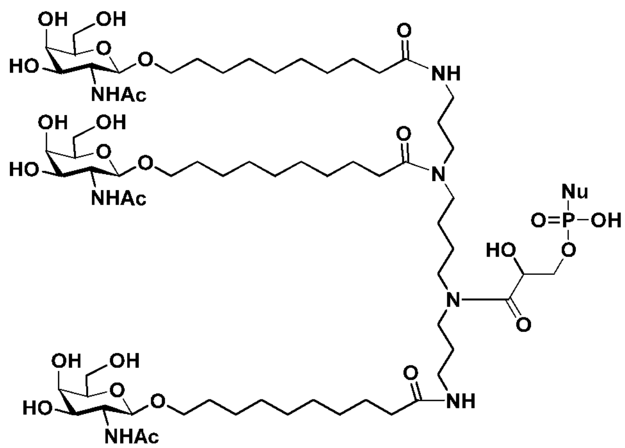

formula (420)

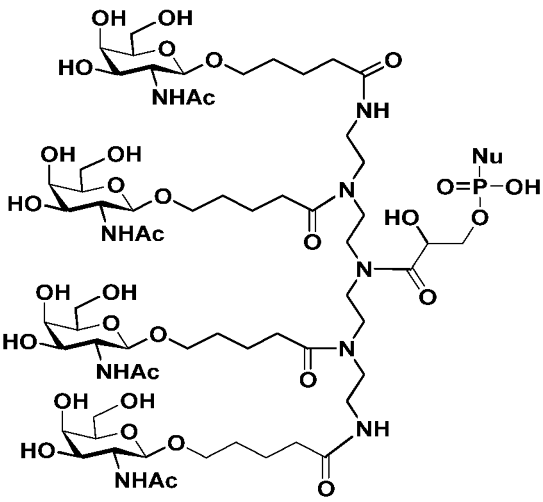

formula (421)

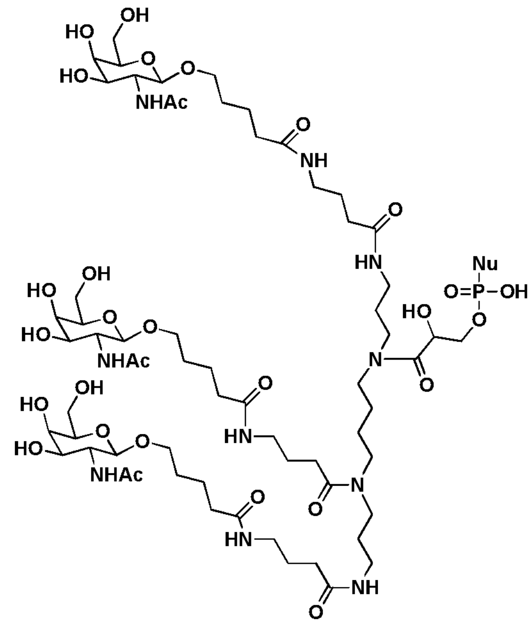

formula (422)

In some embodiments, P in formula A59 may be linked to any possible position in the siRNA sequence. For example, P in formula A59 may be linked to any nucleotide in the sense strand or the antisense strand of the siRNA. In some embodiments, P in formula A59 is linked to any nucleotide in the sense strand of the siRNA. In some embodiments, P in formula A59 is linked to the end of the sense or antisense strand of the siRNA. In some embodiments, P in formula A59 is linked to the end of the sense strand of the siRNA. The end refers to the first 4 nucleotides in the sense strand or the antisense strand from one end thereof. In some embodiments, P in formula A59 is linked to the end of the sense or antisense strand of the siRNA. In some embodiments, P in formula A59 is linked to the 3' end of the sense strand of the siRNA. In the case of being linked to the position described above of the sense strand of the siRNA, after the conjugate provided by the present disclosure enters the cell, upon unwinding, a separate antisense strand of the siRNA may be released to regulate target gene expression through an RNAi mechanism.

P in formula A59 may be linked to any possible position on the nucleotide in the siRNA, for example, the 5' position of the nucleotide, the 2' position of the nucleotide, the 3' position of the nucleotide or the base of the nucleotide. In some embodiments, P in formula A59 may be linked to the 2' position, the 3' position or the 5' position of the nucleotides in the siRNA by forming a phosphodiester bond. In some embodiments, P in formula A59 is linked to the oxygen atom

formed after the dehydrogenation of the 3' hydroxyl of the 3' end nucleotide in the sense strand of the siRNA, or P in formula A59 is linked to the nucleotide by substituting the hydrogen in 2'-hydroxyl of one nucleotide in the sense strand of the siRNA, or P in formula A59 is linked to the nucleotide by substituting the hydrogen in 5'-hydroxyl of the nucleotide at 5' end in the sense strand of the siRNA.

In some embodiments, the siRNA contained in the siRNA conjugate of the present disclosure may be, for example, any siRNA listed in Table 1a, 1b or 1c. The siRNA conjugates containing these siRNAs exhibit low off-target effects and high mRNA inhibition activity on APOC3 gene expression.

Table 1a. First siRNA sequence of the present disclosure

| siRNA No. | SEQ ID NO: | Sequence direction 5'- 3' |
|---|---|---|
| siAPOC3a1-M1 | 13 | CmAmAmUmAmAmAfGfCfUmGmGmAmCmAmAmGmAmAm |
| | 14 | UmUfCSUmUmGfUmCmCmAmGmCmUmUfUmAfUmUmGmGmGm |
| siAPOC3a1-M2 | 15 | CmAmAmUmAmAmAfGfCfUmGmGmAmCmAmAmGmAmAm |
| | 16 | UmUfCmUmUSGfUmCmCmAmGmCmUmUfUmAfUmUmGmGmGm |
| siAPOC3a2-M1 | 17 | CmCmCmAmAmUmAmAmAfGfCfUmGmGmAmCmAmAmGmAmAm |
| | 18 | UmUfCSUmUmGfUmCmCmAmGmCmUmUfUmAfUmUmGmGmGmAmGm |
| siAPOC3a2-M2 | 19 | CmCmCmAmAmUmAmAmAfGfCfUmGmGmAmCmAmAmGmAmAm |
| | 20 | UmUfCmUmUSGfUmCmCmAmGmCmUmUfUmAfUmUmGmGmGmAmGm |
| siAPOC3a1-M1S | 21 | CmsAmsAmUmAmAmAfGfCfUmGmGmAmCmAmAmGmAmAm |
| | 22 | UmsUfsCSUmUmGfUmCmCmAmGmCmUmUfUmAfUmUmGmsGmsGm |
| siAPOC3a1-M2S | 23 | CmsAmsAmUmAmAmAfGfCfUmGmGmAmCmAmAmGmAmAm |
| | 24 | UmsUfsCmUmUSGfUmCmCmAmGmCmUmUfUmAfUmUmGmsGmsGm |
| siAPOC3a2-M1S | 25 | CmsCmsCmAmAmUmAmAmAfGfCfUmGmGmAmCmAmAmGmAmAm |
| | 26 | UmsUfsCSUmUmGfUmCmCmAmGmCmUmUfUmAfUmUmGmGmGmsAmsGm |
| siAPOC3a2-M2S | 27 | CmsCmsCmAmAmUmAmAmAfGfCfUmGmGmAmCmAmAmGmAmAm |
| | 28 | UmsUfsCmUmUSGfUmCmCmAmGmCmUmUfUmAfUmUmGmGmGmsAmsGm |
| siAPOC3a1-M1P1 | 29 | CmAmAmUmAmAmAfGfCfUmGmGmAmCmAmAmGmAmAm |
| | 30 | P1UmUfCSUmUmGfUmCmCmAmGmCmUmUfUmAfUmUmGmGmGm |

(continued)

| siRNA No. | SEQ ID NO: | Sequence direction 5'- 3' |
|---|---|---|
| siAPOC3a1-M2P1 | 31 | CmAmAmUmAmAmAfGfCfUmGmGmAmCmAmAmGmAmAm |
| | 32 | P1UmUfCmUmUSGfUmCmCmAmGmCmUmUfUmAfUmUmGmGmGm |
| siAPOC3a2-M1P1 | 33 | CmCmCmAmAmUmAmAmAfGfCfUmGmGmAmCmAmAmGmAmAm |
| | 34 | P1UmUfCSUmUmGfUmCmCmAmGmCmUmUfUmAfUmUmGmGmGmAmGm |
| siAPOC3a2-M2P1 | 35 | CmCmCmAmAmUmAmAmAfGfCfUmGmGmAmCmAmAmGmAmAm |
| | 36 | P1UmUfCmUmUSGfUmCmCmAmGmCmUmUfUmAfUmUmGmGmGmAmGm |
| siAPOC3a1-M1SP1 | 37 | CmsAmsAmUmAmAmAfGfCfUmGmGmAmCmAmAmGmAmAm |
| | 38 | P1UmsUfsCSUmUmGfUmCmCmAmGmCmUmUfUmAfUmUmGmsGmsGm |
| siAPOC3a1-M2SP1 | 39 | CmsAmsAmAmUmAmAmAfGfCfUmGmGmAmCmAmAmGmAmAm |
| | 40 | P1UmsUfsCmUmUSGfUmCmCmAmGmCmUmUfUmAfUmUmGmsGmsGm |
| siAPOC3a2-M1SP1 | 41 | CmsCmsCmAmAmUmAmAmAfGfCfUmGmGmAmCmAmAmGmAmAm |
| | 42 | P1UmsUfsCSUmUmGfUmCmCmAmGmCmUmUfUmAfUmUmGmGmGmsAmsGm |
| siAPOC3a2-M2SP1 | 43 | CmsCmsCmAmAmUmAmAmAfGfCfUmGmGmAmCmAmAmGmAmAm |
| | 44 | P1UmsUfsCmUmUSGfUmCmCmAmGmCmUmUfUmAfUmUmGmGmGmsAmsGm |

Table 1b. Second siRNA sequence of the present disclosure

| siRNA No. | SEQ ID NO: | Sequence direction 5'- 3' |
|---|---|---|
| siAPOC3b1-M1 | 57 | UmUmAmAmAmAmGfGfGfAmCmAmGmUmAmUmUmCmUm |
| | 58 | AmGfASAmUmAfCmUmGmUmCmCmCmUfUmUfUmAmAmGmCm |
| siAPOC3b1-M2 | 59 | UmUmAmAmAmAmGfGfGfAmCmAmGmUmAmUmUmCmUm |
| | 60 | AmGfAmAmUSAfCmUmGmUmCmCmCmUfUmUfUmAmAmGmCm |

(continued)

| siRNA No. | SEQ ID NO: | Sequence direction 5'- 3' |
|---|---|---|
| siAPOC3b2-M1 | 61 | GmCmUmUmAmAmAmAmGfGfGfAmCmAmGmUmAmUmUmCmUm |
| | 62 | AmGfASAmUmAfCmUmGmUmCmCmCmUfUmUfUmAmAmGmCmAmAm |
| siAPOC3b2-M2 | 63 | GmCmUmUmAmAmAmAmGfGfGfAmCmAmGmUmAmUmUmCmUm |
| | 64 | AmGfAmAmUSAfCmUmGmUmCmCmCmUfUmUfUmAmAmGmCmAmAm |
| siAPOC3b1-M1S | 65 | UmsUmsAmAmAmAmGfGfGfAmCmAmGmUmAmUmUmCmUm |
| | 66 | AmsGfsASAmUmAfCmUmGmUmCmCmCmUfUmUfUmAmAmsGmsCm |
| siAPOC3b1-M2S | 67 | UmsUmsAmAmAmAmGfGfGfAmCmAmGmUmAmUmUmCmUm |
| | 68 | AmsGfsAmAmUSAfCmUmGmUmCmCmCmUfUmUfUmAmAmsGmsCm |
| siAPOC3b2-M1S | 69 | GmsCmsUmUmAmAmAmAmGfGfGfAmCmAmGmUmAmUmUmCmUm |
| | 70 | AmsGfsASAmUmAfCmUmGmUmCmCmCmUfUmUfUmAmAmGmCmsAmsAm |
| siAPOC3b2-M2S | 71 | GmsCmsUmUmAmAmAmAmGfGfGfAmCmAmGmUmAmUmUmCmUm |
| | 72 | AmsGfsAmAmUSAfCmUmGmUmCmCmCmUfUmUfUmAmAmGmCmsAmsAm |
| siAPOC3b1-M1P1 | 73 | UmUmAmAmAmAmGfGfGfAmCmAmGmUmAmUmUmCmUm |
| | 74 | P1AmGfASAmUmAfCmUmGmUmCmCmCmUfUmUfUmAmAmGmCm |
| siAPOC3b1-M2P1 | 75 | UmUmAmAmAmAmGfGfGfAmCmAmGmUmAmUmUmCmUm |
| | 76 | P1AmGfAmAmUSAfCmUmGmUmCmCmCmUfUmUfUmAmAmGmCm |
| siAPOC3b2-M1P1 | 77 | GmCmUmUmAmAmAmAmGfGfGfAmCmAmGmUmAmUmUmCmUm |
| | 78 | P1AmGfASAmUmAfCmUmGmUmCmCmCmUfUmUfUmAmAmGmCmAmAm |
| siAPOC3b2-M2P1 | 79 | GmCmUmUmAmAmAmAmGfGfGfAmCmAmGmUmAmUmUmCmUm |
| | 80 | P1AmGfAmAmUSAfCmUmGmUmCmCmCmUfUmUfUmAmAmGmCmAmAm |

(continued)

| siRNA No. | SEQ ID NO: | Sequence direction 5'- 3' |
|---|---|---|
| siAPOC3b1-M1SP1 | 81 | UmsUmsAmAmAmAmGfGfGfAmCmAmGmUmAmUmUmCmUm |
| | 82 | P1AmsGfsASAmUmAfCmUmGmUmCmCmCmUfUmUfUmAmAmsGmsCm |
| siAPOC3b1-M2SP1 | 83 | UmsUmsAmAmAmAmGfGfGfAmCmAmGmUmAmUmUmCmUm |
| | 84 | P1AmsGfsAmAmUSAfCmUmGmUmCmCmCmUfUmUfUmAmAmsGmsCm |
| siAPOC3b2-M1SP1 | 85 | GmsCmsUmUmAmAmAmAmGfGfGfAmCmAmGmUmAmUmUmCmUm |
| | 86 | |
| siAPOC3b2-M2SP1 | 87 | GmsCmsUmUmAmAmAmAmGfGfGfAmCmAmGmUmAmUmUmCmUm |
| | 88 | P1AmsGfsAmAmUSAfCmUmGmUmCmCmCmUfUmUfUmAmAmGmCmsAmsAm<br>P1AmsGfsAmAmUSAfCmUmGmUmCmCmCmUfUmUfUmAmAmGmCmsAmsAm |
| siAPOC3b3-M1 | 57 | UmUmAmAmAmAmGfGfGfAmCmAmGmUmAmUmUmCmUm |
| | 89 | AmGfASAmUmAfCmUmGmUmCmCmCmUfUmUfUmAmAmUmUm |
| siAPOC3b3-M2 | 59 | UmUmAmAmAmAmGfGfGfAmCmAmGmUmAmUmUmCmUm |
| | 90 | AmGfAmAmUSAfCmUmGmUmCmCmCmUfUmUfUmAmAmUmUm |
| siAPOC3b4-M1 | 61 | GmCmUmUmAmAmAmAmGfGfGfAmCmAmGmUmAmUmUmCmUm |
| | 91 | AmGfASAmUmAfCmUmGmUmCmCmCmUfUmUfUmAmAmGmCmUmUm |
| siAPOC3b4-M2 | 63 | GmCmUmUmAmAmAmAmGfGfGfAmCmAmGmUmAmUmUmCmUm |
| | 92 | AmGfAmAmUSAfCmUmGmUmCmCmCmUfUmUfUmAmAmGmCmUmUm |
| siAPOC3b3-M1S | 65 | UmsUmsAmAmAmAmGfGfGfAmCmAmGmUmAmUmUmCmUm |
| | 93 | AmsGfsASAmUmAfCmUmGmUmCmCmCmUfUmUfUmAmAmsUmsUm |
| siAPOC3b3-M2S | 67 | UmsUmsAmAmAmAmGfGfGfAmCmAmGmUmAmUmUmCmUm |
| | 94 | AmsGfsAmAmUSAfCmUmGmUmCmCmCmUfUmUfUmAmAmsUmsUm |

(continued)

| siRNA No. | SEQ ID NO: | Sequence direction 5'- 3' |
|---|---|---|
| siAPOC3b4-M1S | 69 | GmsCmsUmUmAmAmAmAmGfGfGfAmCmAmGmUmAmUmUmCmUm |
| | 95 | AmsGfsASAmUmAfCmUmGmUmCmCmCmUfUmUfUmAmAmGmCmsUmsUm |
| siAPOC3b4-M2S | 71 | GmsCmsUmUmAmAmAmAmGfGfGfAmCmAmGmUmAmUmUmCmUm |
| | 96 | AmsGfsAmAmUSAfCmUmGmUmCmCmCmUfUmUfUmAmAmGmCmsUmsUm |
| siAPOC3b3-M1P1 | 73 | UmUmAmAmAmAmGfGfGfAmCmAmGmUmAmUmUmCmUm |
| | 97 | P1AmGfASAmUmAfCmUmGmUmCmCmCmUfUmUfUmAmAmUmUm |
| siAPOC3b3-M2P1 | 75 | UmUmAmAmAmAmGfGfGfAmCmAmGmUmAmUmUmCmUm |
| | 98 | P1AmGfAmAmUSAfCmUmGmUmCmCmCmUfUmUfUmAmAmUmUm |
| siAPOC3b4-M1P1 | 77 | GmCmUmUmAmAmAmAmGfGfGfAmCmAmGmUmAmUmUmCmUm |
| | 99 | P1AmGfASAmUmAfCmUmGmUmCmCmCmUfUmUfUmAmAmGmCmUmUm |
| siAPOC3b4-M2P1 | 79 | GmCmUmUmAmAmAmAmGfGfGfAmCmAmGmUmAmUmUmCmUm |
| | 100 | P1AmGfAmAmUSAfCmUmGmUmCmCmCmUfUmUfUmAmAmGmCmUmUm |
| siAPOC3b3-M1SP1 | 81 | UmsUmsAmAmAmAmGfGfGfAmCmAmGmUmAmUmUmCmUm |
| | 101 | P1AmsGfsASAmUmAfCmUmGmUmCmCmCmUfUmUfUmAmAmsUmsUm |
| siAPOC3b3-M2SP1 | 83 | UmsUmsAmAmAmAmGfGfGfAmCmAmGmUmAmUmUmCmUm |
| | 102 | P1AmsGfsAmAmUSAfCmUmGmUmCmCmCmUfUmUfUmAmAmsUmsUm |
| siAPOC3b4-M1SP1 | 85 | GmsCmsUmUmAmAmAmAmGfGfGfAmCmAmGmUmAmUmUmCmUm |
| | 103 | P1AmsGfsASAmUmAfCmUmGmUmCmCmCmUfUmUfUmAmAmGmCmsUmsUm |
| siAPOC3b4-M2SP1 | 87 | GmsCmsUmUmAmAmAmAmGfGfGfAmCmAmGmUmAmUmUmCmUm |
| | 104 | P1AmsGfsAmAmUSAfCmUmGmUmCmCmCmUfUmUfUmAmAmGmCmsUmsUm |

Table 1c. Third siRNA sequence of the present disclosure

| siRNA No. | SEQ ID NO: | Sequence direction 5'- 3' |
|---|---|---|
| siAPOC3c1-M1 | 117 | GmGmAmCmAmGmUfAfUfUmCmUmCmAmGmUmGmCmUm |
| | 118 | AmGfCSAmCmUfGmAmGmAmAmUmAmCfUmGfUmCmCmCmUm |
| siAPOC3c1-M2 | 119 | GmGmAmCmAmGmUfAfUfUmCmUmCmAmGmUmGmCmUm |
| | 120 | AmGfCmAmCSUfGmAmGmAmAmUmAmCfUmGfUmCmCmCmUm |
| siAPOC3c2-M1 | 121 | AmGmGmGmAmCmAmGmUfAfUfUmCmUmCmAmGmUmGmCmUm |
| | 122 | AmGfCSAmCmUfGmAmGmAmAmUmAmCfUmGfUmCmCmCmUmUmUm |
| siAPOC3c2-M2 | 123 | AmGmGmGmAmCmAmGmUfAfUfUmCmUmCmAmGmUmGmCmUm |
| | 124 | AmGfCmAmCSUfGmAmGmAmAmUmAmCfUmGfUmCmCmCmUmUmUm |
| siAPOC3c1-M1S | 125 | GmsGmsAmCmAmGmUfAfUfUmCmUmCmAmGmUmGmCmUm |
| | 126 | AmsGfsCSAmCmUfGmAmGmAmAmUmAmCfUmGfUmCmCmsCmsUm |
| siAPOC3c1-M2S | 127 | GmsGmsAmCmAmGmUfAfUfUmCmUmCmAmGmUmGmCmUm |
| | 128 | AmsGfsCmAmCSUfGmAmGmAmAmUmAmCfUmGfUmCmCmsCmsUm |
| siAPOC3c2-M1S | 129 | AmsGmsGmGmAmCmAmGmUfAfUfUmCmUmCmAmGmUmGmCmUm |
| | 130 | AmsGfsCSAmCmUfGmAmGmAmAmUmAmCfUmGfUmCmCmCmUmsUmsUm |
| siAPOC3c2-M2S | 131 | AmsGmsGmGmAmCmAmGmUfAfUfUmCmUmCmAmGmUmGmCmUm |
| | 132 | AmsGfsCmAmCSUfGmAmGmAmAmUmAmCfUmGfUmCmCmCmUmsUmsUm |
| siAPOC3c1-M1P1 | 133 | GmGmAmCmAmGmUfAfUfUmCmUmCmAmGmUmGmCmUm |
| | 134 | P1AmGfCSAmCmUfGmAmGmAmAmUmAmCfUmGfUmCmCmCmUm |
| siAPOC3c1-M2P1 | 135 | GmGmAmCmAmGmUfAfUfUmCmUmCmAmGmUmGmCmUm |
| | 136 | P1AmGfCmAmCSUfGmAmGmAmAmUmAmCfUmGfUmCmCmCmUm |

(continued)

| siRNA No. | SEQ ID NO: | Sequence direction 5'- 3' |
|---|---|---|
| siAPOC3c2-M1P1 | 137 | AmGmGmGmAmCmAmGmUfAfUfUmCmUmCmAmGmUmGmCmUm |
| | 138 | P1AmGfCSAmCmUfGmAmGmAmAmUmAmCfUmGfUmCmCmCmUmUmUm |
| siAPOC3c2-M2P1 | 139 | AmGmGmGmAmCmAmGmUfAfUfUmCmUmCmAmGmUmGmCmUm |
| | 140 | P1AmGfCmAmCSUfGmAmGmAmAmUmAmCfUmGfUmCmCmCmUmUmUm |
| siAPOC3c1-M1SP1 | 141 | GmsGmsAmCmAmGmUfAfUfUmCmUmCmAmGmUmGmCmUm |
| | 142 | P1AmsGfsCSAmCmUfGmAmGmAmAmUmAmCfUmGfUmCmCmsCmsUm |
| siAPOC3c1-M2SP1 | 143 | GmsGmsAmCmAmGmUfAfUfUmCmUmCmAmGmUmGmCmUm |
| | 144 | P1AmsGfsCmAmCSUfGmAmGmAmAmUmAmCfUmGfUmCmCmsCmsUm |
| siAPOC3c2-M1SP1 | 145 | AmsGmsGmGmAmCmAmGmUfAfUfUmCmUmCmAmGmUmGmCmUm |
| | 146 | P1AmsGfsCSAmCmUfGmAmGmAmAmUmAmCfUmGfUmCmCmCmUmsUmsUm |
| siAPOC3c2-M2SP1 | 147 | AmsGmsGmGmAmCmAmGmUfAfUfUmCmUmCmAmGmUmGmCmUm |
| | 148 | P1AmsGfsCmAmCSUfGmAmGmAmAmUmAmCfUmGfUmCmCmCmUmsUmsUm |

The uppercase letters C, G, U, and A represent the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the uppercase letter S underlined indicates that the nucleotide adjacent to the letter S on the left side is a stabilizing modified nucleotide; the lowercase letter s indicates that the two nucleotides on the left and right sides of the letter s are linked by a phosphorothioate group; P1 indicates that the nucleotide adjacent to the P1 on the right side is a 5'-phosphate nucleotide or 5'-phosphate analog-modified nucleotide. In some embodiments, S represents a specific stabilizing modification such as moe, wherein the underlined letter combination moe indicates that a nucleotide adjacent to the left side of the letter combination moe has a 2'-O-methoxyethyl-modified nucleotide. In some embodiments, P1 is VP, Ps or P representing a specific modification, wherein the letter combination VP indicates that a nucleotide adjacent to the right side of the letter combination VP is vinylphosphate (5'-(E)- inylphosphonate, E-VP) modified nucleotide, the letter combination Ps indicates that a nucleotide adj acent to the right side of the letter combination Ps is a phosphorothioate modified nucleotide, and the uppercase letter P indicates that a nucleotide adj acent to the right side of the letter P is 5'-phosphate nucleotide. In addition, each U in the sequences listed in Table 1a to Table 1c may be arbitrarily replaced by T without significant effect on the activity or off-target effects of the siRNA.

Preparation of siRNA conjugate of the present disclosure

The siRNA conjugates may be synthesized by methods that have been described in detail in the prior art. For

example, WO2015006740A2 describes preparation methods for various siRNA conjugates in detail. The siRNA conjugates of the present disclosure may also be obtained by means well known to those skilled in the art. For example, the preparation method for the structure represented by formula (305) is described in WO2014025805A1, and the preparation method for the structure represented by formula (307) is described by Rajeev et al. in ChemBioChem 2015, 16, 903-908. Chinese patent application CN110959011A also discloses in detail a method for preparing the siRNA conjugate represented by formula (308). The contents of the documents are incorporated herein in their entirety by reference.

The siRNA conjugate of the present disclosure may also be used in combination with other pharmaceutically acceptable adjuvants. The adjuvants may be one or more of various preparations or compounds commonly used in the art. For details, please refer to the description about the pharmaceutical composition of the present disclosure.

## Use of siRNA, pharmaceutical composition and siRNA conjugate of the present disclosure

In some embodiments, the present disclosure provides use of the siRNA and/or the pharmaceutical composition and/or the siRNA conjugate of the present disclosure in the preparation of a medicament for treating and/or preventing diseases or symptoms associated with the level of an mRNA expressed by an APOC3 gene. In some embodiments, the disease or symptom associated with the level of the mRNA expressed by the APOC3 gene is dyslipidemia. In some embodiments, the dyslipidemia is hypercholesterolemia, hypertriglyceridemia, or atherosclerosis.

In some embodiments, the present disclosure provides a method for treating and/or preventing diseases or symptoms associated with the level of the mRNA expressed by the APOC3 gene, the method comprising: administering to a subject in need thereof the siRNA and/or the pharmaceutical composition and/or the siRNA conjugate of the present disclosure. In some embodiments, the disease or symptom associated with the level of the mRNA expressed by the APOC3 gene is dyslipidemia. In some embodiments, the dyslipidemia is hypercholesterolemia, hypertriglyceridemia, or atherosclerosis.

In some embodiments, the present disclosure also provides a method for inhibiting the expression level of the APOC3 gene in a cell, the method comprising: contacting an effective dose of the siRNA and/or the pharmaceutical composition and/or the siRNA conjugate of the present disclosure with the cell.

By administering to a subject in need thereof the siRNA, the pharmaceutical composition and/or the siRNA conjugate provided by the present disclosure, the purpose of preventing and/or treating pathological conditions or diseases caused by the expression of specific genes in cells may be achieved by a mechanism that regulates the gene expression. Therefore, the siRNA, the pharmaceutical composition and/or the siRNA conjugate provided by the present disclosure may be used to prevent and/or treat the pathological condition or disease, or to prepare a medicament for the prevention and/or treatment of the pathological condition or disease as described herein.

As used herein, the term "administration/administer" refers to a method or approach of positioning at least a part of the siRNA, the pharmaceutical composition and/or the siRNA conjugate at a desired site to produce a desired effect so as to place the siRNA, the pharmaceutical composition and/or the siRNA conjugate into a subject. Routes of administration suitable for the methods of the present disclosure include topical and systemic administration. In general, the topical administration results in delivery of more siRNAs, pharmaceutical compositions and/or siRNA conjugates to a specific site compared to the entire body of a subject, whereas the systemic administration results in delivery of the siRNAs, pharmaceutical compositions and/or siRNA conjugates to substantially the entire body of the subject. Considering that the present disclosure is intended to provide means for the prevention and/or treatment of pathological conditions or diseases caused by the expression of specific genes in liver cells, the means is a mode of administration capable of delivering a medicament to the liver in some embodiments.

The administration to a subject may be by any suitable route known in the art, including, but not limited to: oral or parenteral routes, such as intravenous, intramuscular, subcutaneous, transdermal, airway (aerosol), pulmonary, nasal, rectal, and topical (including buccal and sublingual) administration. The frequency of administration may be one or more times every day, every week, every two weeks, every three weeks, every month or every year.

The doses of the siRNA, the pharmaceutical composition and/or the siRNA conjugate of the present disclosure may be conventional doses in the art, and the dose may be based on various parameters, especially the age, weight and sex of the subject. Toxicity and efficacy may be determined by standard pharmaceutical procedures in cell culture or experimental animals, such as determining the LD50 (the dose that causes 50% of the population to die) and the ED50 (the dose that may cause 50% of the maximum response intensity in terms of quantitative response, and the dose that may cause 50% of the test subjects to have a positive reaction in terms of qualitative response). The range of doses for use in humans may be derived based on the data obtained from cell culture assays and animal studies.

When administering the siRNA, the pharmaceutical composition and/or the siRNA conjugate of the present disclosure, for example, to C57BL/6J, or C3H/HeNCrlVr mice (male or female, 6-12 weeks old, weighing 18-25 g), the administration is based on the amount of the siRNA in the siRNA, the pharmaceutical composition and/or the siRNA conjugate: for the siRNA conjugate formed from the siRNA and the pharmaceutically acceptable conjugated molecule, the amount of the siRNA may be 0.001-100 mg/kg body weight. In some embodiments, the amount is 0.01-50 mg/kg

body weight. In a further embodiment, the amount is 0.05-20 mg/kg body weight. In a yet further embodiment, the amount is 0.1-15 mg/kg body weight. In a still further embodiment, the amount is 0.1-10 mg/kg body weight. The doses may be preferred when administering the siRNA, the pharmaceutical composition and/or the siRNA conjugate of the present disclosure.

In addition, by introducing the siRNA, the pharmaceutical composition and/or the siRNA conjugate of the present disclosure into cells with abnormal expression of specific genes, it is also possible to achieve the purpose of inhibiting the expression of the specific gene in the cells through the mechanism of gene expression regulation. In some embodiments, the cells are hepatocytes. In some embodiments, the hepatocytes may be cells selected from hepatoma cell lines such as Hep3B, HepG2 and Huh7, or isolated primary hepatocytes, and in some embodiments, the cells are primary hepatocytes.

For using the method provided by the present disclosure to inhibit the expression of specific genes in cells, the amount of the siRNA in the provided siRNA, pharmaceutical composition and/or siRNA conjugate are easily determined by those skilled in the art according to the desired effect. For example, in some embodiments, the amount of the siRNA in the provided siRNA conjugate is such that: it is sufficient to reduce the expression of the target gene and result in an extracellular concentration of 1 pM to 1 μM, or 0.01 nM to 100 nM, or 0.05 nM to 50 nM or to about 5 nM at the target cell surface. The amount necessary to achieve this local concentration will vary depending on various factors including the method of delivery, the site of delivery, the number of cell layers between the site of delivery and the target cell or tissue, delivery range (local or systemic delivery), and the like. The concentration at the site of delivery may be significantly higher than the concentration at the surface of the target cell or tissue.

Kit

The present disclosure provides a kit, comprising the siRNA, the pharmaceutical composition and/or the siRNA conjugate provided by the present disclosure.

In some embodiments, the kit may provide the siRNA, the pharmaceutical composition and/or the conjugate in one container. In some embodiments, the kit may contain a container providing a pharmaceutically acceptable excipient. In some embodiments, the kit may further comprise other components, such as stabilizers or preservatives. In some embodiments, the kit may comprise at least one additional therapeutic agent in a container other than the container in which the siRNA, the pharmaceutical composition and/or the conjugate are provided. In some embodiments, the kit may comprise an instruction for mixing the siRNA, the pharmaceutical composition and/or the conjugate with a pharmaceutically acceptable carrier and/or an excipient or other components (if any).

In the kit of the present disclosure, the siRNA and the pharmaceutically acceptable carrier and/or the adjuvant and the pharmaceutical composition and/or the conjugate, and/or the pharmaceutically acceptable adjuvant may be provided in any form, for example, liquid form, dry form or lyophilized form. In some embodiments, the siRNA and the pharmaceutically acceptable carrier and/or the adjuvant and the pharmaceutical composition and/or the conjugate and optionally the pharmaceutically acceptable adjuvant are substantially pure and/or sterile. In some embodiments, sterile water may be provided in the kit of the present disclosure.

The following examples will further illustrate the present disclosure, but the present disclosure is not limited thereby.

## Examples

Unless otherwise specified, the reagents and media used in the following examples are all commercially available, and the operations such as nucleic acid electrophoresis and real-time PCR used are all performed by referring to the methods recorded in the Molecular Cloning (Cold Spring Harbor LBboratory Press (1989)).

Preparation Examples 1-7. Synthesis of siRNA conjugates provided by the present disclosure

According to the preparation method described in Preparation Example 1 of CN110959011A, the following conjugates 1-7 in Table 2 were prepared, the only difference being that the sense strand and antisense strand of the siRNA contained in each siRNA conjugate were as shown in Table 2. For the nucleic acid sequences, according to the nucleic acid sequences of the siRNAs designated conjugate 1-conjugate 7 in Table 2, the sense strands and the antisense strands of the siRNAs were synthesized. Ultrapure water (Milli-Q ultrapure water instrument, resistivity 18.2 MS2*cm (25 °C)) were used to dilute each siRNA conjugate to a concentration of 0.2 mg/mL (based on siRNA), and then a liquid chromatography-mass spectrometry instrument (LC-MS, purchased from Waters Inc., model: LCT Premier) was used for molecular weight determination. The found value was consistent with the calculated value, indicating that the synthesized conjugates 1-7 have the target designed double-stranded nucleic acid sequences. The siRNA conjugates have a structure represented by formula (403), and the siRNAs contained in the siRNA conjugates have the siRNA sequences

corresponding to conjugates 1-7 in Table 2. For example, for the molecular weight (mw) of conjugate 5, the sense strand theoretical value: 7581.42; measured value: 7581.22; the antisense strand theoretical value: 6948.55; measured value: 6948.72. The measured values are consistent with the theoretical values, indicating that the conjugate has the structure represented by formula (403) and that the conjugate has the siRNA sequence corresponding to conjugate 5 in Table 2.

Table 2. siRNA sequences in siRNA conjugates

| Preparation Example No. | siRNA No. | Sequence direction 5'-3' | | SEQ ID NO |
|---|---|---|---|---|
| Preparation Example 1 | Conjugate 1 | Sense strand | CmsAmsAmUmAmAmAfGfCfUmGmGmA mCmAmAmGmAmAm | 37 |
| | | Antisense strand | PUmsUfsCmoeUmUmGfUmCmCmAmGmC mUmUfUmAfUmUmGmsGmsGm | 151 |
| Preparation Example 2 | Conjugate 2 | Sense strand | CmsAmsAmUmAmAmAfGfCfUmGmGmA mCmAmAmGmAmAm | 39 |
| | | Antisense strand | PUmsUfsCmUmTmoeGfUmCmCmAmGmC mUmUfUmAfUmUmGmsGmsGm | 152 |
| Preparation Example 3 | Conjugate 3 | Sense strand | UmsUmsAmAmAmAmGfGfGfAmCmAmG mUmAmUmUmCmUm | 81 |
| | | Antisense strand | PAmsGfsAmoeAmUmAfCmUmGmUmCmC mCmUfUmUfUmAmAmsGmsCm | 153 |
| Preparation Example 4 | Conjugate 4 | Sense strand | UmsUmsAmAmAmAmGfGfGfAmCmAmG mUmAmUmUmCmUm | 83 |
| | | Antisense strand | PAmsGfsAmAmTmoeAfCmUmGmUmCmC mCmUfUmUfUmAmAmsGmsCm | 154 |
| Preparation Example 5 | Conjugate 5 | Sense strand | UmsUmsAmAmAmAmGfGfGfAmCmAmG mUmAmUmUmCmUm | 65 |
| | | | AmsGfsAmoeAmUmAfCmUmGmUmCmC mCmUfUmUfUmAmAmsUmsUm | 155 |
| Preparation Example 6 | Conjugate 6 | Sense strand | GmsGmsAmCmAmGmUfAfUfUmCmUmC mAmGmUmGmCmUm | 141 |
| | | Antisense strand | PAmsGfsCmoeAmCmUfGmAmGmAmAmU mAmCfUmGfUmCmCmsCmsUm | 156 |
| Preparation Example 7 | Conjugate 7 | Sense strand | GmsGmsAmCmAmGmUfAfUfUmCmUmC mAmGmUmGmCmUm | 143 |
| | | Antisense strand | PAmsGfsCmAmCmoeUfGmAmGmAmAmU mAmCfUmGfUmCmCmsCmsUm | 157 |

(continued)

| Preparation Example No. | siRNA No. | Sequence direction 5'-3' | | SEQ ID NO |
|---|---|---|---|---|
| Comparative Preparation Example 1 | Reference conjugate 1 | Sense strand | CmsAmsAmUmAmAmAfGfCfUmGmGmA mCmAmAmGmAmAm | 37 |
| | | Antisense strand | PUmsUfsCmUmUmGfUmCmCmAmGmCm UmUfUmAfUmUmGmsGmsGm | 158 |
| Comparative Preparation Example 2 | Reference conjugate 2 | Sense strand | UmsUmsAmAmAmAmGfGfGfAmCmAmG mUmAmUmUmCmUm | 65 |
| | | Antisense strand | PAmsGfsAmAmUmAfCmUmGmUmCmCm CmUfUmUfUmAmAmsGmsCm | 159 |
| Comparative Preparation Example 3 | Reference conjugate 3 | Sense strand | GmsGmsAmCmAmGmUfAfUfUmCmUmC mAmGmUmGmCmUm | 125 |
| | | Antisense strand | PAmsGfsCmAmCmUfGmAmGmAmAmUm AmCfUmGfUmCmCmsCmsUm | 160 |
| Preparation Example 8 | siRNA1 | Sense strand | CmsAmsAmUmAmAmAfGfCfUmGmGmA mCmAmAmGmAmAm | 21 |
| | | Antisense strand | UmsUfsCmoeUmUmGfUmCmCmAmGmC mUmUfUmAfUmUmGmsGmsGm | 22 |
| Preparation Example 9 | siRNA2 | Sense strand | CmsAmsAmUmAmAmAfGfCfUmGmGmA mCmAmAmGmAmAm | 23 |
| | | Antisense strand | UmsUfsCmUmUmoeGfUmCmCmAmGmC mUmUfUmAfUmUmGmsGmsGm | 24 |
| Preparation Example 10 | siRNA3 | Sense strand | UmsUmsAmAmAmAmGfGfGfAmCmAmG mUmAmUmUmCmUm | 65 |
| | | Antisense strand | AmsGfsAmoeAmUmAfCmUmGmUmCmC mCmUfUmUfUmAmAmsGmsCm | 66 |
| Preparation Example 11 | siRNA4 | Sense strand | UmsUmsAmAmAmAmGfGfGfAmCmAmG mUmAmUmUmCmUm | 67 |
| | | Antisense strand | AmsGfsAmAmUmoeAfCmUmGmUmCmC mCmUfUmUfUmAmAmsGmsCm | 68 |
| Preparation Example 12 | siRNA5 | Sense strand | UmsUmsAmAmAmAmGfGfGfAmCmAmG mUmAmUmUmCmUm | 65 |
| | | Antisense strand | AmsGfsAmoeAmUmAfCmUmGmUmCmC mCmUfUmUfUmAmAmsUmsUm | 93 |

(continued)

| Preparation Example No. | siRNA No. | Sequence direction 5'-3' | | SEQ ID NO |
|---|---|---|---|---|
| Preparation Example 13 | siRNA6 | Sense strand | GmsGmsAmCmAmGmUfAfUfUmCmUmCmAmGmUmGmCmUm | 125 |
| | | Antisense strand | AmsGfsCmoeAmCmUfGmAmGmAmAmUmAmCfUmGfUmCmCmsCmsUm | 126 |
| Preparation Example 14 | siRNA7 | Sense strand | GmsGmsAmCmAmGmUfAfUfUmCmUmCmAmGmUmGmCmUm | 127 |
| | | Antisense strand | AmsGfsCmAmCmoeUfGmAmGmAmAmUmAmCfUmGfUmCmCmsCmsUm | 128 |
| Comparative Preparation Example 4 | Reference siRNA1 | Sense strand | CmsAmsAmUmAmAmAfGfCfUmGmGmAmCmAmAmGmAmAm | 21 |
| | | Antisense strand | UmsUfsCUmUmGfUmCmCmAmGmCmUmUfUmAfUmUmGmsGmsGm | 171 |
| Comparative Preparation Example 5 | Reference siRNA2 | Sense strand | CmsAmsAmUmAmAmAfGfCfUmGmGmAmCmAmAmGmAmAm | 23 |
| | | Antisense strand | UmsUfsCmUmUGfUmCmCmAmGmCmUmUfUmAfUmUmGmsGmsGm | 172 |
| Comparative Preparation Example 6 | Reference siRNA3 | Sense strand | UmsUmsAmAmAmAmGfGfGfAmCmAmGmUmAmUmUmCmUm | 65 |
| | | Antisense strand | AmsGfsAAmUmAfCmUmGmUmCmCmCmUfUmUfUmAmAmsGmsCm | 173 |
| Comparative Preparation Example 7 | Reference siRNA4 | Sense strand | UmsUmsAmAmAmAmGfGfGfAmCmAmGmUmAmUmUmCmUm | 67 |
| | | Antisense strand | AmsGfsAmAmUAfCmUmGmUmCmCmCmUfUmUfUmAmAmsGmsCm | 174 |
| Comparative Preparation Example 8 | Reference siRNA5 | Sense strand | UmsUmsAmAmAmAmGfGfGfAmCmAmGmUmAmUmUmCmUm | 65 |
| | | Antisense strand | AmsGfsAAmUmAfCmUmGmUmCmCmCmUfUmUfUmAmAmsUmsUm | 175 |
| Comparative Preparation Example 9 | Reference siRNA6 | Sense strand | GmsGmsAmCmAmGmUfAfUfUmCmUmCmAmGmUmGmCmUm | 125 |
| | | Antisense strand | AmsGfsCAmCmUfGmAmGmAmAmUmAmCfUmGfUmCmCmsCmsUm | 176 |

(continued)

| Preparation Example No. | siRNA No. | Sequence direction 5'-3' | | SEQ ID NO |
|---|---|---|---|---|
| Comparative Preparation Example 10 | Reference siRNA7 | Sense strand | GmsGmsAmCmAmGmUfAfUfUmCmUmCmAmGmUmGmCmUm | 127 |
| | | Antisense strand | AmsGfsCmAmCUfGmAmGmAmAmUmAmCfUmGfUmCmCmsCmsUm | 177 |

The uppercase letters C, G, U, A, and T represents the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the letter combination moe underlined indicates that the nucleotide adjacent to the letter combination moe on the left side is a ribose 2'-O-methoxyethyl-modified nucleotide; the lowercase letter s indicates that the two nucleotides on the left and right sides of the letter s are linked by a phosphorothioate group; P indicates that the nucleotide adjacent to the letter P on the right side is a 5'-phosphate nucleotide.

Comparative Preparation Examples 1-3. Synthesis of reference siRNA conjugates According to the preparation method described in Preparation Example 1 of CN110959011A, the following reference conjugates designated reference conjugates 1-3 in Table 2 were prepared, the only difference being that the sense strand and antisense strand of the siRNA contained in each reference siRNA conjugate were as shown in Table 2. According to the nucleic acid sequences of the siRNAs designated reference conjugates 1-3 in Table 2, the sense strands and the antisense strands of the siRNAs were synthesized. Ultrapure water (Milli-Q ultrapure water instrument, resistivity 18.2 MΩ*cm (25 °C)) was used to dilute each reference siRNA conjugate to a concentration of 0.2 mg/mL (based on siRNA), and then a liquid chromatography-mass spectrometry instrument (LC-MS, purchased from Waters Inc., model: LCT Premier) was used for molecular weight determination. The found value was consistent with the calculated value, indicating that the synthesized reference conjugates 1-3 have the target designed double-stranded nucleic acid sequences. The reference siRNA conjugates have a structure represented by formula (403), and the siRNAs contained have the siRNA sequences corresponding to reference conjugates 1-3 in Table 2. These siRNA sequences do not contain stabilizing modified nucleotides.

Comparative Preparation Example 4. Synthesis of reference siRNA NC

According to the method described in Preparation Example 1 in WO2019105418(A1), the following reference siRNA NC was synthesized by a solid-phase synthesis method, the only difference being that DEPC water was used to dissolve the equimolar complementary sense strand set forth in SEQ ID NO: 161 and antisense strand set forth in SEQ ID NO: 162, followed by annealing to obtain a reference siRNANC:

5'-UmsUmsCmUmCmCmGfAfAfCmGmUmGmUmCmAmCmGmUm-3'
(SEQ ID NO: 161);

5'-AmsCfsGmUmGmAfCmAmCmGmUmUmCmGfGmAfGmAmAmsCmsUm-3'
(SEQ ID NO: 162).

Preparation Examples 8-14. Synthesis of siRNAs provided by the present disclosure

According to the method described in Preparation Example 1 in WO2019105418(A1), the siRNA sequences listed in Table 2 were synthesized by solid-phase synthesis, the only difference being that DEPC water was used to dissolve the equimolar complementary sense strand and antisense strand in Table 2. After annealing, siRNA1-siRNA7 provided by the present disclosure were obtained, and their sequences are shown in Table 2.

Comparative Preparation Examples 4-10

Reference siRNA-reference siRNA7 were prepared according to the same method as in Preparation Examples 8-14.

Experimental Example 1. Toxic effects of siRNA conjugates in mice

Conjugate 1, conjugate 2 and reference conjugate 1 were dissolved in PBS to obtain a 10 mg/mL solution (based on siRNA conjugate). ICR mice (half were male and half female, weighing 18-22 g, 5-6 weeks old, purchased from SiPeiFu Inc.) were randomly divided into groups of 6 (half were male and half female) and numbered. In the manner of subcutaneous injection on the back of the neck, the siRNA conjugate solution was administered to each mouse respectively at an administration volume of 10 mL/kg, as a test group. In addition, PBS was administered to each of a group of mice at an administration volume of 10 mL/kg, as a blank control group.

Taking the administration time point as day 1, on day 8, each mouse in the test group and the blank control group was subjected to orbital blood collection. The blood collection volume was 0.6 mL. The blood was incubated at 37 °C for 60 min and then centrifuged at 3000 rpm at 4 °C for 15 min to obtain serum. The concentrations of alanine aminotransferase (ALT) and aspartate aminotransferase (AST) in serum were further detected by PM1P000/3 automatic serum biochemical analyzer (SABA, Italy). The results are shown in Table 1.

Further, after blood collection on day 8, the mice were sacrificed and dissected, preserved in 10% neutral buffered formalin fixative, and pathological sections were made. The severity of hepatic steatosis and inflammation in the pathological sections was evaluated and graded.

Table 1. Blood biochemical results of mice administered siRNA conjugates

| Experimental group | Reference conjugate 1 | Conjugate 1 | Conjugate 2 |
|---|---|---|---|
| Dose | 100 mg/kg | 100 mg/kg | 100 mg/kg |
| ALT | 420% | 116% | 118% |
| AST | 126% | - | 32% |

In Table 1, % and the preceding figures represent the difference between the concentration of alanine aminotransferase (ALT) or aspartate aminotransferase (AST) in the mouse serum and the concentration in the serum of the blank control group and the percentage of the concentration in the serum of the blank control group. For example, 420% in Table 1 means that the concentration of alanine aminotransferase in the mice administered reference conjugate 1 at 100 mg/Kg was 420% higher than that in the blank control group.

According to the results in Table 1, compared with the blank control group, the mice administered reference conjugate 1, which contained no stabilizing modified nucleotides, showed significant changes in blood biochemical indicators: the concentration of ALT was increased by 420%, and the concentration of AST was increased by 126%. In the mice administered siRNA conjugate 1 or 2 of the present disclosure, the concentrations of ALT were increased by only 116% and 118%, respectively, and the concentration of AST was increased by only 32%, showing significantly decreased blood biochemical indicators.

The results of pathological sections showed that compared with the blank control group, one of the 6 mice administered reference conjugate 1, which contained no stabilizing modified nucleotides, had a moderate hepatic inflammatory response, specifically manifested that a small amount of diffuse inflammatory cell infiltration was visible in the hepatic lobules thereof, diffuse proliferation of fibroblasts in hepatic sinusoids. Three mice had mild hepatic inflammatory reaction, focal infiltration of inflammatory cells in local hepatic lobules, and inflammatory necrosis of local hepatic lobule in 1 animal, individual hepatocytes with punctate necrosis. However, among the mice administered conjugate 1 of the present disclosure, only 2 mice showed mild hepatocyte inflammatory response, showing a small amount of inflammatory cell infiltration, and no moderate or worse inflammatory response and necrosis were found. Among the mice administered conjugate 2 of the present disclosure, only one mouse showed mild hepatocyte inflammatory response, and no moderate or worse inflammatory response and necrosis were found. Conjugates 1 and 2 exhibited significantly lower toxicity than reference conjugate 1.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 2. Toxic effects of siRNA conjugates in mice

Conjugate 6, conjugate 7 and reference conjugate 3 were respectively dissolved in PBS to obtain a 10 mg/mL solution and a 30 mg/mL solution (based on siRNA conjugate). ICR mice (half were male and half female, weighing 18-22 g, 5-6 weeks old, purchased from SiPeiFu Inc.) were randomly divided into groups and numbered. For each

conjugate at each concentration, the animals were divided into a 2-week group and a 4-week group, with 6 mice in each group (half were male and half female). In the manner of subcutaneous injection on the back of the neck, the siRNA conjugate solution was administered to each mouse at an administration volume of 10 mL/kg, as a test group. In addition, PBS was administered to each one in the two groups of mice at an administration volume of 10 mL/kg, as the blank control group in the 2-week group and 4-week group respectively.

Taking the administration time point as day 1, after 15 days, the 6 mice dosed with 300 mg/kg of the conjugate from the 2-week group were sacrificed and dissected, and preserved in 10% neutral buffered formalin fixative solution, and pathological sections were made. Six mice in the 4-week group of the test group and the blank control group were subjected to orbital blood collection on day 29, respectively. The blood collection volume was 0.6 mL, the collected blood was incubated at 37 °C for 60 min and then centrifuged at 3000 rpm at 4 °C for 15 min to obtain serum. The concentrations of alanine aminotransferase (ALT) and aspartate aminotransferase (AST) in serum were further detected by PM1P000/3 automatic serum biochemical analyzer (SABA, Italy), and compared to that of the blank control group, with results shown in Table 2. The severity of inflammatory cell infiltration and hepatocyte necrosis in the pathological sections was assessed, graded and compared.

Table 2. Blood biochemical results of mice administered siRNA conjugates

| Experimental group | | Reference conjugate 3 | | Conjugate 6 | | Conjugate 7 | |
|---|---|---|---|---|---|---|---|
| Dose | | 100 mg/kg | 300 mg/kg | 100 mg/kg | 300 mg/kg | 100 mg/kg | 300 mg/kg |
| ALT | D29 | - | F:212% | - | - | - | F:130% |
| AST | D29 | - | F:36% | - | - | - | F:32% |

In Table 2, F represents a female mouse, and % and the preceding figure represent the difference between the concentration of alanine aminotransferase (ALT) or aspartate aminotransferase (AST) in the mouse serum and the concentration in the serum of the blank control group and the percentage of the concentration in the serum of the blank control group. For example, F: 212% in Table 2 means that the concentration of alanine aminotransferase in female mice administered reference conjugate 3 at 300 mg/Kg was 212% higher than that in the blank control group.

According to the results in Table 2, compared with the blank control, mice administered reference conjugate 3, which contained no stabilizing modified nucleotides, showed significant changes in blood biochemical indicators. At a high dose of 300 mg/Kg, the concentrations of ALT and AST in female mice were increased by 212% and 36%, respectively. At the same dose, the concentrations of ALT and AST in female mice administered conjugate 7 of the present disclosure were increased by 130% and 32%, respectively. Compared with reference conjugate 3, the concentration of ALT decreased significantly. No increase was found in the concentrations of ALT and AST in the mice administered conjugate 6 of the present disclosure, and the degree of change in the blood biochemical indicators was significantly reduced compared with reference conjugate 3.

The results of pathological sections showed that compared with the blank control, the mice administered reference conjugate 3, which contained no stabilizing modified nucleotides, showed significant liver inflammation, and all 6 mice showed inflammatory cell infiltration. Whereas only 3 mice among the mice administered conjugate 6 showed inflammatory cell infiltration. Only 3 of the mice administered conjugate 7 showed inflammatory cell infiltration. Compared with the reference conjugate, there was also a significant reduction in the number of mice suffering from inflammatory cell infiltration after being administered the conjugates of the present disclosure.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 3. Toxic effects of siRNA conjugates in mice

According to the method in Experimental Example 2, the toxicity effect of the siRNA conjugate in mice was verified, the only difference being that the experiment was carried out with conjugate 3 and reference conjugate 2, and each conjugate was respectively dissolved in PBS to obtain a 10 mg/mL solution (based on siRNA conjugate). The 2-week group and 4-week group (marked as D15 group and D29 group in Table 19) each had 3 mice, all of which were male. That is, the toxic effects of conjugate 13 and reference conjugate 2 in mice were tested at a dose of 100 mg/kg.

Table 3. Blood biochemical results of mice administered siRNA conjugates

| Experimental group | | Reference conjugate 2 | Conjugate 3 |
|---|---|---|---|
| Dose | | 100 mg/kg | 100 mg/kg |
| ALT | D15 | 71% | - |
| | D29 | 118% | - |
| AST | D15 | 54% | - |
| | D29 | 94% | - |

In Table 3, % and the preceding figures represent the difference between the concentration of alanine aminotransferase (ALT) or aspartate aminotransferase (AST) in the mouse serum and the concentration in the serum of the blank control group and the percentage of the concentration in the serum of the blank control group.

According to the results in Table 3, compared with the blank control, the mice administered reference conjugate 2 containing no stabilizing modified nucleotide showed significant changes in blood biochemical indicators. On day 15 after the administration, the concentrations of ALT and AST in serum were increased by 71% and 54%, respectively, and on day 29 after administration, the concentrations of ALT and AST in serum were increased by 118% and 94%, respectively. However, no increase was observed in the serum ALT and AST concentrations in mice administered the same dose of conjugate 3. Conjugate 3 of the present disclosure showed significantly lower blood biochemical indicators.

The results of pathological sections showed that, compared with the blank control group, among the mice administered with the reference conjugate 2 containing no stabilizing modified nucleotides, 1 mice exhibited severe hepatocyte degeneration, specifically manifested that the gepatocyte swelling was extensively visible, the cytoplasms were loose and stained, vacuolar degeneration occurred to some liver cells, and tiny round vacuoles were visible in the cytoplasms. Two mice showed moderate hepatocyte degeneration, specifically manifested that many to extensive hepatocyte cytoplasms were loose and stained, vacuolar degeneration occurred to some hepatocytes, and tiny round vacuoles were visible in the cytoplasms. Of the mice dosed with siRNA conjugate 3 of the present disclosure, 3 showed mild hepatocyte degeneration, with a lower number of hepatocytes undergoing cytoplasmic rarefaction. The degree of hepatocyte degeneration was significantly reduced in histopathology, showing significantly lower toxicity compared to the reference conjugate.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 4. Toxic effects of siRNA conjugates in mice

Conjugate 3 and conjugate 5 were respectively dissolved in PBS to obtain a 30 mg/mL solution (based on siRNA conjugate). ICR mice (half were male and half female, weighing 18-22 g, 5-6 weeks old, purchased from SiPeiFu Inc.) were randomly divided into groups of 10 (half were male and half female) and numbered. In the manner of subcutaneous injection on the back of the neck, the siRNA conjugate solution was administered to each mouse respectively at an administration volume of 10 mL/kg, as a test group. In addition, PBS was administered to each of a group of mice at an administration volume of 10 mL/kg, as a blank control group.

Taking the time point of the first administration as day 1, each mouse was repeatedly administered on day 8 and day 15, and the concentration and administration volume of the siRNA conjugate solution (or PBS) used were the same as those of the first administration. Each mouse in the test group and the blank control group was subjected to orbital blood collection on day 16. The blood collection volume was 0.6 mL, and the collected blood was incubated at 37 °C for 60 min and then centrifuged at 3000 rpm at 4 °C for 15 min to obtain serum. The concentrations of alanine aminotransferase (ALT) and aspartate aminotransferase (AST) in serum were further detected by PM1P000/3 automatic serum biochemical analyzer (SABA, Italy). The results are shown in FIG. 1A and FIG. 1B.

FIGs. 1A and 1B are scatter plots showing ALT and AST concentrations in mouse serum after weekly administration of 300 mg/kg of siRNA conjugates of the present disclosure or PBS for three consecutive weeks. As shown in FIGs. 1A and 1B, compared with the blank control group, after administration of the conjugates of the present disclosure, the serum ALT and AST concentrations were comparable to those of the blank control group, indicating that the siRNA conjugates of the present disclosure have very low hepatotoxicity.

Further, after blood collection, the mice were sacrificed and dissected, and preserved in 10% neutral buffered

formalin fixative, and pathological sections were made. A relative comparison was made. The pathological sections showed that the mice administered siRNA conjugate 3 or conjugate 5 of the present disclosure showed a similar response to the blank control group in terms of hepatic steatosis and inflammation, without significant abnormalities. It also indicates that the siRNA conjugates of the present disclosure have very low hepatotoxicity.

The above results show that the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 4. Inhibition activity of siRNA conjugates in the *in vitro* sicheck system

In this Experimental Example, the *in vitro* sicheck system was used to detect the inhibition activity of conjugate 1, conjugate 2, conjugate 3, conjugate 4, conjugate 6, conjugate 7, reference conjugate 1, reference conjugate 2, reference conjugate 3 or reference siRNA NC on the target sequence in the *in vitro* sicheck system.

[1] Construction of detection plasmid

A detection plasmid was constructed using the psiCHECK™-2 (Promega™) plasmid, and the plasmid contained a target sequence 1, i.e., the siRNA target sequence. For the siRNA conjugate to be tested, target sequence 1 is as follows:

TGCTCAGTTCATCCCTAGAGGCAGCTGCTCCAGGAACAGAGGTGCCATGCAGCCCCGGG
TACTCCTTGTTGTTGCCCTCCTGGCGCTCCTGGCCTCTGCCCGAGCTTCAGAGGCCGAG
GATGCCTCCCTTCTCAGCTTCATGCAGGGTTACATGAAGCACGCCACCAAGACCGCCAA
GGATGCACTGAGCAGCGTGCAGGAGTCCCAGGTGGCCCAGCAGGCCAGGGGCTGGGT
GACCGATGGCTTCAGTTCCCTGAAAGACTACTGGAGCACCGTTAAGGACAAGTTCTCTG
AGTTCTGGGATTTGGACCCTGAGGTCAGACCAACTTCAGCCGTGGCTGCCTGAGACCTC
AATACCCCAAGTCCACCTGCCTATCCATCCTGCGAGCTCCTTGGGTCCTGCAATCTCCAG
GGCTGCCCCTGTAGGTTGCTTAAAAGGGACAGTATTCTCAGTGCTCTCCTACCCCACCTC
ATGCCTGGCCCCCCTCCAGGCATGCTGGCCTCCCAATAAAGCTGGACAAGAAGCTGCTA
TG (SEQ ID NO: 163)

Target sequence 1 is a nucleotide sequence in the mRNA expressed by the human APOC3 gene targeted by the tested siRNA, so the inhibition effect of each siRNA conjugate on the target sequence 1 may reflect the abilities of the tested siRNA conjugate to inhibit APOC3 gene expression. Target sequence 1 and the complementary sequence thereof were cloned into the Xho I/Not I site of the psiCHECK™-2 plasmid.

[2] Transfection

HEK293A cells (purchased from Nanjing CoBioer Biosciences Co., Ltd.) were cultured at 37 °C in DMEM complete medium (Hyclone Inc.) supplemented with 20% fetal bovine serum (FBS, Hyclone Inc.) and 0.2 vol% of penicillin-streptomycin (Gibco, Invitrogen Inc.) in an incubator containing 5% $CO_2$/95% air.

HEK293A cells were seeded at $8 \times 10^3$ cells/well in 96-well plates. When the cell growth density reached 70-80% after 16 h, H-DMEM complete medium was aspirated off the wells, and 80 μL of Opti-MEM medium (GIBCO Inc.) was added to each well for an additional 1.5 h.

DEPC water was used to dilute the detection plasmid into 200 ng/μL detection plasmid working solution; DEPC water was used to prepare each of the following siRNA conjugates or reference siRNA NC into three kinds of siRNA conjugate working solution or reference siRNA NC working solution with three different concentrations (based on siRNA): 10 nM, 3 nM, and 1 nM. The siRNA conjugates used were conjugate 1, conjugate 2, conjugate 3, conjugate 4, conjugate 6, conjugate 7, reference conjugate 1, reference conjugate 2 and reference conjugate 3 prepared.

For each siRNA conjugate or reference siRNA NC, 1A1-1A3 solutions were prepared, and each 1A1-1A3 solution contained 1 μL of the 3 concentrations of siRNA conjugate working solution or reference siRNA NC working solution, 0.05 μL of detection plasmid working solution (containing 10 ng of the detection plasmid) and 10 μL of Opti-MEM medium.

1B solutions were prepared, each 1B solution containing 0.2 μL of Lipofectamine™ 2000 and 10 μL of Opti-MEM media.

1C solutions were prepared, each 1C solution containing 0.05 μL of the detection plasmid working solution (containing 10 ng of the detection plasmid) and 10 μL of Opti-MEM media.

An aliquot of the 1B solution was mixed with the resulting aliquot of the 1A1-1A3 solutions of each siRNA conjugate or reference siRNA NC, and incubated at room temperature for 20 min to obtain transfection complexes 1X1-1X3 for each siRNA conjugate or reference siRNA NC.

An aliquot of the 1B solution was mixed with an aliquot of the 1C solution, and incubated at room temperature for 20 min, respectively, to obtain a blank transfection complex 1X4.

Transfection complexes 1X1-1X3 for each siRNA conjugate or reference siRNANC were added to the culture wells in an amount of 20 μL/well, respectively, and mixed uniformly to obtain transfection complexes for each siRNA conjugate or reference siRNA NC at final concentrations of about 0.1 nM, 0.03 nM and 0.01 nM (based on siRNA). The transfection complexes 1X1-1X3 for each siRNA conjugate or reference siRNA NC were transfected into 3 culture wells, respectively, to obtain a co-transfection mixture containing siRNA conjugates or reference siRNA NC, which was designated as a test group.

For each siRNA conjugate or reference siRNA NC, the transfection complex 1X4 was added into another 3 culture wells in an amount of 20 μL/well to obtain a transfection mixture without siRNA, which was designated as a blank control group.

The co-transfection mixture with siRNA and the co-transfection mixture without siRNA were respectively transfected in culture wells for 4 h, and 100 μL of H-DMEM complete medium containing 20% FBS was added to each well. The 96-well plate was placed in a $CO_2$ incubator and incubated for another 24 h.

[3] Detection

The culture media in the culture wells were aspirated, and 150 μL of Dual-Glo® Luciferase reagents and H-DMEM mixed solution (the volume ratio was 1:1) were added into each well, mixed fully and uniformly, and incubated at room temperature for 10 min; 120 μL of the mixed solution was transferred onto a 96-well microplate, and the chemiluminescence values (Fir) of Firefly in each culture well on the 96-well microplate was read by using a Synergy II multifunctional microplate reader (BioTek company); 60 μL of Dual-Glo® Stop & Glo® reagent was added into every well of the 96-well microplate again, mixed fully and uniformly, and incubated at room temperature for 10 min; and the chemical luminescence value (Ren) of *Renilla* in each culture well of the 96-well microplate was read with a microplate reader according to the arrangement of reading Fir.

The luminescence ratio Ratio = Ren / Fir of each well on the 96-well microplate was calculated, the luminescence ratio Ratio (test) of each test group or Ratio (control) of each control group being the average value of Ratio of three culture wells; with the luminescence ratio of the control group as the baseline, the luminescence ratio of each test group was normalized to obtain the ratio R of Ratio (test) / Ratio (control), which represented the relative expression level of the *Renilla* reporter gene, that is, the residual activity. Inhibition rate of each siRNA conjugate or reference siRNA NC against target sequence 1 = (1 - R) × 100%.

The inhibition effect of each siRNA conjugate or reference siRNA NC on target sequence 1 is shown in FIG. 2. FIG. 2 is a bar chart showing the relative expression levels of target sequence 1 in the *in vitro* sicheck system after co-transfection with plasmids containing target sequence 1 and siRNA conjugates to be tested or reference siRNA NC. Further, the expression inhibition rates of the siRNA conjugates or reference siRNA NC against target sequence 1 are summarized in Table 3.

Table 4. Target sequence 1 expression inhibition rates in the *in vitro* sicheck system

| siRNA conjugate No. (reference siRNA No.) | Target sequence 4 expression inhibition ratio (%) | | |
|---|---|---|---|
| | 0.1 nM | 0.03 nM | 0.01 nM |
| Conjugate 6 | 85.17 | 66.64 | 38.92 |
| Conjugate 7 | 88.32 | 74.61 | 52.08 |
| Reference conjugate 3 | 88.80 | 73.81 | 45.12 |
| Conjugate 3 | 88.72 | 77.90 | 55.52 |
| Conjugate 4 | 88.50 | 77.85 | 52.61 |
| Reference conjugate 2 | 91.04 | 82.47 | 64.65 |

(continued)

| siRNA conjugate No. (reference siRNA No.) | Target sequence 4 expression inhibition ratio (%) | | |
|---|---|---|---|
| | 0.1 nM | 0.03 nM | 0.01 nM |
| Conjugate 1 | 89.35 | 83.33 | 67.54 |
| Conjugate 2 | 84.73 | 74.36 | 50.13 |
| Reference conjugate 1 | 86.20 | 78.95 | 59.69 |
| Reference siRNA NC | 3.15 | -11.56 | 8.68 |

According to the results in FIG. 2 and Table 4, the siRNA conjugates provided by the present disclosure has very high target sequence inhibition activity in the *in vitro* sicheck system. At a low concentration of 0.01 nM, the target sequence 1 expression inhibition rate was at least 38.92 nM and could reach up to 67.54%. At a concentration of 0.1 nM, the target sequence 1 expression inhibition rate was at least 84.73% and could reach up to 89.35%. Meanwhile, the siRNA conjugates have similar target sequence inhibition activity to reference conjugate 1, reference conjugate 2 or reference conjugate 3, which contained no stabilizing modified nucleotides.

Experimental Example 6. Inhibition activity of siRNA conjugates in the *in vitro* sicheck system In this Experimental Example, the *in vitro* sicheck system was used to detect the inhibition activity of conjugate 6, conjugate 7 or reference conjugate 3 on the target sequence in the *in vitro* sicheck system.

According to method described in Kumico Ui-Tei et al., Functional dissection of siRNA sequence by systematic DNA substitution: modified siRNA with a DNA seed arm is a powerful tool for mammalian gene silencing with significantly reduced off-target effect. Nucleic Acids Research, 2008.36(7), 2136-2151, a detection plasmid was constructed, and HEK293A cells were co-transfected with the detection plasmid and conjugate to be tested. The target sequence inhibition activity of the siRNAs was reflected by the expression level of the dual luciferase reporter gene. Specific steps are as follows:

[1] Construction of detection plasmid

A detection plasmid was constructed using the psiCHECK™-2 (Promega™) plasmid, and the plasmid contained a target sequence 2, i.e., the siRNA conjugate target sequence. For the siRNA conjugate to be tested, target sequence 2 is as follows:

TTGCTTAAAAGGGACAGTATTCTCAGTGCTCTCCTACC (SEQ ID NO: 164)

Target sequence 2 is a sequence completely complementary to the antisense strands of the tested siRNA conjugate, so the inhibition effect of each siRNA conjugate on the target sequence 2 can reflect the abilities of the tested siRNA conjugate to inhibit target gene expression. Target sequence 2 and the complementary sequence thereof were cloned into the Xho I/Not I site of the psiCHECK™-2 plasmid.

[2] Transfection

HEK293A cells (purchased from Nanjing CoBioer Biosciences Co., Ltd.) were cultured at 37 °C in DMEM complete medium (Hyclone Inc.) supplemented with 20% fetal bovine serum (FBS, Hyclone Inc.) and 0.2% by volume of penicillin-streptomycin diabody (Gibco, Invitrogen Inc.) in an incubator containing 5% $CO_2$/95% air.

HEK293A cells were seeded at $8 \times 10^3$ cells/well in 96-well plates. When the cell growth density reached 70-80% after 16 h, H-DMEM complete medium was aspirated off the wells, and 80 μL of Opti-MEM medium (GIBCO Inc.) was added to each well for an additional 1.5 h.

The detection plasmid was diluted into 200 ng/μL detection plasmid working solution with DEPC water; each siRNA conjugate in Table 4 was formulated into siRNA conjugate working solutions at the following 11 different concentrations with DEPC water: 1.00 μM, 0.330 μM, 0.110 μM, 0.0370 μM, 0.0123 μM, 0.00412 μM, 0.00137 μM, 0.000457 μM, 0.000152 μM, 0.0000508 μM, and 0.0000169 μM (based on the amount of siRNA in the siRNA conjugate). The siRNA conjugates used were respectively conjugate 6, conjugate 7 and reference conjugate 3 prepared as described above.

For each siRNA conjugate, 2A1-2A11 solutions were prepared, and each of the 2A1-2A11 solutions sequentially contained 1 μL of the 11 concentrations of siRNA working solution, respectively, 0.05 μL of detection plasmid working solution (containing 10 ng of the detection plasmid) and 10 μL of Opti-MEM medium.

2B solutions were prepared, each 2B solution containing 0.2 μL of Lipofectamine™ 2000 and 10 μL of Opti-

MEM media.

2C solutions were prepared, each 2C solution containing 0.05 μL of the detection plasmid working solution (containing 10 ng of the detection plasmid) and 10 μL of Opti-MEM media.

An aliquot of the 2B solution was mixed with the resulting aliquot of 2A1-2A11 solutions for each siRNA conjugate, and incubated at room temperature for 20 min, respectively, to obtain transfection complexes 2X1-2X11 for each siRNA conjugate.

An aliquot of the 2B solution was mixed with an aliquot of the 2C solution, and incubated at room temperature for 20 min, respectively, to obtain a transfection complex 2X12.

In the culture wells, transfection complexes 2X1-2X11 for each siRNA were added, respectively, and mixed uniformly in an amount of 20 μL/well to obtain transfection complexes for each siRNA at final concentrations of about 0.01 μM, 0.0033 μM, 0.0011 μM, 0.00037 μM, 0.000123 μM, 0.0000412 μM, 0.0000137 μM, 0.00000457 μM, 0.00000152 μM, 0.000000508 μM and 0.000000169 μM, respectively (based on the amount of siRNA in the siRNA conjugate). The transfection complexes 2X1-2X11 for each siRNA conjugate were transfected into 3 culture wells, respectively, to obtain a co-transfection mixture containing the siRNA conjugate, which was designated as a test group.

For each siRNA conjugate, transfection complex 2X12 was added into another 3 culture wells in an amount of 20 μL/well to obtain a transfection mixture without an siRNA conjugate, which was designated as a blank control group.

The co-transfection mixture with the siRNA conjugate and the co-transfection mixture without an siRNA conjugate were respectively transfected in culture wells for 4 h, and 100 μL of H-DMEM complete medium containing 20% FBS was added to each well. The 96-well plate was placed in a $CO_2$ incubator and incubated for another 24 h.

[3] Detection

The culture media in the culture wells were aspirated, and 150 μL of Dual-Glo® Luciferase reagents and H-DMEM mixed solution (the volume ratio is 1: 1) were added into each well, mixed fully and uniformly, and incubated at room temperature for 10 min; 120 μL of the mixed solution was transferred onto a 96-well microplate, and the chemiluminescence values (Fir) of Firefly in each culture well on the 96-well microplate was read by using a Synergy II multifunctional microplate reader (BioTek company); 60 μL of Dual-Glo® Stop & Glo® reagent was added into every well of the 96-well microplate again, mixed fully and uniformly, and incubated at room temperature for 10 min; and the chemical luminescence value (Ren) of Renilla in each culture well of the 96-well microplate was read with a microplate reader according to the arrangement of reading Fir.

The luminescence ratio Ratio = Ren / Fir of each well on the 96-well microplate was calculated, the luminescence ratio Ratio (test) of each test group or Ratio (control) of each control group being the average value of Ratio of three culture wells; with the luminescence ratio of the control group as the baseline, the luminescence ratio of each test group was normalized to obtain the ratio R of Ratio (test) / Ratio (control), which represented the relative expression level of the *Renilla* reporter gene, that is, the residual activity. Inhibition rate of siRNA to target sequence = (1 - R) × 100%.

According to the relative residual activity of *Renilla* in HEK293A cells transfected with different concentrations of siRNA to be tested, the dose-effect curve of log (inhibitor) vs. response-Variable slope (four parameters) was fitted using the nonlinear regression analysis function of Graphpad 5.0 software.

According to the function corresponding to the fitted dose-effect curve, the $IC_{50}$ value of the target sequence targeted by the siRNA to be tested was calculated, with the function shown as follows:

$$Y = \mathrm{Bot} + \frac{\mathrm{Top} - \mathrm{Bot}}{1 + 10^{(X' - X) \times \mathrm{HillSlope}}}$$

wherein:

Y is the ratio R, the relative residual activity of *Renilla,*

X is the logarithmic value of transfection siRNA concentration,

Bot is the Y value at the bottom of the steady state period,

Top is the Y value at the top of the steady state period, and

X' is the corresponding X value when Y is halfway between the bottom and the top, and HillSlope is the slope of the curve at X'.

From the dose-effect curve and the corresponding function, the corresponding $X_{50}$ value when Y = 50% was determined, the $IC_{50}$ value of each siRNA was calculated by $IC_{50} = 10^{\wedge}X_{50}$ (nM), and the $IC_{50}$ values are summarized in Table 5.

Table 5. $IC_{50}$ of siRNA conjugates in the pscheck system

| Conjugate No. | $IC_{50}$ |
|---|---|
| Conjugate 6 | 8.55 pM |
| Conjugate 7 | 6.89 pM |
| Reference conjugate 3 | 6.68 pM |

According to the results in Table 5, the siRNA conjugates of the present disclosure have very high target sequence inhibition activity in the *in vitro* sicheck system, with $IC_{50}$ of 6.89-8.55 pM. Meanwhile, the siRNA conjugates have similar target sequence inhibition activity to reference conjugate 3, which contained the same sequence but no stabilizing modified nucleotides.

Experimental Example 7. Inhibition activity of siRNA conjugates in the *in vitro* sicheck system

The off-target sequence inhibition activity of conjugate 3 and conjugate 6 in the *in vitro* sicheck system was detected according to the method in Experimental Example 6. The only difference is that conjugate 3 or conjugate 6 was tested instead of the tested siRNA conjugate. For conjugate 6, the target sequence used was target sequence 2, or target sequences 3-5 as shown below were used instead of target sequence 2. For conjugate 3, the target sequence used was target sequence 2, or target sequences 6-8 as shown below were used instead of target sequence 2, respectively:

Target sequence 3:
TAGGCCCCTTTCAAGTATTCT (SEQ ID NO: 165)

Target sequence 4:
AGAATACTGTCCCTTTTAAGC (SEQ ID NO: 166)

Target sequence 5:
CTCCGCAGTGAAATTTTAAGC (SEQ ID NO: 167)

Target sequence 6:
CTTTCACTGCGGATCAGTGCT(SEQ ID NO: 168)

Target sequence 7:
AGCACTGAGAATACTGTCCCT (SEQ ID NO: 169)

Target sequence 8:
CTACAGTCTCCGCCTGTCCCT (SEQ ID NO: 170)

Among them, target sequence 2 contains a sequence that is completely complementary to the antisense strands of the siRNAs in conjugate 3 and conjugate 6, so the inhibition effect of conjugate 3 or 6 on target sequence 2 can reflect the ApoC3 mRNA inhibition activity of conjugate 3 or 6. Target sequence 3 contains a sequence that is partially complementary to the antisense strand of the siRNA in conjugate 6, target sequence 4 contains a sequence that is completely complementary to the sense strand of the siRNA in conjugate 6, and target sequence 5 contains a sequence that is partially complementary to the sense strand of the siRNA in conjugate 6. Therefore, the inhibition effect of conjugate 6 on target sequence 3, 4 or 5 may reflect the degree of off-target effect. That is, the higher the inhibition effect, the more likely conjugate 6 is off-target. Similarly, target sequence 6 contains a sequence that is partially complementary to the antisense strand of siRNA in conjugate 3, target sequence 7 contains a sequence that is completely complementary to the sense strand of siRNA in conjugate 3, and target sequence 8 contains a sequence that is partially complementary to the sense strand of siRNA in conjugate 3. Therefore, the inhibition effect of conjugate 3 on target sequence 6, 7 or 8 may reflect the degree of off-target effect. That is, the higher the inhibition effect, the more likely conjugate 3 is off-target.

As a result, in the *in vitro* sicheck system, the inhibitory $IC_{50}$ of conjugate 6 on target sequence 2 was 11.3 pM,

and the inhibition rate on target sequence 3, 4 or 5 was less than 50% within the concentration ranges of all tested siRNAs, that is, none of them was off-target. The inhibitory $IC_{50}$ of conjugate 3 on target sequence 2 was 4.50 pM, and the inhibition rate on target sequence 6, 7 or 8 was less than 50% within the concentration ranges of all tested siRNAs, that is, none was off-target.

Hence, in the *in vitro* sicheck system, the siRNA conjugates of the present disclosure exhibited excellent target sequence on-target inhibition activity, with an $IC_{50}$ value of 4.50 pM-11.3 pM. Meanwhile, the siRNA conjugates of the present disclosure have low off-target effects.

Experimental Example 8. Inhibition activity of siRNA conjugates in the *in vitro* sicheck system

According to the method in Experimental Example 6, the inhibition activity of conjugate 5 in the *in vitro* sicheck system was tested, the only difference being that conjugate 5 was tested instead of the tested siRNA conjugate. As a result, conjugate 5 showed high target sequence inhibition activity in the *in vitro* sicheck system, with $IC_{50}$ of 49.8 pM.

Experimental Example 9. Lowering effects of siRNA conjugates on blood lipid in mice *(in vivo)*

Human APOC3 transgenic mice Tg(APOC3)3707Bres (purchased from Jackson Laboratory, USA) with serum TG content > 2 mmol/L were selected and randomly divided into groups of 6, half of which were male and half female. Conjugate 6, reference conjugate 3 and PBS blank control were administered to each group of mice respectively. All animals were dosed according to their body weight, and administered in a single subcutaneous injection. The dose of each siRNA conjugate (based on the amount of siRNA) was 3 mg/kg mouse body weight, and the administration volume was 5 mL/kg. Each siRNA conjugate was provided in PBS aqueous solution, and the concentration that the conjugate should be prepared was calculated according to the dose and volume of administration. Each of the mice in the other group was administered 1× PBS with a volume of 5 mL/kg, serving as a blank control group.

Taking the administration time point as day 1, blood was collected from the orbital venous plexus of the mice on days 1, 9 and 15, with 100 μL each time. The collected blood was left at room temperature for 30 min, and then centrifuged at 3000 rpm at 4 °C for 15 min to obtain serum. PM1P000/3 automatic serum biochemical analyzer (SABA, Italy) was then used to detect the content of total cholesterol (CHO) and triglyceride (TG) in serum.

Standardized blood lipid level = (blood lipid content of test group after administration/blood lipid content of test group before administration) × 100%.

Inhibition rate of blood lipid level = (1 - blood lipid content of test group after administration/blood lipid content of test group before administration) × 100%.

Blood lipid refers to total cholesterol (CHO) or triglyceride (TG).

FIGs. 3A and 3B are line graphs showing changes in the level of serum TG or serum CHO over time after administration of a siRNA conjugate of the present disclosure, a reference siRNA conjugate or PBS. Further, the serum TG inhibition rates and serum CHO inhibition rates at these time points are summarized in Table 6A and Table 6B:

Table 6A. Serum TG inhibition rates of siRNA conjugates in transgenic mice

| Group | Dose mg/kg | TG level inhibition rate (%) | |
|---|---|---|---|
| | | D9 | D15 |
| Blank control | / | 8.2 | 19.2 |
| Reference conjugate 3 | 3 | 88.2 | 89.0 |
| Conjugate 6 | 3 | 77.3 | 82.8 |

Table 6B. Serum CHO inhibition rates of siRNA conjugates in transgenic mice

| Group | Dose mg/kg | CHO level inhibition rate (%) | |
|---|---|---|---|
| | | D9 | D15 |
| Blank control | / | -1.9 | 9.5 |
| Reference conjugate 3 | 3 | 52.2 | 54.8 |
| Conjugate 6 | 3 | 46.9 | 42.0 |

According to the results in FIGs. 3A and 3B and Tables 6A and 6B, at different time points after administration, conjugate 6 can significantly reduce the levels of TG and CHO in mouse serum, and showed a blood lipid level lowering effect that decreased not more than 11% compared with the corresponding reference conjugate 3, which contained no stabilizing modified nucleotides.

Experimental Example 10. Lowering effects of siRNA conjugates on blood lipid in mice (*in vivo*)

The effect of conjugate 7 of the present disclosure on the reduction of blood lipid in mice was examined by using the method in Experimental Example 9. The only difference was that conjugate 7, reference conjugate 3 and PBS blank control were administered to each group of mice, respectively. All animals were dosed according to their body weight, and administered in a single subcutaneous injection. The dose of each siRNA conjugate (based on the amount of siRNA) was 3 mg/kg and 1 mg/kg mouse body weight, and the administration volume was 5 mL/kg. Taking the administration time point as day 1, blood was collected from the orbital venous plexus of the mice on days 1, 9, 15, 22, 29, 36 and 50.

FIGs. 4A and 4B are line graphs showing changes in the level of serum TG or serum CHO over time after administration of a siRNA conjugate of the present disclosure, a reference siRNA conjugate or PBS. Further, the serum TG inhibition rates and serum CHO inhibition rates at these time points are summarized in Table 7A and Table 7B:

Table 7A. Serum TG inhibition rates of siRNA conjugates in transgenic mice

| Group | Dose mg/kg | TG level inhibition rate (%) | | | | | |
|---|---|---|---|---|---|---|---|
| | | D9 | D15 | D22 | D29 | D36 | D50 |
| Blank control | / | 8.2 | 19.2 | -3.6 | 3.5 | 3.7 | -16.3 |
| Reference conjugate 3 | 3 | 88.2 | 89.0 | 88.6 | 85.9 | 86.9 | 85.4 |
| | 1 | 86.1 | 86.5 | 83.7 | 78.1 | 80.5 | 70.0 |
| Conjugate 7 | 3 | 90.2 | 88.6 | 88.2 | 86.5 | 86.1 | 82.7 |
| | 1 | 83.9 | 80.8 | 76.5 | 74.3 | 67.0 | 54.0 |

Table 7B. Serum CHO inhibition rates of siRNA conjugates in transgenic mice

| Group | Dose mg/kg | CHO level inhibition rate (%) | | | | | |
|---|---|---|---|---|---|---|---|
| | | D9 | D15 | D22 | D29 | D36 | D50 |
| Blank control | / | -1.9 | 9.5 | 2.8 | 2.1 | -1.5 | -1.3 |
| Reference conjugate 3 | 3 | 52.2 | 54.8 | 56.3 | 53.2 | 50.8 | 53.2 |
| | 1 | 47.5 | 44.2 | 39.5 | 42.8 | 44.6 | 41.5 |
| Conjugate 7 | 3 | 51.4 | 43.8 | 53.2 | 47.6 | 46.0 | 47.3 |
| | 1 | 51.8 | 45.8 | 51.2 | 45.5 | 40.7 | 39.1 |

According to the results in FIGs. 4A and 4B and Tables 7A and 7B, at different time points after administration, conjugate 7 can significantly reduce the levels of TG and CHO in mouse serum, and showed a blood lipid level lowering effect equal to and even better than that of the corresponding reference conjugate 3, which contained no stabilizing modified nucleotides. In particular, at a dose of 3 mg/kg, conjugate 7 has always shown a very high blood lipid TG

lowering effect throughout the administration period of up to 50 days, and the highest inhibition rate can reach up to 90.2%.

Experimental Example 11. Lowering effects of siRNA conjugates on blood lipid in mice (*in vivo*)

According to the method in Experimental Example 9, the blood lipid-lowering effect of the siRNA conjugate in mice was tested, the only difference being that the siRNA conjugate used was conjugate 3 or conjugate 4. The results are shown in FIGs. 9A and 9B.

FIGs. 9A and 9B are line graphs showing changes in the level of serum TG or serum CHO over time after administration of siRNA conjugates of the present disclosure or PBS. Further, the serum TG inhibition rates and serum CHO inhibition rates at these time points are summarized in Table 8A and Table 8B:

Table 8A. Serum TG inhibition rates of siRNA conjugates in transgenic mice

| Group | Dose mg/kg | TG level inhibition rate (%) | | | | | |
|---|---|---|---|---|---|---|---|
| | | D9 | D15 | D22 | D29 | D36 | D50 |
| Blank control | / | 8.2 | 19.2 | -3.6 | 3.5 | 3.7 | -16.3 |
| Conjugate 3 | 3 | 87.5 | 87.1 | 85.3 | 77.8 | 63.2 | 55.9 |
| | 1 | 81.7 | 82.2 | 80.8 | 65.3 | 58.8 | 31.2 |
| Conjugate 4 | 3 | 90.5 | 92.0 | 90.3 | 82.5 | 81.3 | 66.0 |
| | 1 | 85.2 | 89.5 | 80.1 | 69.2 | 57.2 | 53.0 |

Table 8B. Serum CHO inhibition rates of siRNA conjugates in transgenic mice

| Group | Dose mg/kg | CHO level inhibition rate (%) | | | | | |
|---|---|---|---|---|---|---|---|
| | | D9 | D15 | D22 | D29 | D36 | D50 |
| Blank control | / | -1.9 | 9.5 | 2.8 | 2.1 | -1.5 | -1.3 |
| Conjugate 3 | 3 | 53.4 | 48.5 | 53.6 | 45.7 | 47.6 | 40.6 |
| | 1 | 50.7 | 48.9 | 50.7 | 37.6 | 40.9 | 24.9 |
| Conjugate 4 | 3 | 47.1 | 43.7 | 51.1 | 45.2 | 53.2 | 42.0 |
| | 1 | 53.0 | 48.3 | 48.1 | 42.2 | 40.1 | 39.8 |

The results in FIG. 5A, FIG. 5B and Tables 8A and 8B show that at different time points after administration, conjugate 3 and conjugate 4 can significantly reduce the levels of TG and CHO in mouse serum. In particular, at doses of 3 mg/kg and 1 mg/kg, conjugate 4 has always shown a very high blood lipid TG lowering effect throughout the administration period of up to 50 days, and the highest inhibition rate can reach 92.0%.

Experimental Example 12. Lowering effects of siRNA conjugates on blood lipid in mice *(in vivo)*

According to the method in Experimental Example 9, the lowering effect of siRNA conjugates on blood lipids in mice was detected, the only difference being that the siRNA conjugates used were conjugate 3, and the dose of each siRNA conjugate (based on siRNA) was 9 mg/kg, 3 mg/kg, 1 mg/kg, 0.5 mg/kg, 0.25 mg/kg, 0.1 mg/kg or 0.05 mg/kg mouse body weight, and the administration volume was 5 mL/kg. Each siRNA conjugate was provided in PBS aqueous solution, and the concentration that the conjugate should be prepared was calculated according to the dose and volume of administration. The administration time point was recorded as day 1, and blood was collected from the orbital venous plexus of mice on days 1, 8, 15, 22, 29, 36, 43, 50, 57 and 64 to detect the level of TG in serum. The results are shown in FIG. 6.

FIG. 6 is a line graph showing changes in the serum TG level over time after administration of different concentrations of conjugate 3 or PBS. Further, the serum TG inhibition rates at these time points are summarized in Table 9:

Table 9. Serum TG inhibition rates of siRNA conjugates in transgenic mice

| Group | Dose mg/kg | TG level inhibition rate (%) | | | | | | | | |
|---|---|---|---|---|---|---|---|---|---|---|
| | | D8 | D15 | D22 | D29 | D36 | D43 | D50 | D57 | D64 |
| Blank control | / | 27.1 | 13.1 | 4.0 | 8.2 | 22.7 | 15.3 | 17.4 | -0.3 | 17.4 |
| Conjugate 3 | 9 | 89.5 | 88.5 | 89.0 | 87.9 | 88.3 | 86.6 | 71.3 | 59.7 | 54.7 |
| | 3 | 88.3 | 86.1 | 87.4 | 79.8 | 70.7 | 63.1 | 45.2 | 30.6 | 24.5 |
| | 1 | 76.3 | 68.2 | 54.4 | 63.1 | 57.9 | 39.8 | 15.5 | 5.0 | 7.0 |
| | 0.5 | 67.4 | 51.9 | 45.3 | 37.7 | 30.3 | 13.1 | 11.7 | 0.8 | 16.1 |
| | 0.25 | 32.6 | 18.3 | 32.1 | 34.7 | 25.0 | 15.2 | 7.3 | 5.3 | 5.3 |
| | 0.1 | 39.1 | 39.4 | 23.6 | 35.1 | 27.4 | 13.5 | 19.9 | 1.7 | 10.9 |
| | 0.05 | 47.8 | 38.7 | 5.6 | 17.0 | 14.6 | -3.7 | -21.6 | -13.2 | -12.5 |

The results in FIG. 6 and Table 9 show that at different time points after administration, different concentrations of conjugate 3 can all reduce the TG level in mouse serum. Especially, at a dose of 9 mg/kg, the siRNA conjugate of the present disclosure, after only one administration, can maintain a TG level inhibition rate of greater than 50% for as long as 64 days, and the inhibition rate can reach up to 89.5%, showing an excellent blood lipid inhibition ability.

Experimental Example 13. Lowering effects of siRNA conjugates on blood lipid in mice *(in vivo)*

Human APOC3 transgenic mice Tg(APOC3)3707Bres (purchased from Jackson Laboratory, USA) with serum TG content > 2 mmol/L were selected and randomly divided into groups of 8, half of which were male and half female. Conjugate 3, reference conjugate 5 and PBS blank control were administered to each group of mice respectively. All animals were dosed according to their body weight, and administered in a single subcutaneous injection. The dose of each siRNA conjugate (based on the amount of siRNA) was 3 mg/kg and 1 mg/kg mouse body weight, and the administration volume was 5 mL/kg. Each siRNA conjugate was provided in PBS aqueous solution, and the concentration that the conjugate should be prepared was calculated according to the dosage and volume of administration. Each of the mice in the other group was administered 1× PBS with a volume of 5 mL/kg, serving as a blank control group.

The time point of administration was recorded as day 1, and blood was collected from the orbital venous plexus of the mice on days 1, 8, 15, 22, 29, 36, and 43, with 100 μL each time. The collected blood was left at room temperature for 30 min, and then centrifuged at 3000 rpm at 4 °C for 15 min to obtain serum. PM1P000/3 automatic serum biochemical analyzer (SABA, Italy) was then used to detect the contents of total cholesterol (CHO) and triglyceride (TG) in serum.

Standardized blood lipid level = (blood lipid content of test group after administration/blood lipid content of test group before administration) × 100%.

Inhibition rate of blood lipid level = (1 - blood lipid content of test group after administration/blood lipid content of test group before administration) × 100%.

Blood lipid refers to total cholesterol (CHO) or triglyceride (TG).

FIGs. 7A and 7B are line graphs showing changes in the level of serum TG or serum CHO over time after administration of siRNA conjugates of the present disclosure or PBS. Further, the serum TG inhibition rates and serum CHO inhibition rates at these time points after administration of the siRNA conjugates of the present disclosure are summarized in Table 10A and Table 10B:

Table 10A. Serum TG inhibition rates of siRNA conjugates in transgenic mice

| Group | Dose mg/kg | TG level inhibition rate (%) | | | | | |
|---|---|---|---|---|---|---|---|
| | | D8 | D15 | D22 | D29 | D36 | D43 |
| Conjugate 3 | 3 | 88.26 | 84.22 | 89.33 | 88.22 | 81.60 | 64.87 |
| | 1 | 80.65 | 75.07 | 70.78 | 56.35 | 41.94 | 44.23 |
| Conjugate 5 | 3 | 88.57 | 86.99 | 87.59 | 88.61 | 80.18 | 60.13 |
| | 1 | 86.09 | 83.67 | 70.63 | 45.51 | 35.31 | 12.17 |

Table 10B. Serum CHO inhibition rates of siRNA conjugates in transgenic mice

| Group | Dose mg/kg | CHO level inhibition rate (%) | | | | | |
|---|---|---|---|---|---|---|---|
| | | D8 | D15 | D22 | D29 | D36 | D43 |
| Conjugate 3 | 3 | 47.92 | 44.15 | 49.14 | 51.02 | 51.18 | 40.19 |
| | 1 | 41.83 | 36.74 | 39.42 | 37.94 | 22.04 | 29.08 |
| Conjugate 5 | 3 | 52.95 | 53.46 | 55.17 | 57.41 | 47.98 | 39.96 |
| | 1 | 45.03 | 44.23 | 38.27 | 29.18 | 29.08 | 10.06 |

According to the results in FIG. 7A, FIG. 7B and Tables 10A and 10B, at different time points after administration, conjugate 3 and conjugate 5 can significantly reduce the levels of TG and CHO in mouse serum. In addition, within the 43 days of the experiment, the inhibition effect was always kept high. Especially, at a dose of 3 mg/kg, both conjugate 3 and conjugate 5 showed excellent inhibition effects on blood lipids in mice, their maximum serum TG inhibition rates were both higher than 88%, and their maximum serum CHO inhibition rates were 51.18% and 57.41%, respectively. The above results show that the siRNA conjugates of the present disclosure can effectively reduce blood lipid levels for a long time, and show excellent development prospects in the preparation of medicaments for treating and/or preventing dyslipidemia-related diseases or symptoms.

Experimental Example 14. Lowering effects of siRNA conjugates on blood lipid in mice *(in vivo)*

Human APOC3 transgenic mice Tg(APOC3)3707Bres (purchased from Jackson Laboratory, USA) with serum TG content > 2 mmol/L were selected and randomly divided into groups of 6, half of which were male and half female. Conjugate 1, conjugate 2, reference conjugate 1 and PBS blank control were administered to each group of mice respectively. All animals were dosed according to their body weight, and administered in a single subcutaneous injection. The dose of each siRNA conjugate (based on the amount of siRNA) was 3 mg/kg and 1 mg/kg mouse body weight, and the administration volume was 5 mL/kg. Each siRNA conjugate was provided in PBS aqueous solution, and the concentration that the conjugate should be prepared was calculated according to the dosage and volume of administration. Each of the mice in the other group was administered 1× PBS with a volume of 5 mL/kg, serving as a blank control group.

The time point of administration was recorded as day 1, and blood was collected from the orbital venous plexus of the mice on days 1, 8, 15 and 22, 100 μL each time. The collected blood was left at room temperature for 30 min, and then centrifuged at 3000 rpm at 4 °C for 15 min to obtain serum. PM1P000/3 automatic serum biochemical analyzer (SABA, Italy) was then used to detect the contents of total cholesterol (CHO) and triglyceride (TG) in serum.

Standardized blood lipid level = (blood lipid content of test group after administration/blood lipid content of test group before administration) × 100%.

Inhibition rate of blood lipid level = (1 - blood lipid content of test group after administration/blood lipid content of test group before administration) × 100%.

Blood lipid refers to total cholesterol (CHO) or triglyceride (TG).

FIGs. 8A and 8B are line graphs showing changes in the level of serum TG or serum CHO over time after administration of a siRNA conjugate of the present disclosure, a reference siRNA conjugate or PBS. Further, the serum TG inhibition rates and serum CHO inhibition rates at these time points after administration of the siRNA conjugates of the present disclosure are summarized in Table 11A and Table 11B:

Table 11A. Serum TG inhibition rates of siRNA conjugates in transgenic mice

| Group | Dose mg/kg | TG level inhibition rate (%) | | |
|---|---|---|---|---|
| | | D9 | D15 | D22 |
| Blank control | / | 17.3 | -3.8 | -3.2 |
| Reference conjugate 1 | 3 | 92.2 | 90.6 | 85.7 |
| | 1 | 84.7 | 80.6 | 80.6 |
| Conjugate 1 | 3 | 92.0 | 92.3 | 90.0 |
| | 1 | 76.1 | 74.6 | 67.6 |
| Conjugate 2 | 3 | 92.9 | 92.3 | 89.9 |
| | 1 | 82.8 | 79.3 | 82.3 |

Table 11B. Serum CHO inhibition rates of siRNA conjugates in transgenic mice

| Group | Dose mg/kg | CHO level inhibition rate (%) | | |
|---|---|---|---|---|
| | | D9 | D15 | D22 |
| Blank control | / | 19.4 | -2.6 | 1.7 |
| Reference conjugate 1 | 3 | 51.6 | 44.3 | 42.7 |
| | 1 | 52.1 | 45.5 | 50.8 |
| Conjugate 1 | 3 | 57.5 | 57.3 | 47.2 |
| | 1 | 49.3 | 43.9 | 42.4 |
| Conjugate 2 | 3 | 54.9 | 50.0 | 55.4 |
| | 1 | 50.9 | 44.0 | 46.7 |

The results in FIG. 8A, FIG. 8B and Tables 11A and 11B show that at different time points after administration, conjugate 1 and conjugate 2 can significantly reduce the levels of TG and CHO in mouse serum, and maintained high inhibition effects throughout the 22 days of the experiment. In addition, the conjugates showed similar blood lipid level lowering effects to the corresponding reference conjugate 1, which contained no stabilizing modified nucleotides.

Especially, at a dose of 3 mg/kg, both conjugate 1 and conjugate 2 showed excellent inhibition effects on blood lipids in mice, their maximum serum TG inhibition rates were both higher than 92%, and their maximum serum CHO inhibition rates were 57.5% and 54.9%, respectively. The above results show that the siRNA conjugates of the present disclosure can effectively reduce blood lipid levels for a long time, and show excellent development prospects in the preparation of medicaments for treating and/or preventing dyslipidemia-related diseases or symptoms.

Experimental Example 15. Determination of double-strand melting temperature Tm

Each of the prepared siRNA1-siRNA7 and reference siRNA1-reference siRNA7 was prepared with 1× PBS buffer into a 0.02 mg/mL solution as a test sample solution, respectively. The test solution was added into a 10 mm path length quartz cuvette on an Agilent cary300 UV spectrophotometer equipped with a thermal program. A temperature-absorbance curve at a wavelength of 260 nm was recorded, wherein the heating rate was 0.5 °C/min, and the temperature was raised from 20.0 °C to 95 °C. The double-strand melting temperature Tm was obtained by calculating the first derivative of the temperature-absorbance curve according to the spectrophotometer manual. The Tm values are shown in Table 12:

Table 12. Double-strand melting temperature Tm

| siRNA | Tm(°C) | Reference siRNA | Tm(°C) | Δ*Tm*(°C) |
|---|---|---|---|---|
| siRNA1 | 80.97 | Reference siRNA1 | 78.06 | 2.91 |
| siRNA2 | 80.02 | Reference siRNA2 | 78.12 | 1.90 |
| siRNA3 | 79.07 | Reference siRNA3 | 76.32 | 2.75 |
| siRNA4 | 80.17 | Reference siRNA4 | 77.98 | 2.19 |
| siRNA5 | 78.22 | Reference siRNA5 | 76.26 | 1.96 |
| siRNA6 | 89.26 | Reference siRNA6 | 87.93 | 1.33 |
| siRNA7 | 90.07 | Reference siRNA7 | 87.98 | 2.09 |

Reference siRNA1-reference siRNA7 are siRNAs having the same base sequence as siRNA1-siRNA7, but are siRNAs without any modified nucleotide at positions with stabilizing modified nucleotides in siRNA1-siRNA7:

$$\Delta \text{ Tm value (siRNA to be tested)} = \text{Tm (siRNA)} - \text{Tm (reference siRNA)}.$$

According to the results in Table 12, compared with the case where an unmodified nucleotide is present at the same position, the double-stranded oligonucleotide of the present disclosure comprising a stabilizing modified nucleotide and the conjugate thereof have a higher double-strand melting temperature. The double-stranded melting temperature increased by at least 1.33 °C, wherein siRNA1 had the most increase, that is 2.91 °C compared with the reference siRNA.

Some embodiments of the present disclosure have been described in detail above, but the present disclosure is not limited to the specific details of the embodiments. Within the scope of the technical concept of the present disclosure, various simple modifications may be made to the technical solutions of the present disclosure. These simple modifications all belong to the protection scope of the present disclosure.

In addition, it should be noted that the various specific technical features described in some embodiments above can be combined in any suitable manner where the features do not contradict each other. In order to avoid unnecessary repetition, such combinations will not be illustrated separately.

In addition, various implementations of the present disclosure may be combined arbitrarily, as long as they do not violate the idea of the present disclosure, and they should also be regarded as the content disclosed in the present disclosure.

## Claims

1. An siRNA, wherein the siRNA comprises a sense strand and an antisense strand; the sense strand comprises a nucleotide sequence I, and the antisense strand comprises a nucleotide sequence II; both the nucleotide sequence I and the nucleotide sequence II consist of 19 nucleotides, and each of the nucleotides in the nucleotide sequence I and the nucleotide sequence II is a modified or unmodified nucleotide; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence with 19 nucleotides in length in an mRNA expressed by an apolipoprotein C3 gene; in the direction from the 5' end to the 3' end, at least one of the 3rd-6th nucleotides in the nucleotide sequence II is a stabilizing modified nucleotide; the stabilizing modified nucleotide refers to a nucleotide whose ribose hydroxyl group at the 2' position is substituted with a stabilizing modification group; compared with an siRNA whose nucleotides at the corresponding positions are unmodified nucleotides, the siRNA comprising the stabilizing modified nucleotide has increased thermostability, and the steric hindrance of the stabilizing modification group is greater than that of 2'-O-methyl.

2. The siRNA according to claim 1, wherein in the direction from the 5' end to the 3' end, the 3rd or 5th nucleotide in the nucleotide sequence II is the stabilizing modified nucleotide.

3. The siRNA according to claim 1 or 2, wherein in the direction from the 5' end to the 3' end, no more than 2 nucleotides of the 3rd-9th nucleotides in the nucleotide sequence II are the stabilizing modified nucleotides.

4. The siRNA according to any one of claims 1-3, wherein the increase in the thermostability of the siRNA refers to an increase of the Tm of the siRNA, and the Tm is the double-strand melting temperature of the siRNA.

5. The siRNA according to claim 4, wherein the increase in the thermostability of the siRNA refers to an increase of at least 0.05 °C of the Tm of the siRNA.

6. The siRNA according to claim 4, wherein the increase in the thermostability of the siRNA refers to an increase of 0.1-6 °C of the Tm of the siRNA.

7. The siRNA according to claim 4, wherein the increase in the thermostability of the siRNA refers to an increase of 0.5-4 °C of the Tm of the siRNA.

8. The siRNA according to any one of claims 1-7, wherein each of the stabilizing modification groups independently has a structure represented by -X-R, wherein X is O, NR', S or $SiR'_2$; R is one of $C_2$-$C_6$ alkyl, substituted $C_2$-$C_6$ alkyl, $C_6$-$C_8$ aryl and substituted $C_6$-$C_8$ aryl; each R' is independently one of H, $C_1$-$C_6$ alkyl, substituted $C_1$-$C_6$ alkyl, $C_6$-$C_8$ aryl and substituted $C_6$-$C_8$ aryl; the substituted $C_2$-$C_6$ alkyl, substituted $C_6$-$C_8$ aryl or substituted $C_1$-$C_6$ alkyl refers to a group formed by replacing one or more hydrogen atoms on $C_2$-$C_6$ alkyl, $C_6$-$C_8$ aryl or $C_1$-$C_6$ alkyl by a substituent, and the substituent is selected from one or more of the following substituents: $C_1$-$C_3$ alkyl, $C_6$-$C_8$ aryl, $C_1$-$C_3$ alkoxy, halogen, oxo and sulfanylidene.

9. The siRNA according to claim 8, wherein each of the stabilizing modification groups is independently selected from one of 2'-O-methoxyethyl, 2'-O-allyl, 2'-C-allyl, 2'-O-2-N-methylamino-2-oxoethyl, 2'-O-2-N,N-dimethylaminoethyl, 2'-O-3-aminopropyl and 2'-O-2,4-dinitrophenyl.

10. The siRNA according to claim 9, wherein each of the stabilizing modification groups is 2'-O-methoxyethyl.

11. The siRNA according to any one of claims 1-10, wherein the nucleotide sequence I and the nucleotide sequence set forth in SEQ ID NO: 1 are equal in length and differ by no more than 3 nucleotides; the nucleotide sequence II and the nucleotide sequence set forth in SEQ ID NO: 2 are equal in length and differ by no more than 3 nucleotides:

5'- CAAUAAAGCUGGACAAGA$Z_1$ -3' (SEQ ID NO: 1);
5'- $Z_2$UCUUGUCCAGCUUUAUUG -3' (SEQ ID NO:2),
wherein $Z_1$ is A, $Z_2$ is U, the nucleotide sequence I contains nucleotide $Z_3$ corresponding to $Z_1$ in position, the nucleotide sequence II contains nucleotide $Z_4$ corresponding to $Z_2$ in position, and $Z_4$ is the first nucleotide at the 5' end of the antisense strand;
or the nucleotide sequence I and the nucleotide sequence set forth in SEQ ID NO: 45 are equal in length and differ by no more than 3 nucleotides; the nucleotide sequence II and the nucleotide sequence set forth in SEQ ID NO: 46 are equal in length and differ by no more than 3 nucleotides:

5'- UUAAAAGGGACAGUAUUC$Z_5$ -3' (SEQ ID NO:45);
5'- $Z_6$GAAUACUGUCCCUUUUAA -3' (SEQ ID NO:46),

wherein $Z_5$ is U, $Z_6$ is A, the nucleotide sequence I contains nucleotide $Z_7$ corresponding to $Z_5$ in position, the nucleotide sequence II contains nucleotide $Z_8$ corresponding to $Z_6$ in position, and $Z_8$ is the first nucleotide at the 5' end of the antisense strand;
or the nucleotide sequence I and the nucleotide sequence set forth in SEQ ID NO: 105 are equal in length and differ by no more than 3 nucleotides; the nucleotide sequence II and the nucleotide sequence set forth in SEQ ID NO: 106 are equal in length and differ by no more than 3 nucleotides:

5'- GGACAGUAUUCUCAGUGC$Z_9$ -3'(SEQ ID NO: 105);
5'- $Z_{10}$GCACUGAGAAUACUGUCC -3'(SEQ ID NO: 106),

wherein $Z_9$ is U, $Z_{10}$ is A, the nucleotide sequence I contains nucleotide $Z_{11}$ corresponding to $Z_9$ in position, the nucleotide sequence II contains nucleotide $Z_{12}$ corresponding to $Z_{10}$ in position, and $Z_8$ is the first nucleotide at the 5' end of the antisense strand.

12. The siRNA according to claim 11, wherein the first nucleotide sequence segment is the nucleotide sequence set forth in SEQ ID NO: 1; or the first nucleotide sequence segment is the nucleotide sequence set forth in SEQ ID NO:

45; or the first nucleotide sequence segment is the nucleotide sequence set forth in SEQ ID NO: 105.

**13.** The siRNA according to claim 11 or 12, wherein the nucleotide sequence I and the nucleotide sequence set forth in SEQ ID NO: 1 differ by no more than 1 nucleotide, and/or the nucleotide sequence II and the nucleotide sequence set forth in SEQ ID NO: 2 differ by no more than 1 nucleotide;

or the nucleotide sequence I and the nucleotide sequence set forth in SEQ ID NO: 45 differ by no more than 1 nucleotide, and/or the nucleotide sequence II and the nucleotide sequence set forth in SEQ ID NO: 46 differ by no more than 1 nucleotide;
or the nucleotide sequence I and the nucleotide sequence set forth in SEQ ID NO: 105 differ by no more than 1 nucleotide, and/or the nucleotide sequence II and the nucleotide sequence set forth in SEQ ID NO: 106 differ by no more than 1 nucleotide.

**14.** The siRNA according to claim 13, wherein the nucleotide difference between the nucleotide sequence II and the nucleotide sequence set forth in SEQ ID NO: 2 comprises the difference at $Z_4$ position, and $Z_4$ is selected from A, G or C;

or the nucleotide difference between the nucleotide sequence II and the nucleotide sequence set forth in SEQ ID NO: 46 comprises the difference at $Z_8$ position, and $Z_8$ is selected from G, C or U;
or the nucleotide difference between the nucleotide sequence II and the nucleotide sequence set forth in SEQ ID NO: 106 comprises the difference at $Z_{12}$ position, and $Z_{12}$ is selected from G, C or U.

**15.** The siRNA according to any one of claims 11-14, wherein $Z_3$ is a nucleotide complementary to $Z_4$; or $Z_5$ is a nucleotide complementary to $Z_6$; or $Z_8$ is a nucleotide complementary to $Z_7$.

**16.** The siRNA according to any one of claims 1-15, wherein the nucleotide sequence II is substantially reversely complementary, virtually reversely complementary or completely reversely complementary to the first nucleotide sequence segment; the substantially reversely complementary means that there are no more than 3 base mismatches between the two nucleotide sequences; the virtually reversely complementary means that there are no more than 1 base mismatch between the two nucleotide sequences; the completely reversely complementary means that there is no mismatch between the two nucleotide sequences.

**17.** The siRNA according to claim 16, wherein in the direction from the 5' end to the 3' end, the nucleotides at the 2nd-19th positions of the nucleotide sequence II are completely reversely complementary to the nucleotides at the 1st-18th positions of the first nucleotide sequence segment.

**18.** The siRNA according to claim 17, wherein the nucleotide sequence II is completely reversely complementary to the nucleotide sequence I, or there is a base mismatch between the second nucleotide in the nucleotide sequence II in the direction from the 5' end to the 3' end and the second nucleotide in the nucleotide sequence I in the direction from the 3' end to the 5' end.

**19.** The siRNA according to any one of claims 1-18, wherein the sense strand and the antisense strand have the same or different lengths; the length of the sense strand is 19-23 nucleotides; the length of the antisense strand is 19-26 nucleotides; the nucleotide sequence I is the nucleotide sequence set forth in SEQ ID NO: 3; the nucleotide sequence II is the nucleotide sequence set forth in SEQ ID NO: 4:

5'- CAAUAAAGCUGGACAAGA$Z_3$ -3'(SEQ ID NO:3);
5'- $Z_4$UCUUGUCCAGCUUUAUUG -3'(SEQ ID NO:4),
wherein $Z_3$ is selected from A, U, G or C, and $Z_4$ is a nucleotide complementary to $Z_3$;
or the nucleotide sequence I is a nucleotide sequence set forth in SEQ ID NO: 47, and the nucleotide sequence II is a nucleotide sequence set forth in SEQ ID NO: 48:

5'- UUAAAAGGGACAGUAUUC$Z_7$ -3'(SEQ ID NO:47);
5'- ZsGAAUACUGUCCCUUUUAA -3'(SEQ ID NO:48),

wherein $Z_7$ is selected from A, U, G or C, and $Z_8$ is a nucleotide complementary to $Z_7$;
or the nucleotide sequence I is a nucleotide sequence set forth in SEQ ID NO: 107, and the nucleotide sequence II is a nucleotide sequence set forth in SEQ ID NO: 108:

5'- GGACAGUAUUCUCAGUGC$Z_{11}$ -3'(SEQ ID NO: 107);
5'- $Z_{12}$GCACUGAGAAUACUGUCC -3'(SEQ ID NO: 108),

wherein $Z_{11}$ is selected from A, U, G or C, and $Z_{12}$ is a nucleotide complementary to $Z_{11}$.

**20.** The siRNA according to claim 19, wherein $Z_3$ is A and $Z_4$ is U; or $Z_7$ is U and $Z_8$ is A; or $Z_{11}$ is U and $Z_{12}$ is A.

**21.** The siRNA according to any one of claims 1-20, wherein in the direction from the 5' end to the 3' end, if the 2nd, 6th, 14th, and 16th nucleotides in the nucleotide sequence II are not the stabilizing modified nucleotides, the same are 2'-fluoro-modified nucleotides.

**22.** The siRNA according to claim 21, wherein all nucleotides in the nucleotide sequence II are modified nucleotides; in the direction from the 5' end to the 3' end, all the nucleotides in the nucleotide sequence II are modified nucleotides; in the direction from the 5' end to the 3' end, if the 2nd, 6th, 14th, and 16th nucleotides in the nucleotide sequence II are not the stabilizing modified nucleotides, the same are 2'-fluoro-modified nucleotides, and the other nucleotides in the nucleotide sequence II are each independently one of non-fluoro-modified nucleotides.

**23.** The siRNA according to any one of claims 1-22, wherein in the direction from the 5' end to the 3' end, the 7th-9th nucleotides in the nucleotide sequence I are 2'-fluoro-modified nucleotides.

**24.** The siRNA according to claim 23, wherein all nucleotides in the nucleotide sequence I are modified nucleotides; in the direction from the 5' end to the 3' end, the 7th-9th nucleotides in the nucleotide sequence I are 2'-fluoro-modified nucleotides.

**25.** The siRNA according to claim 24, wherein all nucleotides in the nucleotide sequence I are modified nucleotides; in the direction from the 5' end to the 3' end, the 7th-9th nucleotides in the nucleotide sequence I are 2'-fluoro-modified nucleotides, and the other nucleotides in the nucleotide sequence I are each independently one of non-fluoro-modified nucleotides.

**26.** The siRNA according to any one of claims 1-25, wherein the sense strand also contains a nucleotide sequence III, and the antisense strand also contains a nucleotide sequence IV; each nucleotide in the nucleotide sequence III and the nucleotide sequence IV is independently one of non-fluoro-modified nucleotides and is not the stabilizing modified nucleotide; the length of the nucleotide sequence III is 1, 2, 3 or 4 nucleotides; the nucleotide sequence IV and the nucleotide sequence III are equal in length, and the nucleotide sequence IV and the nucleotide sequence III are virtually reversely complementary or completely reversely complementary; the nucleotide sequence III is linked to the 5' end of the nucleotide sequence I, and the nucleotide sequence IV is linked to the 3' end of the nucleotide sequence II; the nucleotide sequence IV is virtually reversely complementary or completely reversely complementary to the second nucleotide sequence segment, and the second nucleotide sequence segment refers to a nucleotide sequence adjacent to the first nucleotide sequence segment and having the same length as the nucleotide sequence IV in the mRNA expressed by an APOC3 gene.

**27.** The siRNA according to claim 26, wherein the nucleotide sequence I and the nucleotide sequence set forth in SEQ ID NO: 1 are equal in length and differ by no more than 3 nucleotides, and both the nucleotide sequences III and IV are 1 nucleotide in length, the base of the nucleotide sequence III is C, and the base of the nucleotide sequence IV is G; or both the nucleotide sequences III and IV are 2 nucleotides in length, the base composition of the nucleotide sequence III is CC, and the base composition of the nucleotide sequence IV is GG; or both the nucleotide sequences III and IV are 3 nucleotides in length, the base composition of the nucleotide sequence III is UCC, and the base composition of the nucleotide sequence IV is GGA; or both the nucleotide sequences III and IV are 4 nucleotides in length, the base composition of the nucleotide sequence III is CUCC, and the base composition of the nucleotide sequence IV is GGAG.

**28.** The siRNA according to claim 27, wherein the length of the second nucleotide sequence segment is 1, 2, 3 or 4 nucleotides, and the base composition is C, CC, UCC or CUCC, respectively.

**29.** The siRNA according to claim 26, wherein the nucleotide sequence I and the nucleotide sequence set forth in SEQ ID NO: 45 are equal in length and differ by no more than 3 nucleotides, and both the nucleotide sequences III and IV are 1 nucleotide in length, the base of the nucleotide sequence III is C, and the base of the nucleotide sequence IV is G; or both the nucleotide sequences III and IV are 2 nucleotides in length, the base composition of the nucleotide

sequence III is GC, and the base composition of the nucleotide sequence IV is GC; or both the nucleotide sequences III and IV are 3 nucleotides in length, the base composition of the nucleotide sequence III is UGC, and the base composition of the nucleotide sequence IV is GCA; or both the nucleotide sequences III and IV are 4 nucleotides in length, the base composition of the nucleotide sequence III is UUGC, and the base composition of the nucleotide sequence IV is GCAA.

**30.** The siRNA according to claim 29, wherein the length of the second nucleotide sequence segment is 1, 2, 3 or 4 nucleotides, and the base composition is C, GC, GCA or GCAA, respectively.

**31.** The siRNA according to claim 26, wherein the nucleotide sequence I and the nucleotide sequence set forth in SEQ ID NO: 105 are equal in length and differ by no more than 3 nucleotides, and both the nucleotide sequences III and IV are 1 nucleotide in length, the base of the nucleotide sequence III is G, and the base of the nucleotide sequence IV is C; or both the nucleotide sequences III and IV are 2 nucleotides in length, the base composition of the nucleotide sequence III is AG and the base composition of the nucleotide sequence IV is CU; or both the nucleotide sequences III and IV are 3 nucleotides in length, the base composition of the nucleotide sequence III is AAG, and the base composition of the nucleotide sequence IV is CUU; or both the nucleotide sequences III and IV are 4 nucleotides in length, the base composition of the nucleotide sequence III is AAAG, and the base composition of the nucleotide sequence IV is CUUU.

**32.** The siRNA according to claim 31, wherein the length of the second nucleotide sequence segment is 1, 2, 3 or 4 nucleotides, and the base composition is G, AG, AAG or AAAG, respectively.

**33.** The siRNA according to any one of claims 1-32, wherein the siRNA also contains an oligonucleotide sequence V, each nucleotide in the oligonucleotide sequence V is independently one of the non-fluoro-modified nucleotides and is not the stabilizing modified nucleotide, the length of the nucleotide sequence V is 1 to 3 nucleotides, and the nucleotide sequence V is linked to the 3' end of the antisense strand, thereby constituting the 3' overhang of the antisense strand.

**34.** The siRNA according to claim 33, wherein the length of the nucleotide sequence V is 2 nucleotides, and in the direction from the 5' end to the 3' end, the nucleotide sequence V is 2 consecutive thymine deoxyribonucleotides, 2 consecutive uracil ribonucleotides, or is completely reversely complementary to a third nucleotide sequence segment; the third nucleotide sequence segment refers to a nucleotide sequence adjacent to the first nucleotide sequence segment or the second nucleotide sequence segment and having the same length as the nucleotide sequence V in the mRNA expressed by an APOC3 gene.

**35.** The siRNA according to claim 34, wherein the first nucleotide sequence segment has the nucleotide sequence set forth in SEQ ID NO: 1, and the base composition of the third nucleotide sequence segment is CC; or the first nucleotide sequence segment has the nucleotide sequence set forth in SEQ ID NO: 45, and the base composition of the third nucleotide sequence segment is GC; or the first nucleotide sequence segment has the nucleotide sequence set forth in SEQ ID NO: 105, and the base composition of the third nucleotide sequence segment is AG.

**36.** The siRNA according to any one of claims 1-35, wherein, the sense strand of the siRNA contains the nucleotide sequence set forth in SEQ ID NO: 5, and the antisense strand contains the nucleotide sequence set forth in SEQ ID NO: 6:

5'- CAAUAAAGCUGGACAAGA$Z_3$ -3'(SEQ ID NO:5);
5'- $Z_4$UCUUGUCCAGCUUUAUUGGG -3'(SEQ ID NO:6),
wherein, $Z_4$ is the first nucleotide at the 5' end of the antisense strand, $Z_3$ is selected from A, U, G or C, and $Z_4$ is a nucleotide complementary to $Z_3$;
or the sense strand of the siRNA contains the nucleotide sequence set forth in SEQ ID NO: 7, and the antisense strand of the siRNA contains the nucleotide sequence set forth in SEQ ID NO: 8:

5'- CCCAAUAAAGCUGGACAAGA$Z_3$ -3'(SEQ ID NO:7);
5'-$Z_4$UCUUGUCCAGCUUUAUUGGGAG-3' (SEQ ID NO: 8), wherein, $Z_4$ is the first nucleotide at the 5' end of the antisense strand, $Z_3$ is selected from A, U, G or C, and $Z_4$ is a nucleotide complementary to $Z_3$;

or the sense strand of the siRNA contains the nucleotide sequence set forth in SEQ ID NO: 49, and the antisense strand contains the nucleotide sequence set forth in SEQ ID NO: 50:

5'- UUAAAAGGGACAGUAUUC$Z_7$ -3'(SEQ ID NO:49);
5'- ZsGAAUACUGUCCCUUUUAAGC -3'(SEQ ID NO:50),

wherein, $Z_8$ is the first nucleotide at the 5' end of the antisense strand, $Z_7$ is selected from A, U, G or C, and $Z_8$ is a nucleotide complementary to $Z_7$;
or the sense strand of the siRNA contains the nucleotide sequence set forth in SEQ ID NO: 51, and the antisense strand contains the nucleotide sequence set forth in SEQ ID NO: 52:

5'- GCUUAAAAGGGACAGUAUUC$Z_7$ -3'(SEQ ID NO:51);
5'- $Z_8$GAAUACUGUCCCUUUUAAGCAA -3'(SEQ ID NO:52),

wherein, $Z_8$ is the first nucleotide at the 5' end of the antisense strand, $Z_7$ is selected from A, U, G or C, and $Z_8$ is a nucleotide complementary to $Z_7$;
or the sense strand of the siRNA contains the nucleotide sequence set forth in SEQ ID NO: 49, and the antisense strand contains the nucleotide sequence set forth in SEQ ID NO: 149:

5'- UUAAAAGGGACAGUAUUC$Z_7$ -3'(SEQ ID NO:49);
5'- ZsGAAUACUGUCCCUUUUAAUU -3'(SEQ ID NO: 149),

wherein, $Z_8$ is the first nucleotide at the 5' end of the antisense strand, $Z_7$ is selected from A, U, G or C, and $Z_8$ is a nucleotide complementary to $Z_7$;
or the sense strand of the siRNA contains the nucleotide sequence set forth in SEQ ID NO: 51, and the antisense strand contains the nucleotide sequence set forth in SEQ ID NO: 150:

5'- GCUUAAAAGGGACAGUAUUC$Z_7$ -3'(SEQ ID NO:51);
5'- ZsGAAUACUGUCCCUUUUAAGCUU -3'(SEQ ID NO: 150),

wherein, $Z_8$ is the first nucleotide at the 5' end of the antisense strand, $Z_7$ is selected from A, U, G or C, and $Z_8$ is a nucleotide complementary to $Z_7$;
or the sense strand of the siRNA contains the nucleotide sequence set forth in SEQ ID NO: 109, and the antisense strand contains the nucleotide sequence set forth in SEQ ID NO: 110:

5'- GGACAGUAUUCUCAGUGC$Z_{11}$ -3'(SEQ ID NO: 109);
5'- $Z_{12}$GCACUGAGAAUACUGUCCCU -3'(SEQ ID NO: 110),

wherein, $Z_{12}$ is the first nucleotide at the 5' end of the antisense strand, $Z_{11}$ is selected from A, U, G or C, and $Z_{12}$ is a nucleotide complementary to $Z_{11}$;
or the sense strand of the siRNA contains the nucleotide sequence set forth in SEQ ID NO: 111, and the antisense strand contains the nucleotide sequence set forth in SEQ ID NO: 112:

5'- AGGGACAGUAUUCUCAGUGC$Z_{11}$ -3'(SEQ ID NO: 111);
5'- $Z_{12}$GCACUGAGAAUACUGUCCCUUU -3'(SEQ ID NO: 112),

wherein $Z_{12}$ is the first nucleotide at the 5' end of the antisense strand, $Z_{11}$ is selected from A, U, G or C, and $Z_{12}$ is a nucleotide complementary to $Z_{11}$.

**37.** The siRNA according to any one of claims 1-36, wherein each of the non-fluoro-modified nucleotides is independently selected from one of a nucleotide or nucleotide analog formed by replacing the hydroxyl at the 2' position of a ribosyl group of the nucleotide with a non-fluorine group.

**38.** The siRNA according to any one of claims 1-37, wherein each of the non-fluoro-modified nucleotides is a methoxy-modified nucleotide, and the methoxy-modified nucleotide refers to a nucleotide formed by replacing the 2'-hydroxyl of a ribosyl group with methoxy.

**39.** The siRNA according to any one of claims 1-38, wherein the siRNA is one of siAPOC3a1-M1, siAPOC3a1-M2, siAPOC3a2-M1, siAPOC3a2-M2, siAPOC3b1-M1, siAPOC3b1-M2, siAPOC3b2-M1, siAPOC3b2-M2, siAPOC3b3-M1, siAPOC3b3-M2, siAPOC3b4-M1, siAPOC3b4-M2, siAPOC3c1-M1, siAPOC3c1-M2, siAPOC3c2-M1, and siAPOC3c2-M2.

**40.** The siRNA according to any one of claims 1-38, wherein at least one phosphate group in a phosphate-sugar backbone of at least one single strand of the sense strand and the antisense strand is a phosphate group having a modified group, and the phosphate group having a modified group exists in at least one in the group consisting of the following positions:

between the 1st nucleotide and the 2nd nucleotide at the 5' end of the sense strand;
between the 2nd nucleotide and the 3rd nucleotide at the 5' end of the sense strand;
between the 1st nucleotide and the 2nd nucleotide at the 3' end of the sense strand;
between the 2nd nucleotide and the 3rd nucleotide at the 3' end of the sense strand;
between the 1st nucleotide and the 2nd nucleotide at the 5' end of the antisense strand;
between the 2nd nucleotide and the 3rd nucleotide at the 5' end of the antisense strand;
between the 1st nucleotide and the 2nd nucleotide at the 3' end of the antisense strand; and
between the 2nd nucleotide and the 3rd nucleotide at the 3' end of the antisense strand.

**41.** The siRNA according to claims 40, wherein the siRNA is one of siAPOC3a1-M1S, siAPOC3a1-M2S, siAPOC3a2-M1S, siAPOC3a2-M2S, siAPOC3b1-M1S, siAPOC3b1-M2S, siAPOC3b2-M1S, siAPOC3b2-M2S, siAPOC3b3-M1S, siAPOC3b3-M2S, siAPOC3b4-M1S, siAPOC3b4-M2S, siAPOC3c1-M1S, siAPOC3c1-M2S, siAPOC3c2-M1S, and siAPOC3c2-M2S.

**42.** The siRNA according to any one of claims 1-38 and 40, wherein the 5' end nucleotide of the antisense strand is a 5'-phosphate nucleotide or a nucleotide modified by a 5'-phosphate analog.

**43.** The siRNA according to claim 42, wherein the siRNA is one of siAPOC3a1-M1P1, siAPOC3a1-M2P1, siAPOC3a2-M1P1, siAPOC3a2-M2P1, siAPOC3a1-M1SP1, siAPOC3a1-M2SP1, siAPOC3a2-M1SP1, siAPOC3a2-M2SP1, siAPOC3b1-M1P1, siAPOC3b1-M2P1, siAPOC3b2-M1P1, siAPOC3b2-M2P1, siAPOC3b1-M1SP1, siAPOC3b1-M2SP1, siAPOC3b2-M1SP1, siAPOC3b2-M2SP1, siAPOC3b3-M1P1, siAPOC3b3-M2P1, siAPOC3b4-M1P1, siAPOC3b4-M2P1, siAPOC3b3-M1SP1, siAPOC3b3-M2SP1, siAPOC3b4-M1SP1, siAPOC3b4-M2SP1, siAPOC3c1-M1P1, siAPOC3c1-M2P1, siAPOC3c2-M1P1, siAPOC3c2-M2P1, siAPOC3c1-M1SP1, siAPOC3c1-M2SP1, siAPOC3c2-M1SP1, and siAPOC3c2-M2SP1.

**44.** A pharmaceutical composition, comprising the siRNA according to any one of claims 1-43 and a pharmaceutically acceptable carrier.

**45.** An siRNA conjugate, comprising the siRNA according to any one of claims 1-43 and a conjugated group conjugated to the siRNA, wherein the conjugated group comprises a linker and a pharmaceutically acceptable targeting group, and the siRNA, the linker and the targeting group are sequentially linked covalently or non-covalently; each of the targeting groups is selected from ligands capable of binding to a cell surface receptor.

**46.** Use of the siRNA according to any one of claims 1 to 43 and/or the pharmaceutical composition according to claim 44 and/or the siRNA conjugate according to claim 45 in the preparation of a medicament for treating and/or preventing diseases or symptoms associated with the level of an mRNA expressed by an APOC3 gene.

**47.** The use according to claim 46, wherein the disease or symptom associated with the level of the mRNA expressed by the APOC3 gene is dyslipidemia.

**48.** A method for treating and/or preventing diseases or symptoms associated with the level of the mRNA expressed by the APOC3 gene, the method comprising: administering to a subject in need thereof the siRNA according to any one of claims 1-43 and/or the pharmaceutical composition according to claim 44 and/or the siRNA conjugate according to claim 45.

**49.** The method according to claim 48, wherein the disease or symptom associated with the level of the mRNA expressed by the APOC3 gene is dyslipidemia.

**50.** A method for inhibiting the expression level of the APOC3 gene in a cell, the method comprising: contacting an effective dose of the siRNA according to any one of claims 1-43 and/or the pharmaceutical composition according to claim 44 and/or the siRNA conjugate according to claim 45 with the cell.

**51.** A kit, comprising the siRNA according to any one of claims 1-43 and/or the pharmaceutical composition according

to claim 44 and/or the siRNA conjugate according to claim 45.

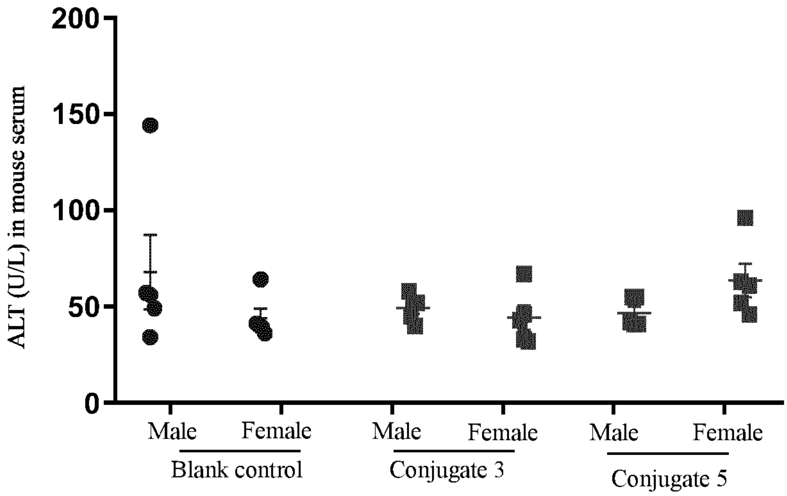
ALT (U/L) in mouse serum
200
150
100
50
0
Male
Female
Blank control
Male
Female
Conjugate 3
Male
Female
Conjugate 5

FIG. 1A

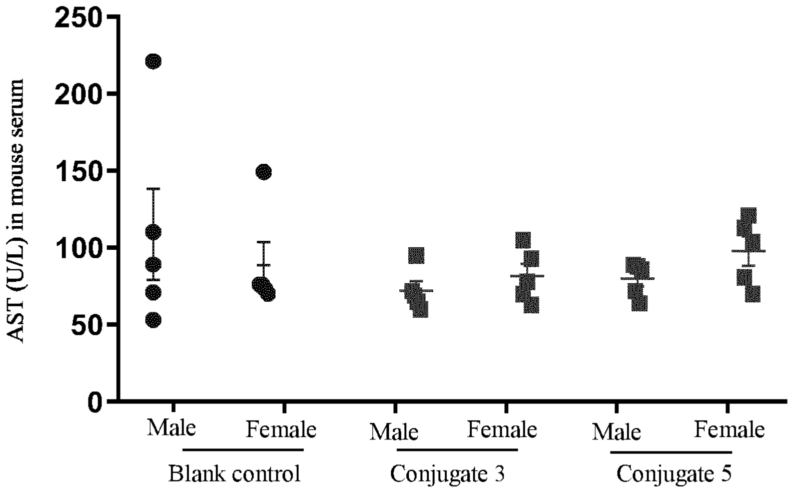
250
200
150
100
50
0
AST (U/L) in mouse serum
Male
Female
Male
Female
Male
Female
Blank control
Conjugate 3
Conjugate 5

FIG. 1B

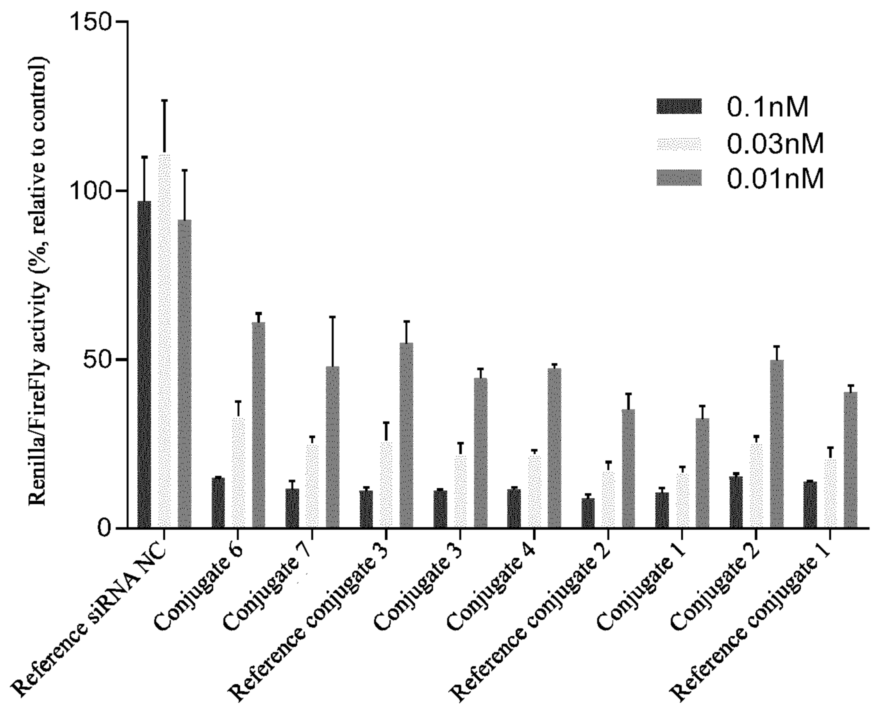
150
100
50
0
Renilla/FireFly activity (%, relative to control)
0.1nM
0.03nM
0.01nM
Reference siRNA NC
Conjugate 6
Conjugate 7
Reference conjugate 3
Conjugate 3
Conjugate 4
Reference conjugate 2
Conjugate 1
Conjugate 2
Reference conjugate 1

FIG. 2

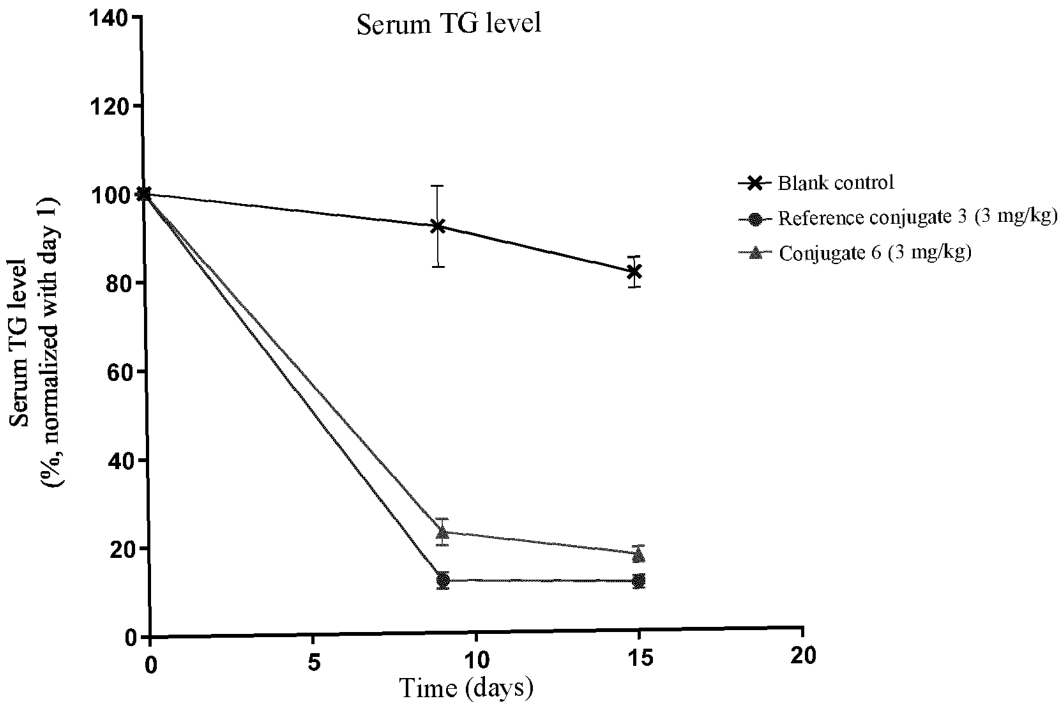
Serum TG level
Serum TG level
(%, normalized with day 1)
140
120
100
80
60
40
20
0
0
5
10
15
20
Time (days)
Blank control
Reference conjugate 3 (3 mg/kg)
Conjugate 6 (3 mg/kg)

FIG. 3A

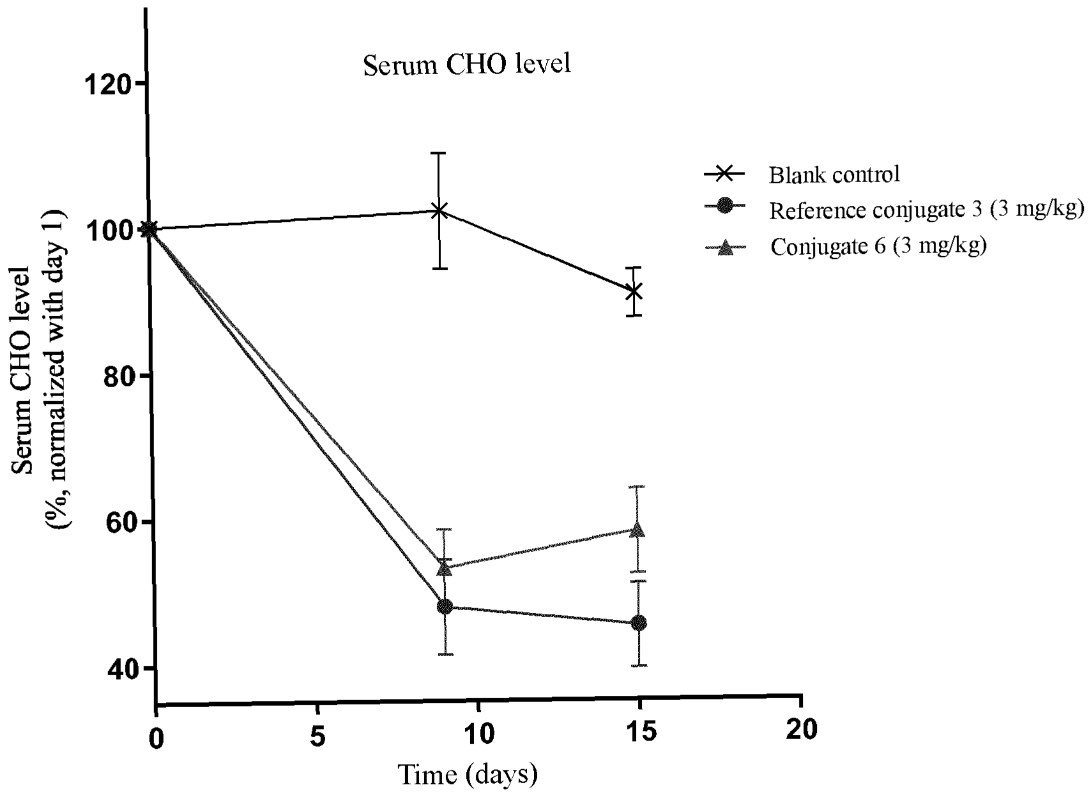
Serum CHO level
Serum CHO level
(%, normalized with day 1)
120
100
80
60
40
0
5
10
15
20
Time (days)
Blank control
Reference conjugate 3 (3 mg/kg)
Conjugate 6 (3 mg/kg)

FIG. 3B

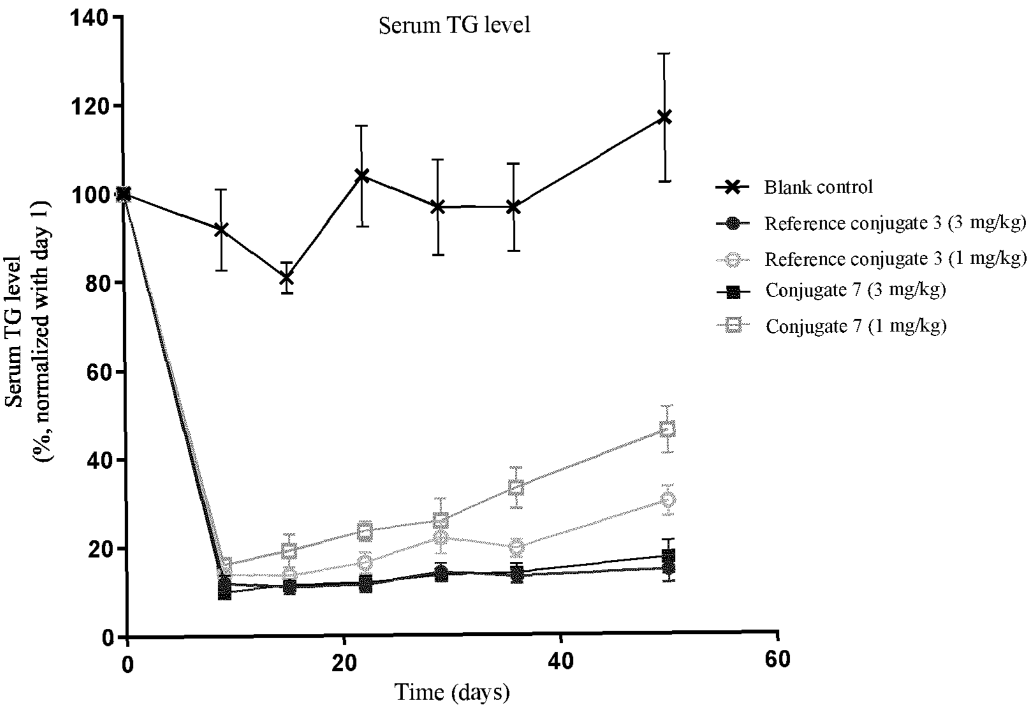
Serum TG level
140
120
100
80
60
40
20
0
Serum TG level
(%, normalized with day 1)
0
20
40
60
Time (days)
Blank control
Reference conjugate 3 (3 mg/kg)
Reference conjugate 3 (1 mg/kg)
Conjugate 7 (3 mg/kg)
Conjugate 7 (1 mg/kg)

FIG. 4A

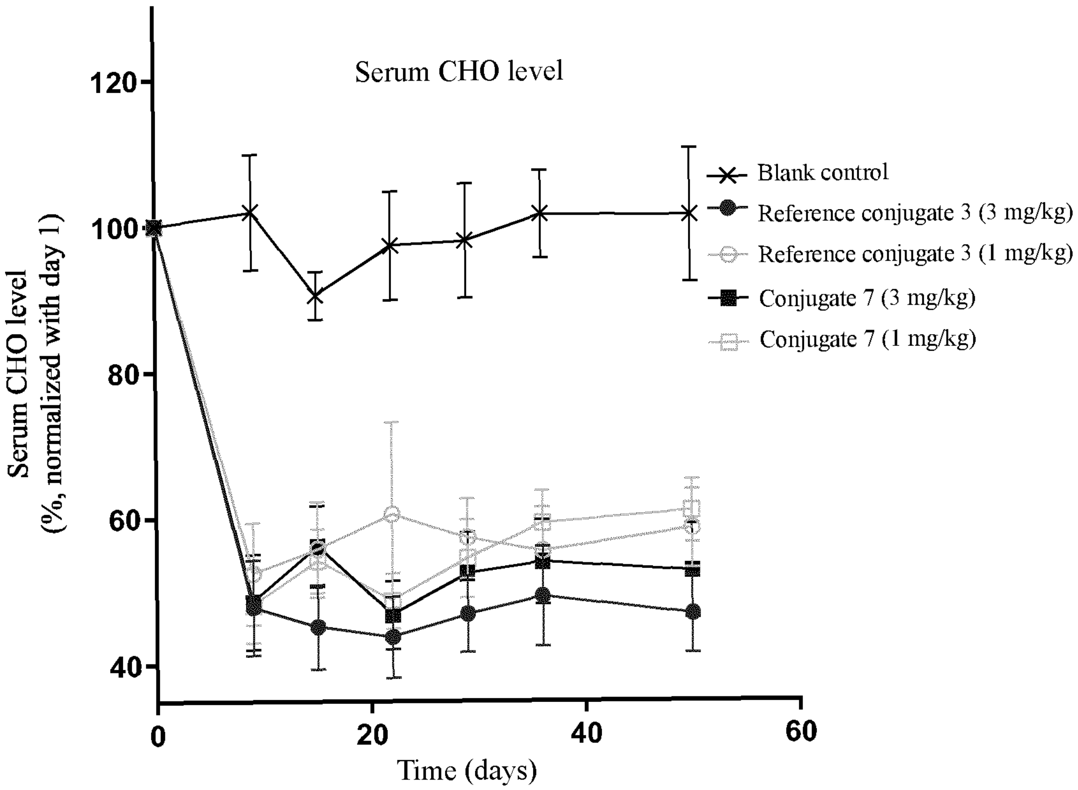
Serum CHO level
120
100
80
60
40
Serum CHO level
(%, normalized with day 1)
0
20
40
60
Time (days)
Blank control
Reference conjugate 3 (3 mg/kg)
Reference conjugate 3 (1 mg/kg)
Conjugate 7 (3 mg/kg)
Conjugate 7 (1 mg/kg)

FIG. 4B

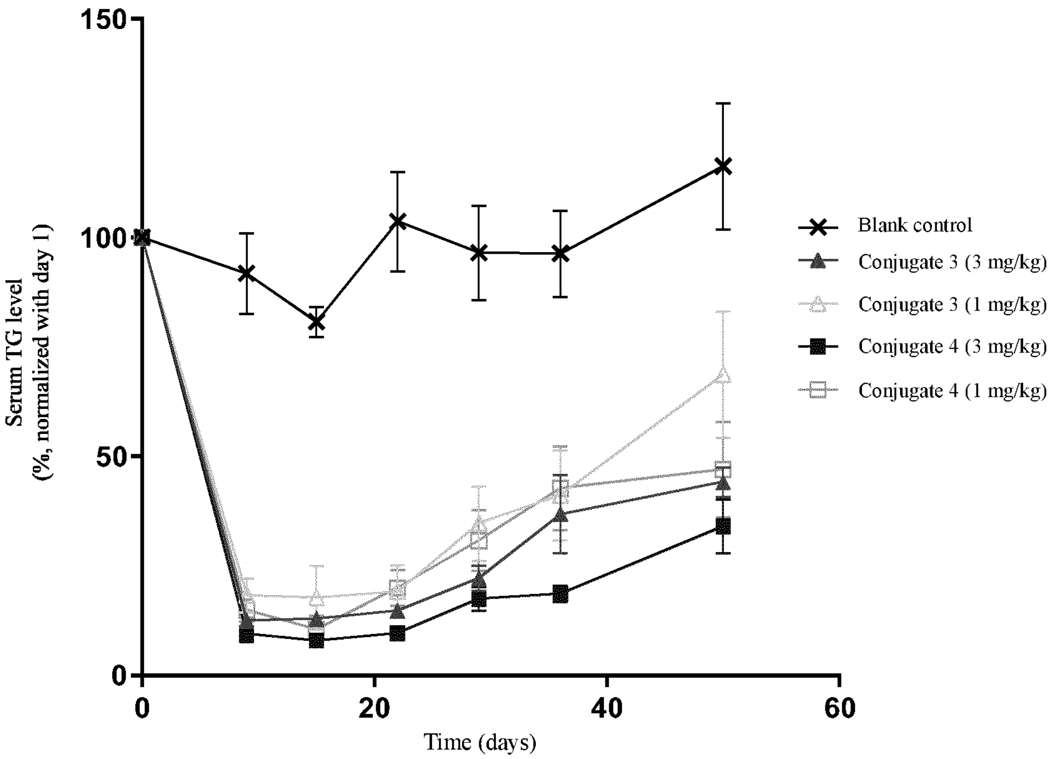
150
100
50
0
Serum TG level
(%, normalized with day 1)
0
20
40
60
Time (days)
Blank control
Conjugate 3 (3 mg/kg)
Conjugate 3 (1 mg/kg)
Conjugate 4 (3 mg/kg)
Conjugate 4 (1 mg/kg)

FIG. 5A

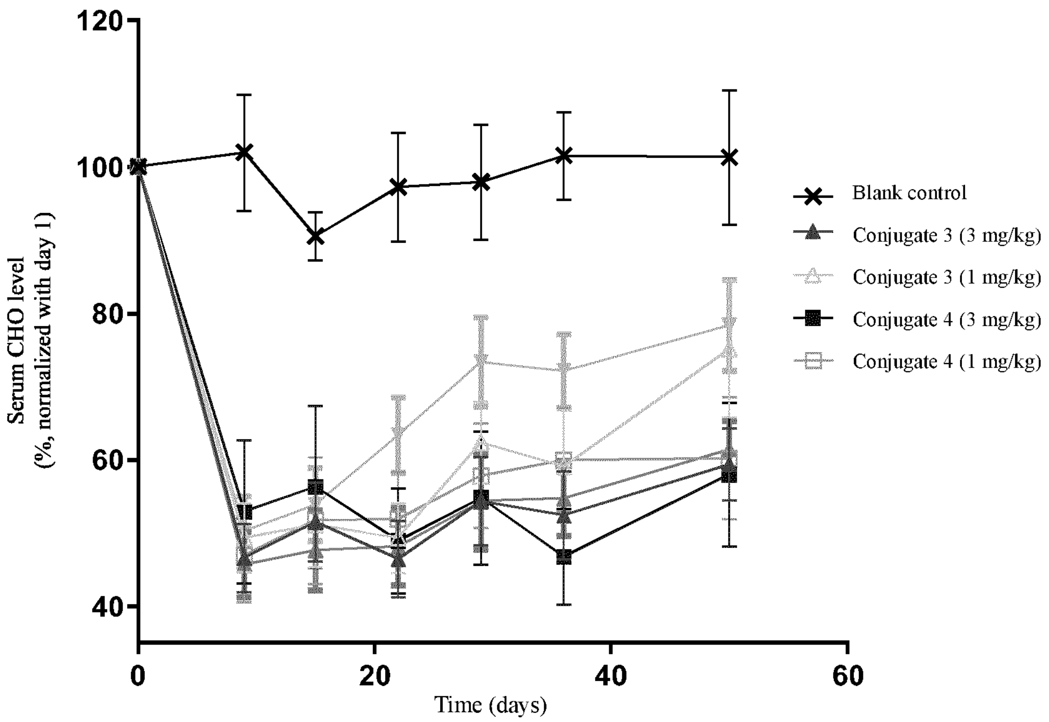
120
100
80
60
40
Serum CHO level
(%, normalized with day 1)
0
20
40
60
Time (days)
Blank control
Conjugate 3 (3 mg/kg)
Conjugate 3 (1 mg/kg)
Conjugate 4 (3 mg/kg)
Conjugate 4 (1 mg/kg)

FIG. 5B

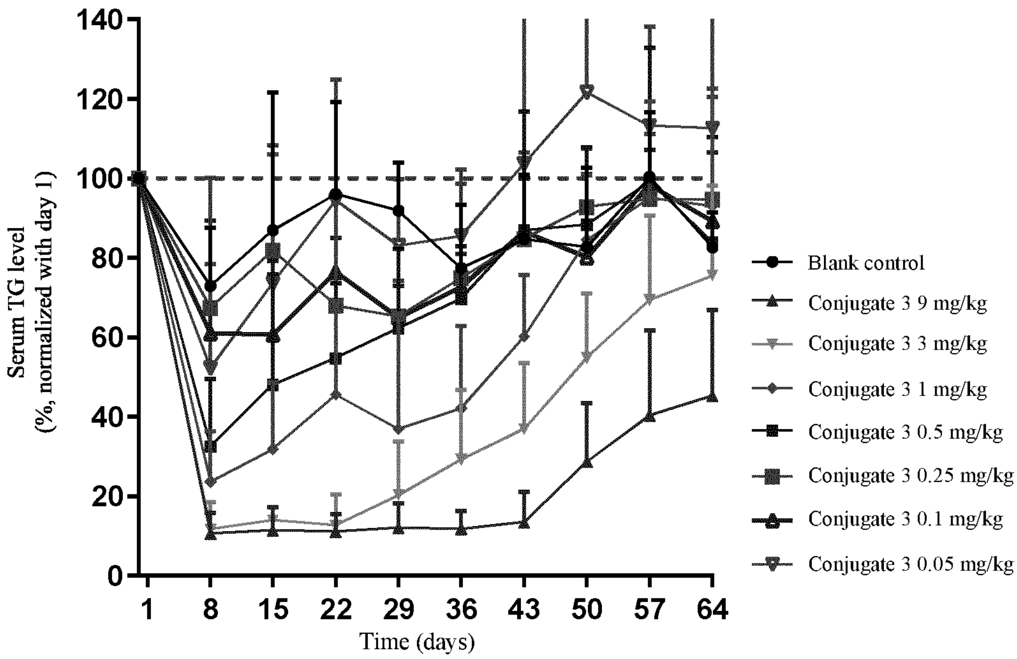
140
120
100
80
60
40
20
0
Serum TG level
(%, normalized with day 1)
1
8
15
22
29
36
43
50
57
64
Time (days)
Blank control
Conjugate 3 9 mg/kg
Conjugate 3 3 mg/kg
Conjugate 3 1 mg/kg
Conjugate 3 0.5 mg/kg
Conjugate 3 0.25 mg/kg
Conjugate 3 0.1 mg/kg
Conjugate 3 0.05 mg/kg

FIG. 6

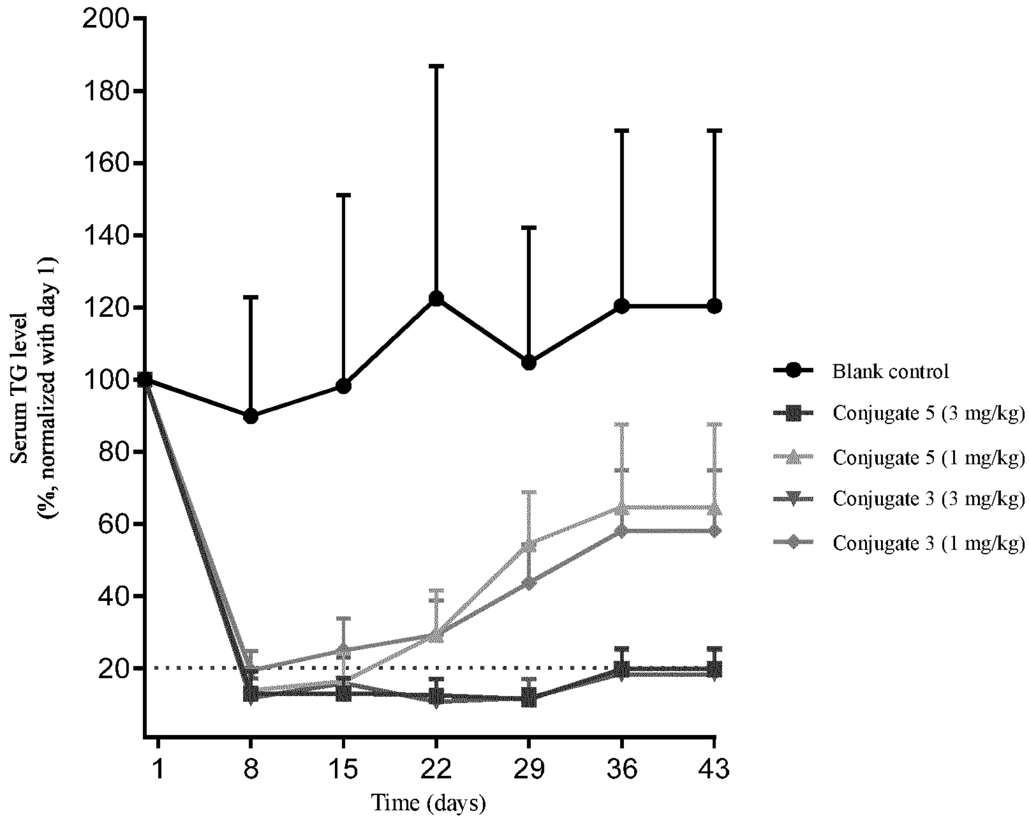
200
180
160
140
120
100
80
60
40
20
Serum TG level
(%, normalized with day 1)
1
8
15
22
29
36
43
Time (days)
Blank control
Conjugate 5 (3 mg/kg)
Conjugate 5 (1 mg/kg)
Conjugate 3 (3 mg/kg)
Conjugate 3 (1 mg/kg)

FIG. 7A

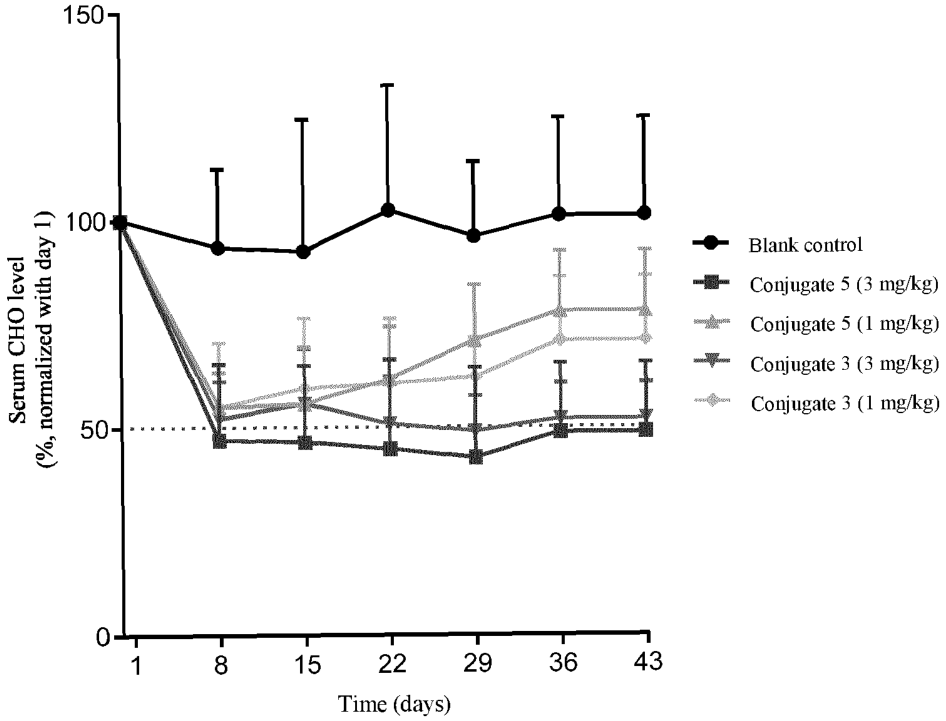
150
100
50
0
Serum CHO level
(%, normalized with day 1)
1
8
15
22
29
36
43
Time (days)
Blank control
Conjugate 5 (3 mg/kg)
Conjugate 5 (1 mg/kg)
Conjugate 3 (3 mg/kg)
Conjugate 3 (1 mg/kg)

FIG. 7B

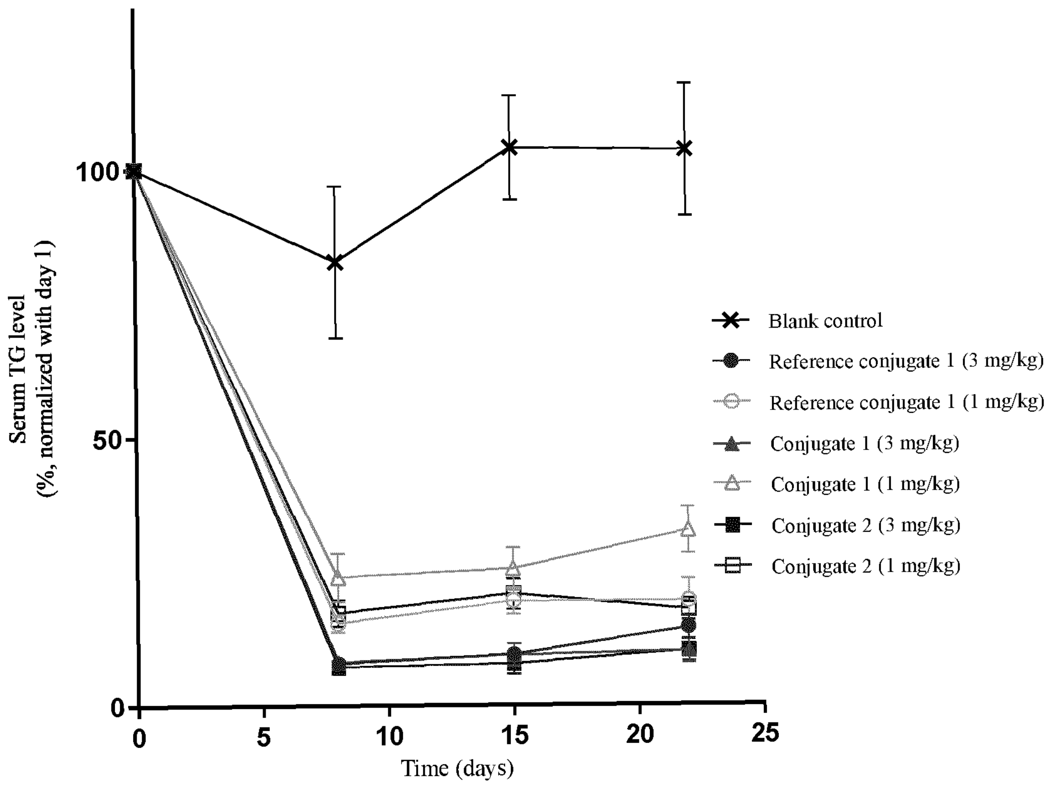
100
50
0
Serum TG level
(%, normalized with day 1)
0
5
10
15
20
25
Time (days)
Blank control
Reference conjugate 1 (3 mg/kg)
Reference conjugate 1 (1 mg/kg)
Conjugate 1 (3 mg/kg)
Conjugate 1 (1 mg/kg)
Conjugate 2 (3 mg/kg)
Conjugate 2 (1 mg/kg)

FIG. 8A

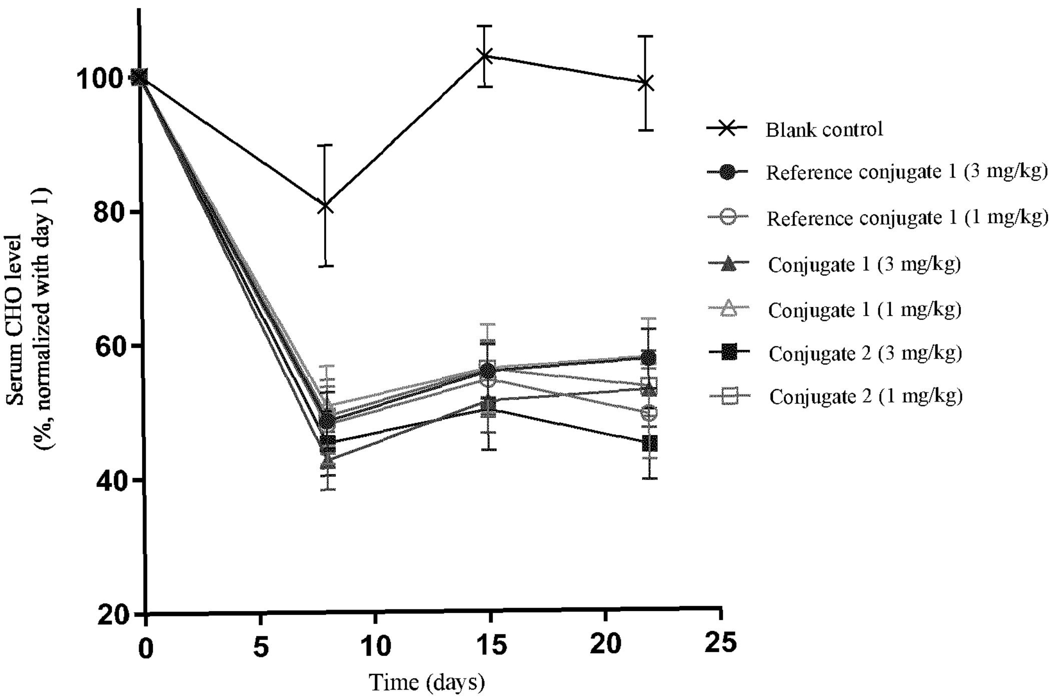
Serum CHO level
(%, normalized with day 1)
100
80
60
40
20
0
5
10
15
20
25
Time (days)
Blank control
Reference conjugate 1 (3 mg/kg)
Reference conjugate 1 (1 mg/kg)
Conjugate 1 (3 mg/kg)
Conjugate 1 (1 mg/kg)
Conjugate 2 (3 mg/kg)
Conjugate 2 (1 mg/kg)

FIG. 8B

**INTERNATIONAL SEARCH REPORT**

International application No.
**PCT/CN2022/103049**

**A. CLASSIFICATION OF SUBJECT MATTER**

C12N 15/113(2010.01)i; A61K 47/54(2017.01)i; A61P 3/06(2006.01)i; A61K 48/00(2006.01)i; A61K 31/713(2006.01)i

According to International Patent Classification (IPC) or to both national classification and IPC

**B. FIELDS SEARCHED**

Minimum documentation searched (classification system followed by classification symbols)

C12N, A61K, A61P,

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, USTXT, WOTXT, CNKI, 万方, WANFANG, EBI, NCBI, 百度, BAIDU, ISI Web of Knowledge, PUBMED, STN, 中国专利生物序列检索系统, Chinese Patent Biological Sequence Retrieval System: 苏州瑞博生物技术股份有限公司, 梁子才, 张鸿雁, 高山, 甲氧基乙基, 甲基乙基, 甲氧乙基, 烯丙基, 二甲基氨基乙基, 氨基丙基, 二硝基苯基, MOE, 2'-MOE, RNA, siRNA, 载脂蛋白C3, 载脂蛋白, APOC, APOC3, 2'-O-甲氧基乙基, 2'-MOE, 2'-O-methoxyethyl, 2'-O-(2-Methoxyethyl), CH2CH2OCH3, 2′-OCH2CH2OCH3, 空间位阻, 2'-O-烯丙基, 2'-C-烯丙基, 2'-O-2-N-甲基氨基-2-氧亚基乙基, 2'-O-2-N, N-二甲基氨基乙基, 2'-O-3-氨基丙基, 2'-O-2,4-二硝基苯基

**C. DOCUMENTS CONSIDERED TO BE RELEVANT**

| Category* | Citation of document, with indication, where appropriate, of the relevant passages | Relevant to claim No. |
|---|---|---|
| Y | US 2020360522 A1 (SUZHOU RIBO LIFE SCIENCE CO., LTD.) 19 November 2020 (2020-11-19)<br>abstract, SEQ ID NOs: 1-3, 6-7, 10-11, 14-15, 18-19, 22-23, 26, 28, 30, 32, and 60, and description, paragraph [0101] | 1-51 |
| Y | WO 2020135673 A1 (SUZHOU RIBO LIFE SCIENCE CO., LTD.) 02 July 2020 (2020-07-02)<br>abstract, SEQ ID NOs: 1-2, 5-6, 9-10, 19-20, 23-24, 49-50, 53-54, 57-58, 61-62, 65-66, 69-70, 75-76, 109-110, 113-114, 121-122, 125-126, 129-130, 135-136, 139-140, and 143-144, and description, p. 15, lines 35-36 | 1-51 |
| Y | CN 111164091 A (WAVE LIFE SCIENCES LTD. et al.) 15 May 2020 (2020-05-15)<br>abstract, description, paragraphs [0378] and [1862], and figure 1 | 1-51 |
| A | US 2012184595 A1 (PROTIVE BIOTHERAPEUTICS INC.) 19 July 2012 (2012-07-19)<br>entire document | 1-51 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 September 2022** | **08 October 2022** |

Name and mailing address of the ISA/CN
**China National Intellectual Property Administration (ISA/CN)**
**No. 6, Xitucheng Road, Jimenqiao, Haidian District, Beijing 100088, China**
Facsimile No. **(86-10)62019451**

Authorized officer

Telephone No.

Form PCT/ISA/210 (second sheet) (January 2015)

**INTERNATIONAL SEARCH REPORT**

International application No.
**PCT/CN2022/103049**

**C. DOCUMENTS CONSIDERED TO BE RELEVANT**

| Category* | Citation of document, with indication, where appropriate, of the relevant passages | Relevant to claim No. |
|---|---|---|
| A | US 2017137814 A1 (ARROWHEAD PHARMACEUTICALS INC.) 18 May 2017 (2017-05-18) entire document | 1-51 |
| A | US 2018245076 A1 (IONIS PHARMACEUTICALS INC.) 30 August 2018 (2018-08-30) entire document | 1-51 |
| A | WO 2019051402 A1 (ARROWHEAD PHARMACEUTICALS INC.) 14 March 2019 (2019-03-14) entire document | 1-51 |

Form PCT/ISA/210 (second sheet) (January 2015)

**INTERNATIONAL SEARCH REPORT**

International application No.
**PCT/CN2022/103049**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

International application No.
**PCT/CN2022/103049**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **48-49**
because they relate to subject matter not required to be searched by this Authority, namely:

[1] claims 48-49 relate to a method for treating diseases, and therefore do not comply with PCT Rule 39.1(iv). The present report was formed on the basis that claims 48-49 are amended to be pharmaceutical use claims.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/103049**

| Patent document cited in search report | Publication date (day/month/year) | Patent family member(s) | Publication date (day/month/year) |
|---|---|---|---|
| US 2020360522 A1 | 19 November 2020 | TW 201925471 A | 01 July 2019 |
| | | EP 3719127 A1 | 07 October 2020 |
| | | WO 2019105419 A1 | 06 June 2019 |
| | | JP 2021503929 A | 15 February 2021 |
| | | CN 110997917 A | 10 April 2020 |
| WO 2020135673 A1 | 02 July 2020 | US 2022062427 A1 | 03 March 2022 |
| | | EP 3903830 A1 | 03 November 2021 |
| | | KR 20210110839 A | 09 September 2021 |
| | | JP 2022515503 A | 18 February 2022 |
| CN 111164091 A | 15 May 2020 | WO 2018223073 A1 | 06 December 2018 |
| | | EP 3630788 A1 | 08 April 2020 |
| | | JP 2020522265 A | 30 July 2020 |
| | | US 2021198305 A1 | 01 July 2021 |
| | | TW 201908483 A | 01 March 2019 |
| US 2012184595 A1 | 19 July 2012 | EP 2405921 A1 | 18 January 2012 |
| | | US 2015315584 A1 | 05 November 2015 |
| | | WO 2010083615 A1 | 29 July 2010 |
| | | CA 2750561 A1 | 29 July 2010 |
| US 2017137814 A1 | 18 May 2017 | EP 3736334 A1 | 11 November 2020 |
| | | WO 2016011123 A1 | 21 January 2016 |
| | | EP 3169784 A1 | 24 May 2017 |
| | | US 2019233817 A1 | 01 August 2019 |
| US 2018245076 A1 | 30 August 2018 | MX 2017011009 A | 20 October 2017 |
| | | HK 1248522 A1 | 19 October 2018 |
| | | RU 2017133142 A | 28 March 2019 |
| | | BR 112017015307 A2 | 16 January 2018 |
| | | KR 20170122769 A | 06 November 2017 |
| | | EP 3270931 A1 | 24 January 2018 |
| | | JP 2018511555 A | 26 April 2018 |
| | | IL 253346 D0 | 28 September 2017 |
| | | CN 107405358 A | 28 November 2017 |
| | | CA 2977971 A1 | 01 September 2016 |
| | | US 2020095581 A1 | 26 March 2020 |
| | | WO 2016138355 A1 | 01 September 2016 |
| | | AU 2016222548 A1 | 27 July 2017 |
| WO 2019051402 A1 | 14 March 2019 | CL 2020000593 A1 | 25 September 2020 |
| | | PH 12020500332 A1 | 28 September 2020 |
| | | JP 2020533334 A | 19 November 2020 |
| | | SG 11201912178V A | 30 January 2020 |
| | | UY 37871 A | 29 March 2019 |
| | | US 2019078088 A1 | 14 March 2019 |
| | | US 2020299690 A1 | 24 September 2020 |
| | | EC SP20017905 A | 30 June 2020 |
| | | KR 20200044013 A | 28 April 2020 |
| | | CR 20200108 A | 28 June 2020 |
| | | JO P20200054 A1 | 11 March 2019 |
| | | CO 2020002586 A2 | 01 April 2020 |
| | | BR 112020002413 A2 | 28 July 2020 |
| | | CN 111107853 A | 05 May 2020 |

Form PCT/ISA/210 (patent family annex) (January 2015)

**INTERNATIONAL SEARCH REPORT**
**Information on patent family members**

International application No.
**PCT/CN2022/103049**

| Patent document cited in search report | Publication date (day/month/year) | Patent family member(s) | | | Publication date (day/month/year) |
|---|---|---|---|---|---|
| | | AU | 2018329190 | A1 | 23 April 2020 |
| | | TN | 2020000039 | A1 | 04 October 2021 |
| | | TW | 201920227 | A | 01 June 2019 |
| | | CA | 3074303 | A1 | 14 March 2019 |
| | | PE | 20201283 | A1 | 24 November 2020 |
| | | EP | 3681516 | A1 | 22 July 2020 |
| | | IL | 273172 | A | 30 April 2020 |
| | | US | 2022064646 | A1 | 03 March 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A
- WO 2015006740 A2
- WO 2009082607 A2
- WO 2014025805 A1
- CN 110959011 A
- WO 2019105418 A


### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- **J.K. WATTS ; G.F. DELEAVEY ; M.J. DAMHA.** Chemically modified siRNA: tools and applications. *Drug Discov Today,* 2008, vol. 13 (19-20), 842-55
- **ANASTASIA KHVOROVA ; JONATHAN K. WATTS.** The chemical evolution of oligonucleotide therapies of clinical utility. *Nature Biotechnology,* 2017, vol. 35 (3), 238-48
- **RAJEEV et al.** *ChemBioChem,* 2015, vol. 16, 903-908
- Molecular Cloning. Cold Spring Harbor LBboratory Press, 1989
- **KUMICO UI-TEI et al.** Functional dissection of siRNA sequence by systematic DNA substitution: modified siRNA with a DNA seed arm is a powerful tool for mammalian gene silencing with significantly reduced off-target effect. *Nucleic Acids Research,* 2008, vol. 36 (7), 2136-2151